(19) Europäisches Patentamt
European Patent Office
Office européen des brevets

(11) EP 0 486 423 B1

(12) EUROPÄISCHE PATENTSCHRIFT

(45) Veröffentlichungstag und Bekanntmachung des Hinweises auf die Patenterteilung:
**22.01.1997 Patentblatt 1997/04**

(21) Anmeldenummer: **91810814.3**

(22) Anmeldetag: **22.10.1991**

(51) Int Cl.6: **C07D 498/04**, C09B 19/02, C09B 62/002, D06P 1/38, D06P 1/40
// (C07D498/04, 265:00, 265:00)

(54) **Oligomerengemische von Triphendioxazinverbindungen, Verfahren zu ihrer Herstellung und ihre Verwendung**

Oligomer mixtures of triphenedioxazine compounds, process for their preparation, and their use

Mélanges des oligomères de composés triphènedioxazine, procédé pour leur préparation et leur utilisation

(84) Benannte Vertragsstaaten:
**BE CH DE ES FR GB IT LI**

(30) Priorität: **30.10.1990 CH 3442/90**

(43) Veröffentlichungstag der Anmeldung:
**20.05.1992 Patentblatt 1992/21**

(73) Patentinhaber: **Ciba SC Holding AG
4057 Basel (CH)**

(72) Erfinder: **Lauk, Urs, Dr.
CH-8047 Zürich (CH)**

(56) Entgegenhaltungen:
**EP-A- 0 271 656**
**EP-A- 0 361 186**
**DE-A- 2 647 426**
**DE-A- 3 410 236**
**GB-A- 2 228 738**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 486 423 B1

**Beschreibung**

Die vorliegende Erfindung betrifft neue Oligomerengemische von Triphendioxazinverbindungen, Verfahren zu ihrer Herstellung sowie ihre Verwendung zum Färben und Bedrucken von Fasermaterialien, insbesondere von textilen Fasermaterialien.

Gegenstand der vorliegenden Erfindung sind somit Oligomerengemische enthaltend mindestens zwei Verbindungen der Formel

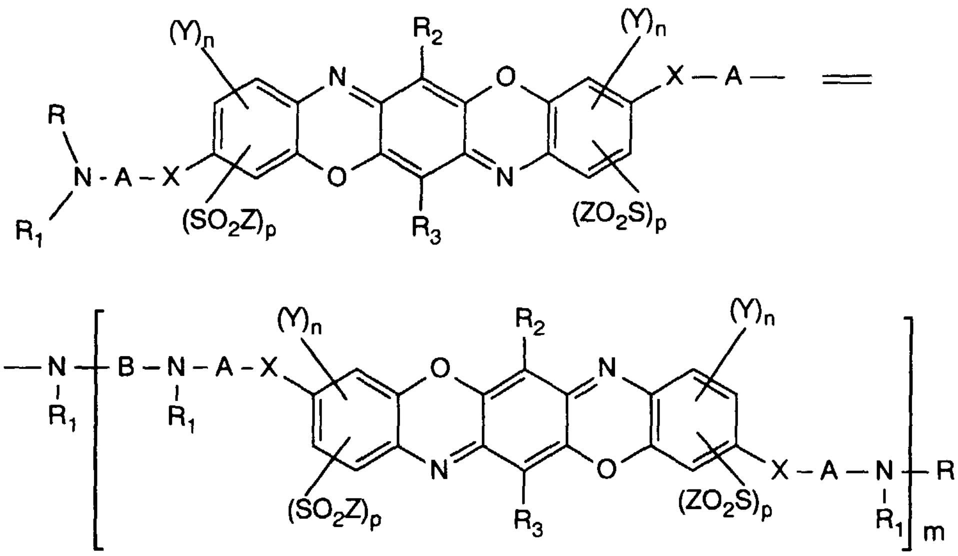

(1),

worin

$R_1$ für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes $C_1$-$C_4$-Alkyl, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl steht,

R unabhängig die Bedeutung von $R_1$ hat, oder

ein Pyridin-, Pyrimidin-, Chinoxalin oder Triazinrest ist, wobei diese Reste unsubstituiert oder durch Hydroxy; $C_1$-$C_4$-Alkyl; Phenyl; $C_1$-$C_4$-Alkoxy; $C_1$-$C_4$-Alkylthio; Amino; unsubstituiertes oder im Alkylteil durch Hydroxy, Carboxy, Cyano, Sulfo, Sulfato oder $C_1$-$C_4$Alkoxy substituiertes N-Mono- oder N,N-Di-$C_1$-$C_4$-Alkylamino; Cyclohexylamino; unsubstituiertes oder im Phenylteil durch $C_1$-$C_4$-Alkyl, $C_1$-$C_4$-Alkoxy, Phenoxy, Carboxy, Sulfo und/oder Halogen substituiertes Phenylamino oder N-$C_1$-$C_4$-Alkyl-N-phenylamino; Morpholino; oder 3-Carboxy- oder 3-Carbamoylpyridin-1-yl; sind, oder

R ein Rest der Formel

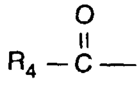

,

ist, worin $R_4$ ein Chinoxalin- oder Pyrimidinrest ist oder die oben für $R_1$ angegebenen Bedeutungen hat, jedoch nicht Wasserstoff bedeutet,

A ein unsubstituierter oder durch Hydroxy, Sulfo, Sulfato, $C_1$-$C_4$-Alkoxy, Carboxy, Cyano, Halogen, Phenyl, Sulfophenyl oder $C_2$-$C_5$-Alkoxycarbonyl substituierter und/oder gegebenenfalls durch 1-2 Gruppen -O-, -N($R_8$)-, worin $R_8$ $C_1$-$C_4$-Alkyl, Acetyl oder insbesondere Wasserstoff bedeutet, -S- oder -$SO_2$- unterbrochener $C_2$-$C_6$-Alkylenrest; unsubstituiertes oder einfach oder mehrfach durch $C_1$ -$C_3$-Alkyl substituiertes Cyclopentylen oder Cyclohexylen;

ein unsubstituierter oder durch $C_1$-$C_4$-Alkyl, $C_1$ -$C_4$-Alkoxy, Sulfo, Halogen oder Carboxy substituierter Phenylen-, Biphenylen- oder Naphthylenrest; oder

ein $C_1$-$C_6$-Alkylen-phenylen-, Phenylen-$C_1$-$C_6$-alkylen-phenylen-, $C_1$-$C_3$-Alkylenphenylen-$C_1$-$C_3$-alkylen- oder ein Methylen-naphthylen-methylenrest ist, wobei in diesen Aralkylenresten das Phenylen und Naphthylen gegebenenfalls noch 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino tragen kann,

X -O-, -S- oder -N($R_5$)- ist, worin $R_5$ die oben für $R_1$ angegebenen Bedeutungen hat, oder worin die Gruppe

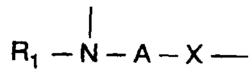

ein Piperazin-1,4-diylrest ist,

Y $C_1$-$C_4$-Alkyl, $C_1$-$C_4$-Alkoxy, Halogen, Sulfo, Carboxy, Carbamoyl, N-Mono- oder N,N-Di-$C_1$-$C_4$-Alkylcarbamoyl, N-Phenyl- oder N,N-Diphenylcarbamoyl, Sulfamoyl, N-Mono- oder N,N-Di-$C_1$-$C_4$-Alkylsulfamoyl oder N-Phenyl- oder N,N-Diphenylsulfamoyl ist,

Z Hydroxy oder $C_1$-$C_4$-Alkyl ist,

$R_2$ und $R_3$ unabhängig voneinander je Wasserstoff; Halogen; Cyano; $C_1$-$C_4$-Alkyl; $C_1$-$C_4$-Alkoxy; Sulfo; Carboxy; Carbamoyl; Phenylcarbamoyl; $C_2$-$C_5$-Alkanoylamino; oder gegebenenfalls durch $C_1$-$C_4$-Alkyl, $C_1$-$C_4$-Alkoxy, Acetylamino, Halogen, Nitro oder Sulfo substituiertes Phenyl, Benzyl, Benzoylamino oder Phenoxy sind,

B ein Brückenglied der Formel

Q'-E'-Q' (3'),

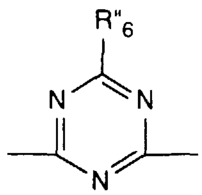

(4'),

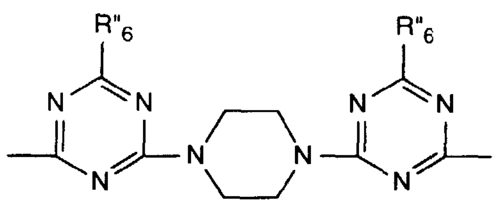

(6'),

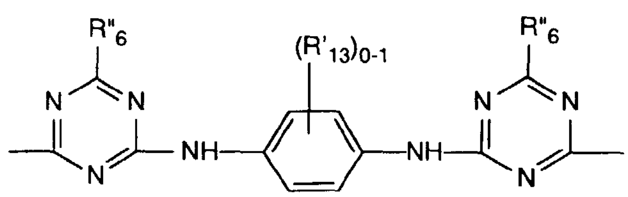

(7')

oder

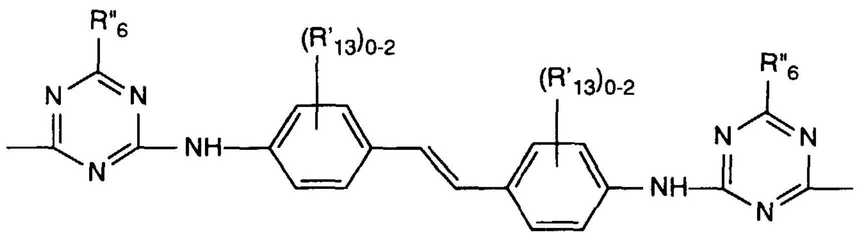

(8)

ist,
worin Q' eine Gruppe

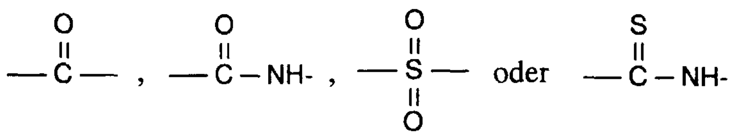

oder

bedeutet,
E' für die direkte Bindung;
unsubstituiertes oder durch Hydroxy, Sulfo, Sulfato, Methoxy, Carboxy, Phenyl oder Sulfophenyl substituiertes $C_1$-$C_6$-Alkylen oder $C_2$-$C_6$-Alkenylen;
unsubstituiertes oder durch 1 bis 3 Methylgruppen substituiertes Cyclohexylen oder $C_1$-$C_2$-Alkylen-cyclohexylen;
Piperazin-1,4-diyl; Thiophen-2,5-diyl; Biphenyl-4,4'-diyl; Stilben-4,4'-diyl;
unsubstituiertes oder durch $C_1$-$C_4$-Alkyl, $C_1$-$C_4$-Alkoxy, Sulfo, Halogen oder Carboxy substituiertes Phenylen oder Naphthylen; oder für
unsubstituiertes oder im Phenylteil durch Methyl, Methoxy, Chlor oder Sulfo substituiertes $C_1$-$C_3$-Alkylen-phenylen oder $C_1$-$C_2$-Alkylen-phenylen-$C_1$-$C_2$-alkylen steht,
$R''_6$ Chlor; Hydroxy; $C_1$-$C_4$-Alkoxy; $C_1$-$C_2$-Alkylthio; Amino; unsubstituiertes oder im Alkylteil durch Hydroxy, Sulfo oder Sulfato substituiertes N-Mono- oder
N,N-Di-$C_1$-$C_4$-Alkylamino; Cyclohexylamino; unsubstituiertes oder im Phenylteil durch Methyl, Methoxy, Carboxy, Sulfo oder Chlor substituiertes Phenylamino oder N-$C_1$-$C_4$-Alkyl-N-phenylamino; oder Morpholino bedeutet und
$R'_{13}$ Sulfo, Methyl oder Methoxy bedeutet,
m eine Zahl von 1 bis 6 bedeutet und
n und p unabhängig voneinander je für die Zahl 0 oder 1 stehen,
wobei sich die verschiedenen Verbindungen der Formel (1) ausschliesslich im Wert von m unterscheiden.

Aus der GB-A-2,228,738 sind Farbstoffe bekannt, welche sich von den Verbindungen der Formel (1) hinsichtlich des fehlenden Restes der Formel -N($R_1$)-A- unterscheiden.

Beispiele für geeignete Alkylreste $R_1$ sind somit Methyl, Ethyl, n-Propyl, iso-Propyl, n-, iso-, sec.- oder tert.-Butyl, β-Chlorethyl, β-Hydroxyethyl, β-Hydroxybutyl, β-Cyanethyl, Sulfomethyl, β-Sulfoethyl, β-Sulfatoethyl, β-Acetoxyethyl, β-Sulfatopropyl, γ-Sulfatopropyl oder der Rest der Formel $-CH_2CH_2OCH_2CH_2OH$ oder $-CH_2CH_2OCH_2CH_2OSO_3H$.

$R_1$ steht als Alkylrest für einen unsubstituierten oder durch Hydroxy, Sulfo, Sulfato, Chlor, Cyano oder Acetoxy substituierten und/oder mit Ausnahme von Methyl gegebenenfalls durch eine Gruppe -O- unterbrochenen $C_1$-$C_4$-Alkylrest.

Besonders bevorzugt ist für $R_1$ als Alkyl die Bedeutung eines unsubstituierten $C_1$-$C_4$-Alkylrestes, und sind insbesondere die Bedeutungen Methyl und Ethyl.

$R_1$ steht als Cycloalkyl für unsubstituiertes oder durch 1 bis 3 Methylgruppen substituiertes Cyclopentyl oder Cyclohexyl und insbesondere bevorzugt für Cyclohexyl.

Besonders bevorzugt ist für $R_1$ als Aryl die Bedeutung eines unsubstituierten oder durch Sulfo, Chlor, Methyl und/oder Methoxy substituierten Phenylrestes.

$R_1$ als Aralkylrest stellt insbesondere bevorzugt den Benzylrest dar.

$R_1$ bedeutet besonders bevorzugt Wasserstoff, $C_1$-$C_4$-Alkyl, Cyclohexyl, unsubstituiertes oder durch Sulfo, Chlor, Methyl und/oder Methoxy substituiertes Phenyl oder Benzyl und insbesondere bevorzugt Wasserstoff, Methyl oder Ethyl. In einer besonders bevorzugten Ausführungsform der Erfindung steht $R_1$ für Wasserstoff.

Hat R unabhängig eine der zuvor für $R_1$ genannten Bedeutungen, so schliesst das die zuvor für $R_1$ genannten Bevorzugungen mit ein.

Steht R für einen Heteroarylrest, so ist dies ein gegebenenfalls durch nicht-faserreaktive Substituenten substituierter Pyridin-, Pyrimidin-, Chinoxalin oder Triazinrest.

Nicht-faserreaktive Substituenten am Heteroarylrest R sind Hydroxy, $C_1$-$C_4$-Alkyl, Phenyl, $C_1$-$C_4$-Alkoxy, $C_1$-$C_4$-Alkylthio, Amino, unsubstituiertes oder im Alkylteil durch Hydroxy, Carboxy, Cyano, Sulfo, Sulfato oder $C_1$-$C_4$-Alkoxy substituiertes N-Mono- oder N,N-Di-$C_1$-$C_4$-Alkylamino, Cyclohexylamino, unsubstituiertes oder im Phenylteil durch $C_1$-$C_4$-Alkyl, $C_1$-$C_4$-Alkoxy, Phenoxy, Carboxy, Sulfo und/oder Halogen substituiertes Phenylamino oder N-$C_1$-$C_4$-Alkyl-N-phenylamino, Morpholino oder 3-Carboxy- oder 3-Carbamoylpyridin-1-yl.

Vorzugsweise handelt es sich bei R als Heteroarylrest um einen Triazinylrest der Formel

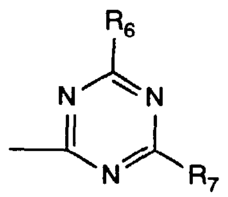

(2),

worin $R_6$ und $R_7$ unabhängig voneinander je einen der zuvor genannten nicht-faserreaktiven Substituenten bedeuten; dabei können die Reste $R_6$ und $R_7$ gleich oder verschieden sein.

Steht $R_4$ für gegebenenfalls substituiertes Alkyl, Cycloalkyl, Aryl oder Aralkyl, so gelten die zuvor für $R_1$ genannten Bedeutungen und Bevorzugungen.

Bei $R_4$ als Heteroarylrest handelt es sich um einen Chinoxalin- oder Pyrimidinrest.

R bedeutet vorzugsweise Wasserstoff, $C_1$-$C_4$-Alkyl, unsubstituiertes oder durch Methyl, Methoxy, Chlor und/oder Sulfo substituiertes Phenyl, Benzyl oder einen Rest der Formel

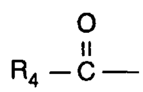

,

worin $R_4$ für Methyl, Ethyl oder unsubstituiertes oder durch Sulfo, Chlor, Methyl und/oder Methoxy substituiertes Phenyl steht; besonders bevorzugte Bedeutungen von R sind Methyl, Ethyl, Benzyl, Acetylamino, Benzoylamino und insbesondere Wasserstoff.

Bei A als gegebenenfalls substituiertem Alkylenrest handelt es sich um einen unsubstituierten oder durch Hydroxy, Sulfo, Sulfato, $C_1$-$C_4$-Alkoxy, Carboxy, Cyano, Halogen, Phenyl, Sulfophenyl oder $C_2$-$C_5$-Alkoxycarbonyl substituierten und/oder gegebenenfalls durch 1-2 Gruppen -O-, -N($R_8$)-, worin $R_8$ $C_1$-$C_4$-Alkyl, Acetyl oder insbesondere Wasserstoff bedeutet, -S- oder -$SO_2$- unterbrochenen $C_2$-$C_6$-Alkylenrest.

Beispiele für geeignete Alkylenreste A sind 1,2-Ethylen, 1,2- und 1,3-Propylen, 1-Ethyl-1,2-ethylen, 2-Hydroxy-1,3-propylen, 2-Sulfato-1,3-propylen, 1- und 2-Phenyl-1,3-propylen, 2-(4'-Sulfophenyl)-1,3-propylen, 1,4-, 2,3- und 2,4-Butylen, 1,2-Dimethyl-1,2-ethylen, 1-Phenyl-1,2-ethylen, 2-Methyl-1,3-propylen, 2,2-Dimethyl-1,3-propylen, 1-Chlor-2,3-propylen, 1,5- und 2,4-pentylen, 2-Methyl-2,4-pentylen, 1-Carboxy-1,5-pentylen, 2,3-Diphenyl-1,4-butylen, 1-Methoxycarbonyl-1,5-pentylen, 1,6 und 2,5-Hexylen, 2-Carboxy-1,3-propylen, 2-Methoxy-1,3-propylen, ein Rest der Formel -$CH_2$-$CH_2$-Z'-$CH_2$-$CH_2$-, worin Z' -O-, -S-, -$SO_2$-, -NH- oder -N($CH_3$)- bedeutet.

A steht als Alkylenrest besonders bevorzugt für einen unsubstituierten oder durch Hydroxy, Sulfo, Sulfato, Methoxy, Carboxy oder Sulfophenyl substituierten $C_2$-$C_4$-Alkylenrest oder -$CH_2$-$CH_2$-Z'-$CH_2$-$CH_2$-, worin Z' -O-, -S-, -$SO_2$-, -NH- oder -N($CH_3$)- bedeutet, insbesondere für einen unsubstituierten oder durch Hydroxy, Sulfo, Sulfato, Methoxy, Carboxy oder Sulfophenyl substituierten $C_2$-$C_4$-Alkylenrest und insbesondere bevorzugt für einen unsubstituierten oder durch Hydroxy oder Sulfato substituierten 1,2-Ethylen oder 1,2- oder 1,3-Propylenrest. In einer besonders bevorzugten Ausführungsform der Erfindung steht A für 1,2-Ethylen, 1,2- oder 1,3-Propylen oder für 2-Sulfato-1,3-propylen.

Bei A als gegebenenfalls substituiertem Cycloalkylen handelt es sich um unsubstituiertes oder einfach oder mehrfach durch $C_1$-$C_3$-Alkyl substituiertes Cyclopentylen oder Cyclohexylen; besonders bevorzugt steht hierbei A für unsubstituiertes oder durch 1 bis 3 Methylgruppen substituiertes Cyclohexylen.

Beispiele für geeignete cycloaliphatische Reste A sind:

1,3- und 1,4-Cyclohexylen, 4-Methyl-1,3-cyclohexylen, 2-Methyl-1,3-cyclohexylen, 5,5-Dimethyl-1,3-cyclohexylen, 2-Methyl-1,4-cyclohexylen, 4,6-Dimethyl-1,3-cyclohexylen und 4-Methyl-1,2-cyclohexylen.

Steht A für einen bivalenten Arylrest, so ist dies ein unsubstituierter oder durch $C_1$-$C_4$-Alkyl, $C_1$-$C_4$-Alkoxy, Sulfo, Halogen oder Carboxy substituierter Phenylen-, Biphenylen- oder Naphthylenrest.

Beispiele für Arylenreste A sind: 1,3- und 1,4-Phenylen, 2-Sulfo-1,4-phenylen, 4-Sulfo-1,3-phenylen, 2-Methyl-1,4-phenylen, 2-Carboxy-1,4-phenylen, 2-Methoxy-1,4-phenylen, 4,8-Disulfo-2,6-naphthylen, 8-Sulfo-2,6-naphthylen,

1,4-Naphthylen und 1,1'-Biphenyl-4,4'-diyl.

A steht als Arylenrest vorzugsweise für einen unsubstituierten oder durch Sulfo, Methyl, Methoxy oder Carboxy substituierten 1,3- oder 1,4-Phenylenrest oder für einen unsubstituierten oder durch Sulfo substituierten Naphthylenrest.

Bei A als Arylenrest handelt es sich besonders bevorzugt um unsubstituiertes oder durch Sulfo substituiertes 1,3- oder 1,4-Phenylen.

Bedeutet A einen gegebenenfalls substituierten Aralkylenrest, so ist dies ein $C_1$-$C_6$-Alkylen-phenylen-, ein Phenylen-$C_1$-$C_6$-alkylen-phenylen-, ein $C_1$-$C_3$-Alkylenphenylen-$C_1$-$C_3$-alkylen- oder ein Methylen-naphthylen-methylenrest sein, wobei in diesen Aralkylenresten das Phenylen und Naphthylen gegebenenfalls noch 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino tragen kann.

Beispiele für geeignete Aralkylenreste A sind

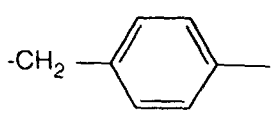
,
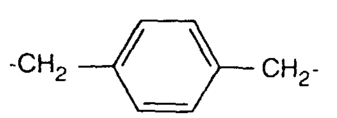
,
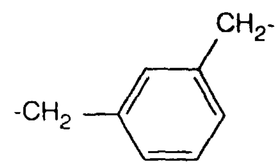
,

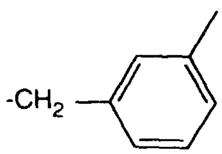
,
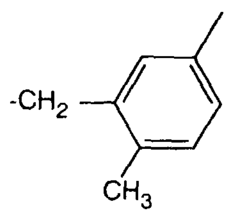
,
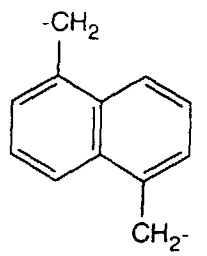
,
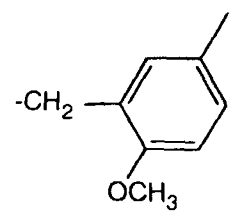
,

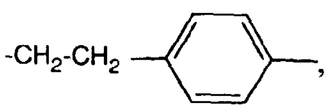
,
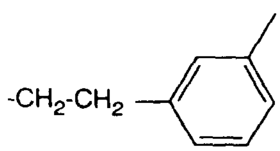
,
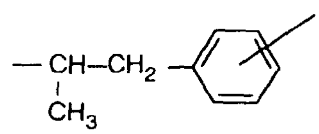
.

A stellt als Aralkylenrest bevorzugt einen $C_1$-$C_3$-Alkylen-phenylen- oder $C_1$-$C_2$-Alkylenphenylen-$C_1$-$C_2$-alkylenrest dar, der im Phenylteil unsubstituiert oder z.B. durch Methyl, Methoxy, Chlor oder Sulfo substituiert ist.

A bedeutet vorzugsweise einen unsubstituierten oder durch Hydroxy, Sulfo, Sulfato, Methoxy, Carboxy, oder Sulfophenyl substituierten $C_2$-$C_4$-Alkylenrest, $-CH_2-CH_2-Z'-CH_2-CH_2-$ worin Z' -O-, -S-, $-SO_2-$, -NH- oder $-N(CH_3)-$ bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder einen $C_1$-$C_3$-Alkylen-phenylen- oder $C_1$-$C_2$-Alkylen-phenylen-$C_1$-$C_2$-alkylenrest, worin das Phenylen jeweils unsubstituiert oder durch Methyl, Methoxy, Chlor oder Sulfo substituiert ist.

Steht $R_5$ für einen gegebenenfalls substituierten Alkyl-, Cycloalkyl-, Aryl- oder Aralkylrest, so gelten unabhängig die zuvor für $R_1$ angegebenen Bedeutungen und Bevorzugungen.

$R_5$ bedeutet bevorzugt Wasserstoff, $C_1$-$C_4$-Alkyl, Cyclohexyl, unsubstituiertes oder durch Sulfo, Chlor, Methyl und/oder Methoxy substituiertes Phenyl oder Benzyl.

Besonders bevorzugte Bedeutungen von $R_5$ sind Wasserstoff und $C_1$-$C_4$-Alkyl, insbesondere Wasserstoff, Methyl und Ethyl und ganz besonders Wasserstoff.

X steht vorzugsweise für eine Gruppe $-N(R_5)-$, worin für $R_5$ die zuvor angegebenen Bedeutungen und Bevorzugungen gelten.

Y bedeutet vorzugsweise Sulfo, $C_1$-$C_4$-Alkyl, $C_1$-$C_4$-Alkoxy oder Chlor und besonders bevorzugt Methoxy, Methyl, Chlor oder Sulfo.

Die Variable n steht bevorzugt für die Zahl 0.

Z steht bevorzugt für Methyl oder Ethyl und insbesondere bevorzugt für Hydroxy.

p bedeutet vorzugsweise die Zahl 1.

Bedeuten $R_2$ und/oder $R_3$ gegebenenfalls substituiertes Phenyl, Benzyl, Benzoylamino oder Phenoxy, so ist der Phenylring jeweils unsubstituiert oder durch $C_1$-$C_4$-Alkyl, $C_1$-$C_4$-Alkoxy, Acetylamino, Halogen, Nitro und/oder Sulfo substituiert; hierbei ist es jeweils bevorzugt, dass der Phenylring keine weiteren Substituenten trägt oder durch Chlor, Methyl, Methoxy, Acetylamino und/oder Sulfo weitersubstituiert ist.

Die Reste $R_2$ und $R_3$ können verschieden oder bevorzugt identisch sein.

$R_2$ und $R_3$ stehen vorzugsweise jedoch für Wasserstoff, Fluor, Chlor, Brom, Methyl, Methoxy, Acetylamino, Phenoxy oder Cyano und besonders bevorzugt für Brom oder Chlor.

Eine bevorzugte Ausführungsform der Erfindung betrifft Oligomerengemische von Verbindungen der Formel (1), worin $R_2$ und $R_3$ jeweils Chlor bedeuten.

Vorzugsweise steht E als Alkylenrest für unsubstituiertes oder durch Hydroxy, Sulfo, Sulfato, Methoxy, Carboxy, Phenyl oder Sulfophenyl substituiertes $C_1$-$C_4$-Alkylen und besonders bevorzugt für Methylen, 1,2-Ethylen oder 1,2- oder 1,3-Propylen.

Bevorzugt als organisches Brückenglied B sind die Reste der Formel (3'), worin Q' eine Gruppe

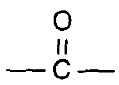 , 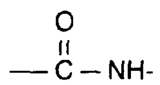 , 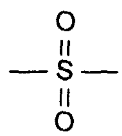 oder 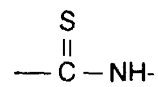

bedeutet, E' für die direkte Bindung, unsubstituiertes oder durch Hydroxy, Sulfo, Sulfato, Methoxy, Carboxy, Phenyl oder Sulfophenyl substituiertes $C_1$-$C_6$-Alkylen oder $C_2$-$C_6$-Alkenylen, unsubstituiertes oder durch 1 bis 3 Methylgruppen substituiertes Cyclohexylen oder $C_1$-$C_2$-Alkylen-cyclohexylen, Piperazin-1,4-diyl, Thiophen-2,5-diyl, Biphenyl-4,4'-diyl, Stilben-4,4'-diyl, unsubstituiertes oder durch $C_1$-$C_4$-Akyl, $C_1$-$C_4$-Alkoxy, Sulfo, Halogen oder Carboxy substituiertes Phenylen oder Naphthylen oder für unsubstituiertes oder im Phenylteil durch Methyl, Methoxy, Chlor oder Sulfo substituiertes $C_1$-$C_3$-Alkylen-phenylen oder $C_1$-$C_2$-Alkylen-phenylen-$C_1$-$C_2$-alkylen steht.

Besonders bevorzugte bivalente Brückenglieder B entsprechen hierbei der Formel

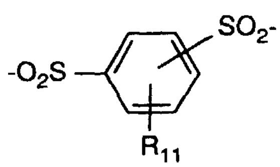 , 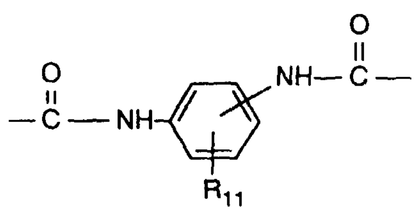 , 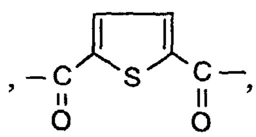 ,

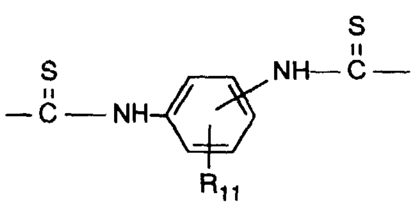 , 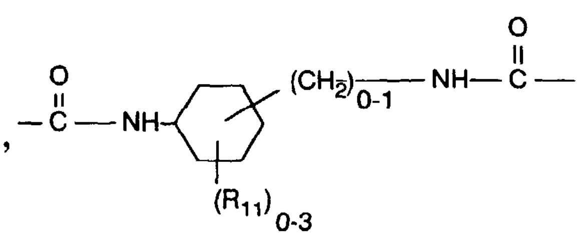 ,

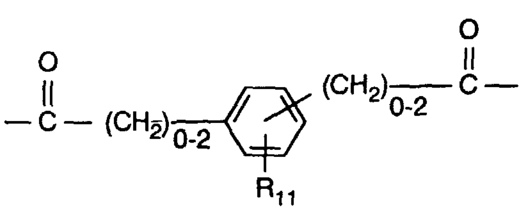 , 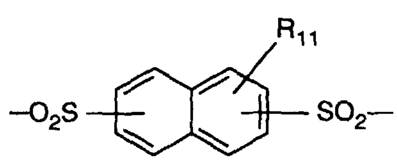 ,

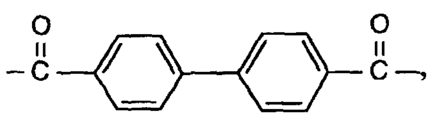, 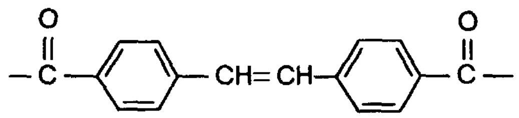,

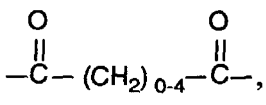, 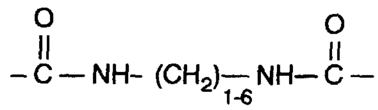, 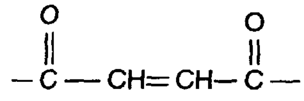,

worin $R_{11}$ Sulfo, Methyl, Methoxy, Chlor, Carboxy oder vorzugsweise Wasserstoff bedeutet.

Eine besonders bevorzugte Ausführungsform der vorliegenden Erfindung betrifft Oligomerengemische von Verbindungen der Formel (1), worin B ein Rest der Formel

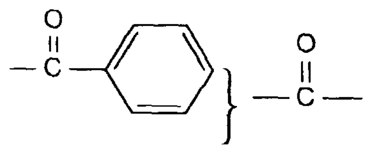

ist.

Beispiele für besonders bevorzugte Reste $R''_6$ sind Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m- oder p-Methoxyphenylamino, o-, m- oder p-Chlorphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino und Morpholino.

Eine weitere Gruppe von brauchbaren Brückengliedern B umfasst solche der Formeln

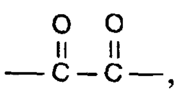, 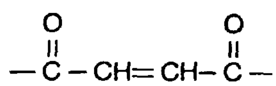

und

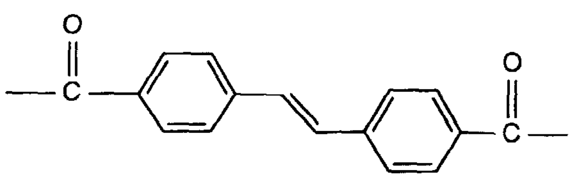 .

Brückenglieder B entsprechen der Formel

$$Q'\text{-}E'\text{-}Q' \qquad (3'),$$

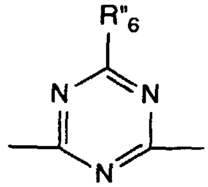

(4'),

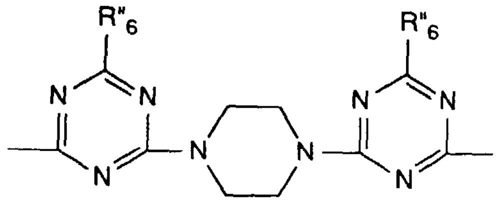

(6'),

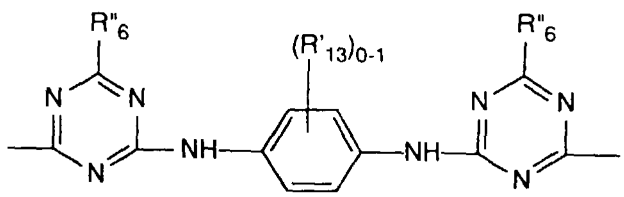

(7')

oder

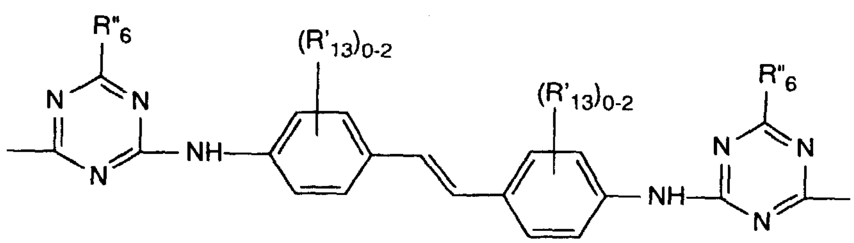

(8'),

worin Q' eine Gruppe

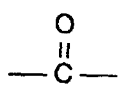 , 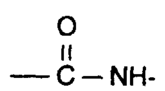 , 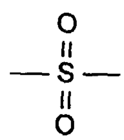 oder 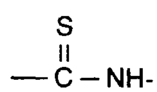

bedeutet, E' für die direkte Bindung, unsubstituiertes oder durch Hydroxy, Sulfo, Sulfato, Methoxy, Carboxy, Phenyl oder Sulfophenyl substituiertes $C_1$-$C_6$-Alkylen oder $C_2$-$C_6$-Alkenylen, unsubstituiertes oder durch 1 bis 3 Methylgruppen substituiertes Cyclohexylen oder $C_1$-$C_2$-Alkylen-cyclohexylen, Piperazin-1,4-diyl, Thiophen-2,5-diyl, Biphenyl-4,4'-diyl, Stilben-4,4'-diyl, unsubstituiertes oder durch $C_1$-$C_4$-Alkyl, $C_1$-$C_4$-Alkoxy, Sulfo, Halogen oder Carboxy substituiertes Phenylen oder Naphthylen oder für unsubstituiertes oder im Phenylteil durch Methyl, Methoxy, Chlor oder Sulfo substituiertes $C_1$-$C_3$-Alkylen-phenylen oder $C_1$-$C_2$-Alkylen-phenylen-$C_1$-$C_2$-alkylen steht, $R''_6$ Chlor, Hydroxy, $C_1$-$C_4$-Alkoxy, $C_1$-$C_2$-Alkylthio, Amino, unsubstituiertes oder im Alkylteil durch Hydroxy, Sulfo oder Sulfato substituiertes N-Mono- oder N,N-Di-$C_1$-$C_4$-Alkylamino, Cyclohexylamino, unsubstituiertes oder im Phenylteil durch Methyl, Methoxy, Carboxy, Sulfo oder Chlor substituiertes Phenylamino oder N-$C_1$-$C_4$-Alkyl-N-phenylamino oder Morpholino bedeutet und $R'_{13}$ Sulfo, Methyl oder Methoxy bedeutet.

Insbesondere bevorzugt als bivalentes Brückenglied B sind die Reste der Formel

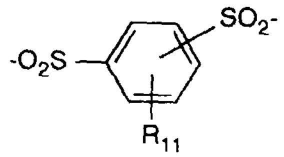, 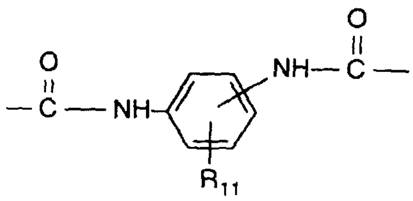, 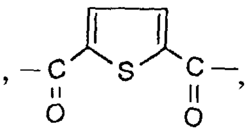,

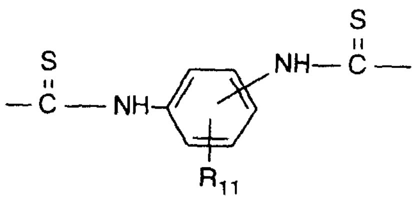, 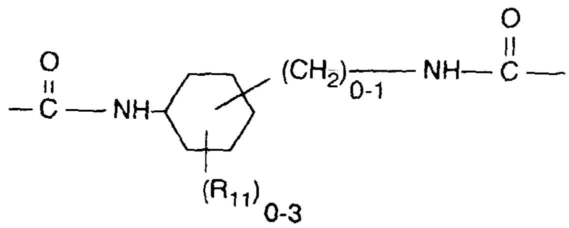,

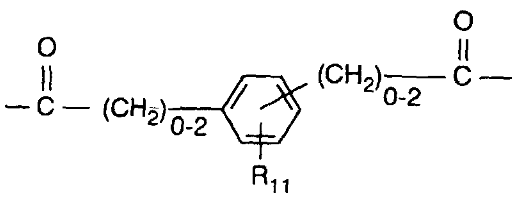, 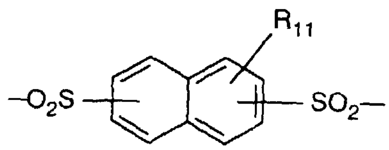,

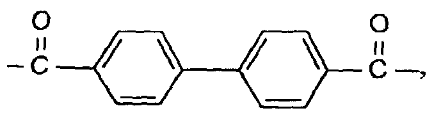, 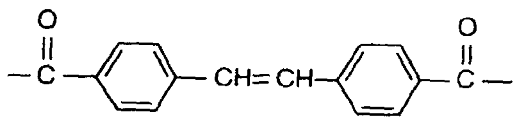,

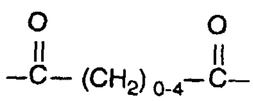, 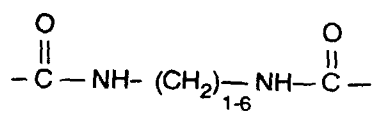, 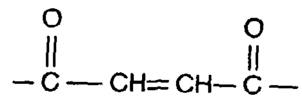

oder

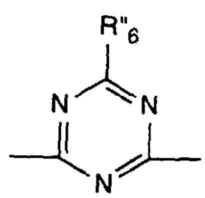,

worin $R_{11}$ Sulfo, Methyl, Methoxy, Chlor, Carboxy oder vorzugsweise Wasserstoff bedeutet und $R''_6$ Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m-, oder p-Methoxyphenylamino, o-, m- oder p-Chlorphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder Morpholino ist.

m steht vorzugsweise für eine beliebige Zahl von 1 bis 3.

Von Bedeutung wegen ihrer guten färberischen Eigenschaften sind Oligomerengemische der Verbindungen der zuvor angegebenen Formel (1), worin $R_1$ für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unter-

brochenes $C_1$-$C_4$-Alkyl, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N, N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl steht, R Wasserstoff, $C_1$-$C_4$-Alkyl, unsubstituiertes oder durch Methyl, Methoxy, Chlor und/oder Sulfo substituiertes Phenyl oder Benzyl oder einen Rest der Formel

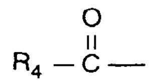 ,

worin $R_4$ für Methyl, Ethyl oder unsubstituiertes oder durch Sulfo, Chlor, Methyl und/oder Methoxy substituiertes Phenyl steht, bedeutet,
A für einen unsubstituierten oder durch Hydroxy, Sulfo, Sulfato, Methoxy, Carboxy oder Sulfophenyl substituierten $C_2$-$C_4$-Alkylenrest, $-CH_2-CH_2-Z'-CH_2-CH_2-$ worin Z' -O-, -S-, $-SO_2-$, -NH- oder $-N(CH_3)-$ bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder für einen $C_1$-$C_3$-Alkylen-phenylen- oder $C_1$-$C_2$-Alkylen-phenylen-$C_1$-$C_2$-alkylenrest, worin das Phenylen jeweils unsubstituiert oder durch Methyl, Methoxy, Chlor oder Sulfo substituiert ist, steht,
X eine Gruppe $-N(R_5)-$, worin $R_5$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet, ist,
oder worin die Gruppe

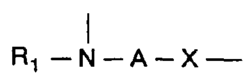

den Piperazin-1,4-diylrest bedeutet,
Y Methoxy, Methyl, Chlor oder Sulfo ist, n für die Zahl 0 oder 1 steht, Z Hydroxy, Methyl oder Ethyl bedeutet, p die Zahl 1 darstellt, $R_2$ und $R_3$ jeweils Wasserstoff, Fluor, Chlor, Brom, Methyl, Methoxy, Acetylamino, Phenoxy oder Cyano bedeuten, das Brückenglied B der Formel

$$Q'\text{-}E'Q' \qquad (3'),$$

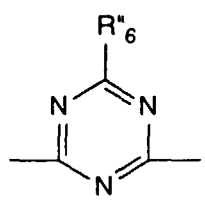

(4'),

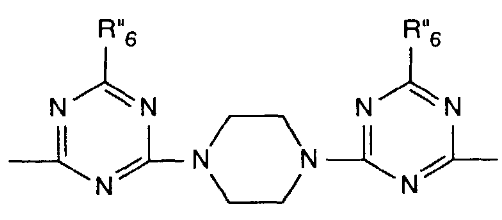

(6'),

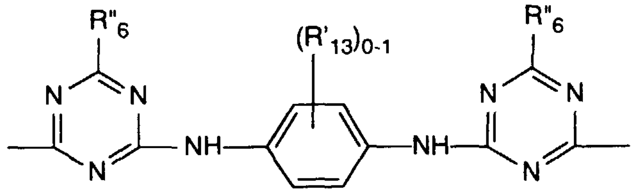

(7')

oder

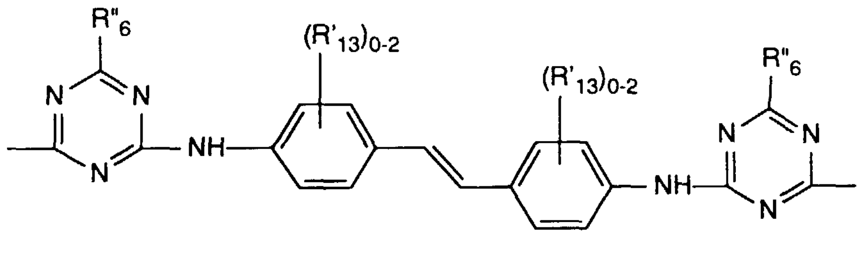

(8'),

worin Q' eine Gruppe

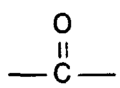 , 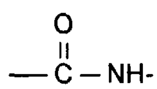 , 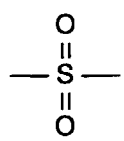 oder 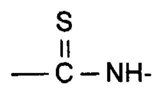

bedeutet, E' für die direkte Bindung, unsubstituiertes oder durch Hydroxy, Sulfo, Sulfato, Methoxy, Carboxy, Phenyl oder Sulfophenyl substituiertes $C_1$-$C_6$-Alkylen oder $C_2$-$C_6$-Alkenylen, unsubstituiertes oder durch 1 bis 3 Methylgruppen substituiertes Cyclohexylen oder $C_1$-$C_2$-Alkylen-cyclohexylen, Piperazin-1,4-diyl, Thiophen-2,5-diyl, Biphenyl-4,4'-diyl, Stilben-4,4'-diyl, unsubstituiertes oder durch $C_1$-$C_4$-Alkyl, $C_1$-$C_4$-Alkoxy, Sulfo, Halogen oder Carboxy substituiertes Phenylen oder Naphthylen oder für unsubstituiertes oder im Phenylteil durch Methyl, Methoxy, Chlor oder Sulfo substituiertes $C_1$-$C_3$-Alkylen-phenylen oder $C_1$-$C_2$-Alkylen-phenylen-$C_1$-$C_2$-alkylen steht, $R''_6$ Chlor, Hydroxy, $C_1$-$C_4$-Alkoxy, $C_1$-$C_2$-Alkylthio, Amino, unsubstituiertes oder im Alkylteil durch Hydroxy, Sulfo oder Sulfato substituiertes N-Mono- oder N,N-Di-$C_1$-$C_4$-Alkylamino, Cyclohexylamino, unsubstituiertes oder im Phenylteil durch Methyl, Methoxy, Carboxy, Sulfo oder Chlor substituiertes Phenylamino oder N-$C_1$-$C_4$-Alkyl-N-phenylamino oder Morpholino bedeutet und $R'_{13}$ Sulfo, Methyl oder Methoxy bedeutet, entspricht und m eine beliebige Zahl von 1 bis 3 bedeutet.

Von besonderer Bedeutung wegen ihrer guten färberischen Eigenschaften sind Oligomerengemische der Verbindungen der Formel

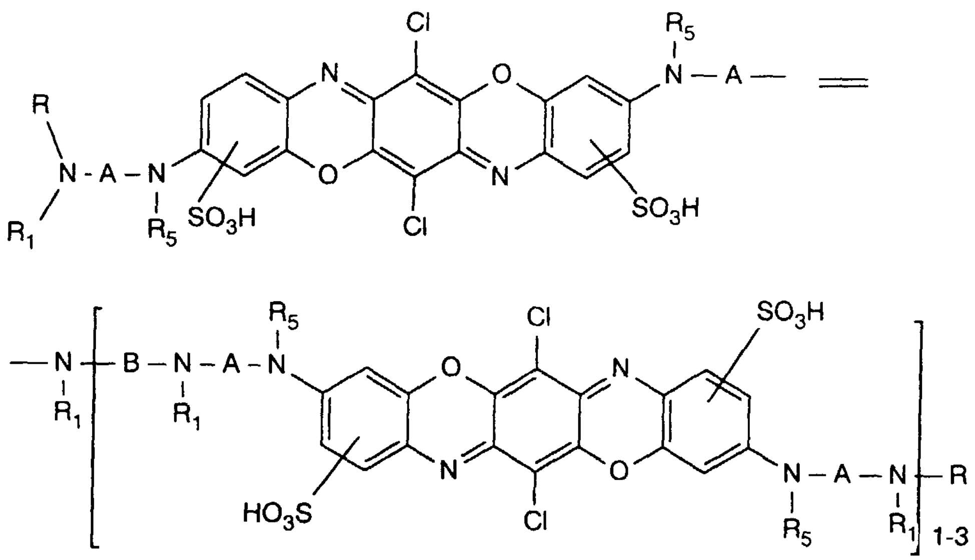

(1a),

worin $R_1$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet,
R für Wasserstoff, Methyl, Ethyl, Benzyl, Acetylamino oder Benzoylamino steht, A einen unsubstituierten oder durch Hydroxy, Sulfo, Sulfato, Methoxy, Carboxy oder Sulfophenyl substituierten $C_2$-$C_4$-Alkylenrest darstellt, $R_5$ Wasserstoff, Methyl oder Ethyl bedeutet, und B für einen Rest der Formel

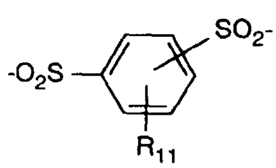
,
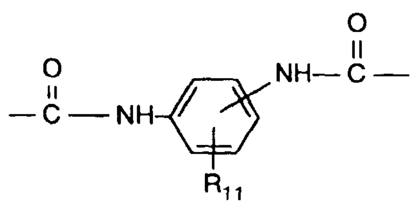
,
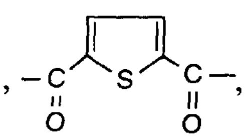
,

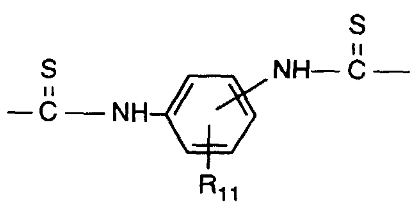
,
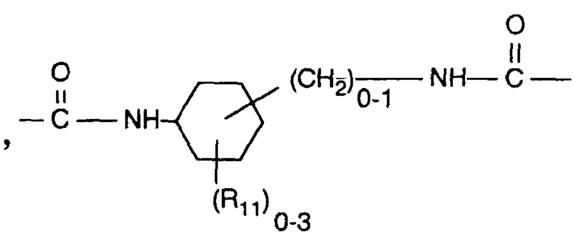
,

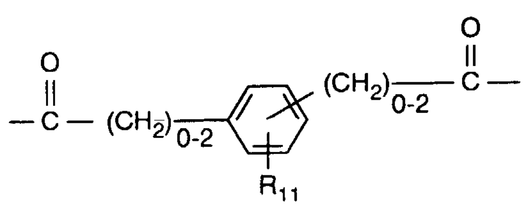
,
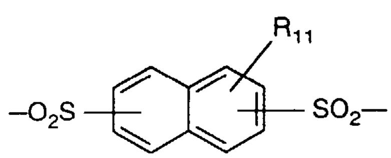
,

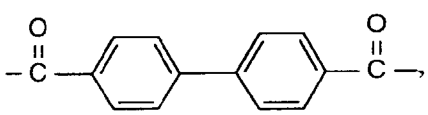, 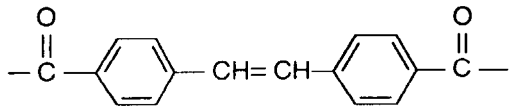,

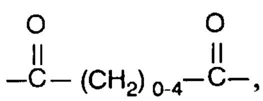, 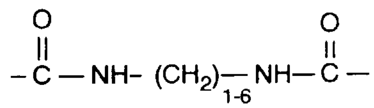, 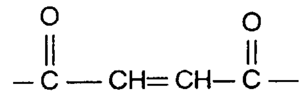

oder

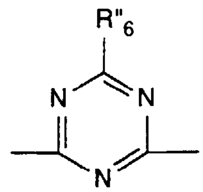,

worin $R_{11}$ Sulfo, Methyl, Methoxy, Chlor, Carboxy oder vorzugsweise Wasserstoff bedeutet und $R''_6$ Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m-, oder p-Methoxyphenylamino, o-, m- oder p-Chlorphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder Morpholino ist, steht.

Eine besonders bevorzugte Ausführungsform der vorliegenden Erfindung betrifft Oligomerengemische der Verbindungen der Formel

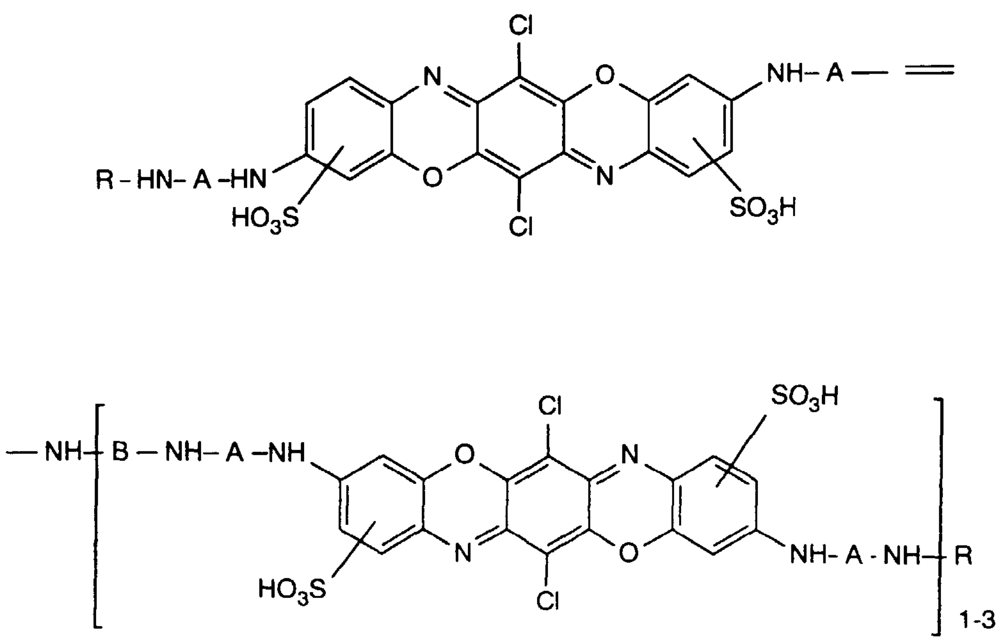

(1b),

worin R Methyl, Ethyl, Benzyl, Acetylamino, Benzoylamino oder insbesondere Wasserstoff bedeutet, A für einen unsubstituierten oder durch Hydroxy oder Sulfato substituierten 1,2-Ethylen- oder 1,2- oder 1,3-Propylenrest steht und B ein Rest der Formel

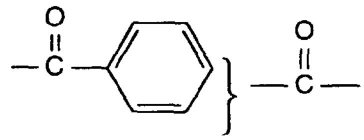

oder

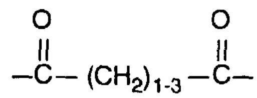

ist.

Die Oligomerengemische der Verbindungen der Formel (1) können erhalten werden, z.B. indem man a) eine Verbindung der Formel

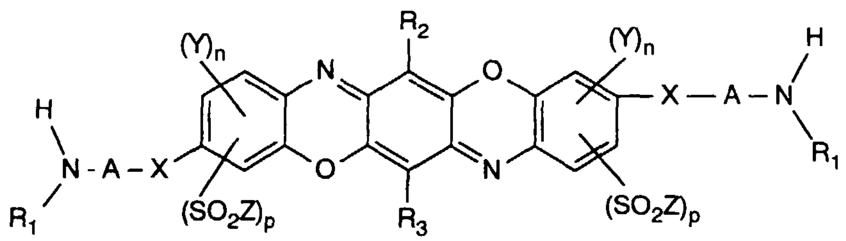

(9),

mit einer Verbindung der Formel

T-B-T (10),

kondensiert und gegebenenfalls b) das gemäss a) erhaltene Produkt mit einer Verbindung der Formel

R*-T (11),

umsetzt, worin A, B, $R_1$, $R_2$, $R_3$, X, Y, Z, p und n jeweils die zuvor angegebene Bedeutung haben, R* die zuvor für R angegebene Bedeutung mit Ausnahme von Wasserstoff hat und T Halogen, vorzugsweise Chlor, ist.

Bevorzugte Oligomerengemische enthalten nebeneinander die drei Verbindungen der Formel (1), worin m 1, 2 und 3 ist.

Die Kondensationsreaktionen a) und b) werden vorteilhaft jeweils in einem wässrigen oder wässrig-organischem Medium bei einer Temperatur von z.B. 0 bis 100°C und vorzugsweise 0 bis 50°C, durchgeführt. Man arbeitet zweckmässig bei einem neutralen bis alkalischen pH-Wert, d.h. bei einem pH-Wert von z.B. 7 bis 13 und vorzugsweise 8 bis 12; der pH-Wert kann durch Zugabe von Basen, z.B. Alkalimetallhydroxiden oder -carbonaten, Ammoniak oder organischen Aminen eingestellt und während der Kondensationsreaktionen konstant gehalten werden.

Man erhält abhängig vom Gewichtsverhältnis, in dem die Verbindungen der Formeln (9) und (10) eingesetzt werden, ein Oligomerengemisch bestehend aus verschiedenen Verbindungen der Formel (1), die sich im Wert von m unterscheiden. Das Oligomerengemisch besteht z.B. aus den sechs Verbindungen der Formel (1) mit m = 1, 2, 3, 4, 5 und 6, wobei im allgemeinen die drei Verbindungen der Formel (1) mit m = 1, 2 oder 3 anteilsmässig am stärksten vertreten sind.

Gemäss Kondensationsschritt a) werden die Verbindungen der Formel (1) mit R = Wasserstoff erhalten; die Umwandlung des Wasserstoffatoms in einen beliebigen Rest R erfolgt durch Umsetzung mit einer Verbindung der Formel (11) in an sich bekannter Weise.

Die Verbindungen der Formeln (9), (10) und (11) sind bekannt oder können in an sich bekannter Weise erhalten

werden.

Ein weiterer Gegenstand der Erfindung ist die Verwendung der Oligomerengemische enthaltend verschiedene Verbindungen der Formel (1), die sich im Wert von m unterscheiden, als Farbstoffe zum Färben oder Bedrucken von stickstoffhaltigen und insbesondere hydroxylgruppenhaltigen Fasermaterialien.

Die erfindungsgemässen Gemische verschiedener oligomerer Verbindungen der Formel (1) eignen sich also zum Färben und Bedrucken von stickstoffhaltigen oder insbesondere von cellulosischen Fasermaterialien, vorzugsweise von textilen Fasermaterialien, aus Seide, Wolle oder synthetischen Polyamiden, sowie bevorzugt aus den cellulosischen Fasern, wie Rayon, Baumwolle oder Hanf.

Bezüglich ihrer färberischen Eigenschaften können sie als direktziehende oder Direktfarbstoffe (C.I. direct dyes) bezeichnet werden.

Ebenfalls können textile Fasermaterialien aus Mischfasern, wie z.B. aus Wolle/Baumwoll-, Polyamid/Baumwoll-, Polyacryl/Baumwoll- oder insbesondere Polyester/Baumwoll-Mischfasern durch Einbad-Färbeverfahren und in Gegenwart von Farbstoffen für die jeweils anderen Fasertypen gefärbt werden.

Die textilen Fasermaterialien können in den verschiedensten Verarbeitungszuständen vorliegen, wie z.B. als Faser, Garn, Gewebe oder Gewirke. Neben den textilen Substraten können auch Leder und Papier mit den erfindungsgemässen Farbstoffgemischen gefärbt werden.

Man erhält egale Färbungen in blauen Farbtönen mit guten Allgemeinechtheiten, insbesondere guten Reib-, Nass-, Nassreib-, Schweiss- und Lichtechtheiten. Sofern nötig, kann man die Nassechtheiten, insbesondere die Waschechtheit, der erhaltenen Direktfärbungen und -drucke durch eine Nachbehandlung mit sog. Fixiermitteln noch wesentlich verbessern.

Die erfindungsgemässen Gemische verschiedener oligomerer Verbindungen der Formel (1) sind gut mit anderen Farbstoffen, insbesondere Dispersionsfarbstoffen kombinierbar. Die erfindungsgemässen Farbstoffgemische weisen eine ausreichende Hochtemperatur-Stabilität auf und lassen sich so unter den Färbebedingungen für Polyesterfasern, d.h. bei Temperaturen im Bereich von etwa 100 bis 150°C, vorzugsweise von 100 bis 130°C, aus wässriger Flotte und bei einem pH-Wert von 4 bis 7,5, vorzugsweise 5 bis 7, färben.

Damit ist es möglich, übliche Dispersionsfarbstoffe zusammen mit den erfindungsgemässen Farbstoffgemischen in einem einstufigen, einbadigen Verfahren zum Färben von Polyester/Baumwoll-Mischfasern (Mischgewebe) einzusetzen, wobei beide Faserarten gleichmässig und echt durch den jeweiligen Farbstoff angefärbt werden. Verwendet man einen Dispersionsfarbstoff mit gleicher Nuance wie das erfindungsgemässe Farbstoffgemisch sie aufweist, so ist es auch möglich Ton-in-Ton-Färbungen zu erhalten.

Mit der Bereitstellung der erfindungsgemässen Farbstoffgemische kann man das Färben von textilen Mischfasern (Mischgeweben), z.B. solchen aus Polyester- und Cellulosefasern, wesentlich vereinfachen. Die an sich übliche Färbung jeder Faserart einer Fasermischung in einem separaten Arbeitsgang unter Anwendung unterschiedlicher Färbebedingungen ist damit nicht mehr nötig.

Die erfindungsgemässen Gemische verschiedener oligomerer Verbindungen der Formel (1) eignen sich auch zur Herstellung von wässrigen Tinten für den Tintenstrahldruck (ink-jet printing).

Das folgende Beispiel dient zur Erläuterung der Erfindung. Teile und Prozente beziehen sich auf das Gewicht, sofern nicht anders angegeben. Gewichtsteile und Volumenteile stehen in gleicher Beziehung zueinander wie Kilogramm und Liter. Die Temperaturen sind in Celsiusgraden angegeben.

Beispiel 1:

a) 96 Teile 4-(2-Aminoethylamino)-anilin-3-sulfonsäure werden in eine Mischung von 3000 Teilen Wasser und 600 Teilen Isopropanol suspendiert und die Suspension mit 13 Teilen eines Phosphatpuffergemisches und anschliessender Zugabe von 14 Teilen einer wässrigen 4-normalen Salzsäurelösung auf einen pH-Wert von 5,9 gestellt. Anschliessend wird auf eine Temperatur von 50° aufgeheizt. Es werden 49,52 Teile Chloranil zugegeben und die Mischung drei Stunden gerührt, wobei der pH durch Zugabe einer 2-normalen Kaliumhydrogencarbonatlösung bei einem Wert von 6 gehalten wird. Die Mischung wird auf eine Temperatur von ca. 40° abgekühlt, das erhaltene Produkt abgenutscht, zweimal mit je 200 Teilen einer wässrigen Natriumchloridlösung und dann einmal mit 200 Teilen Aceton gewaschen. Nach Trocknung bei einer Temperatur von 40° erhält man eine Verbindung der Formel

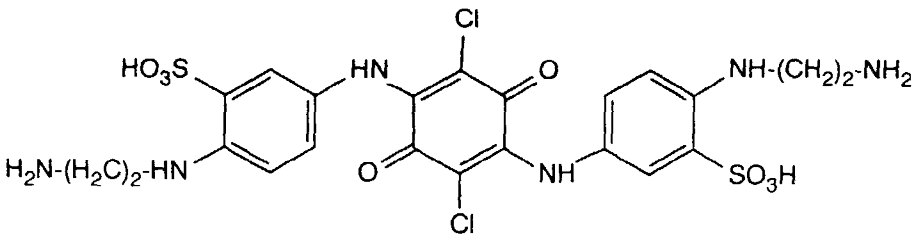

(101).

b) 900 Teile Oleum (25%) werden vorgelegt und innerhalb von drei Stunden werden 166,2 Teile der wie oben unter a) angegeben erhaltenen Verbindung der Formel (101) bei einer Temperatur von 0 bis 5° in gleichmässigen Portionen eingetragen. Anschliessend wird 30 Minuten bei Raumtemperatur nachgerührt. Dann werden innerhalb von 90 Minuten bei einer Temperatur von 20 bis 30° 118,95 Teile Kaliumperoxodisulfat eingetragen und es wird 75 Minuten nachgerührt. Das Reaktionsgemisch wird auf ein Gemisch von 3000 Teilen Eis und 600 Teilen Wasser ausgetragen, wobei die Temperatur 30° nicht übersteigen sollte. Das erhaltene Produkt wird abgenutscht, das feuchte Nutschgut in 2000 Teilen Wasser verrührt und mit 1400 Teilen Natriumhydroxidlösung (30%) auf einen pH-Wert von 7 gestellt. Es wird über Nacht ausgerührt, das Produkt abgenutscht, dreimal mit je 100 Teilen Wasser und anschliessend mit 100 Teilen Ethanol gewaschen. Nach Trocknung erhält man die Verbindung der Formel

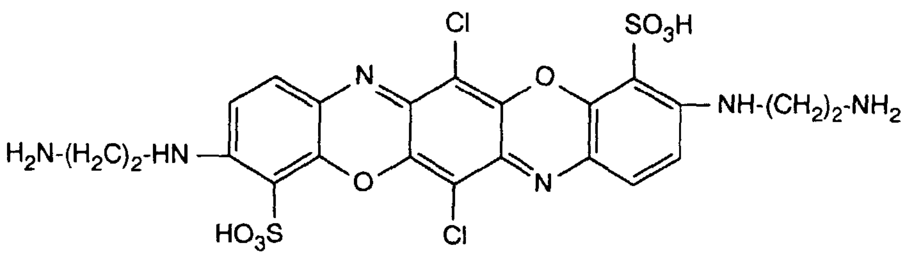

(102).

c) 13,4 Teile der wie oben gemäss Schritt b) erhältlichen Verbindung der Formel (102) werden in 3000 Teilen Wasser unter Zugabe von Natriumhydroxidlösung suspendiert. Zu dieser Suspension, die einen pH-Wert von ca. 11 aufweist, werden innerhalb von ca. 30 Minuten 10,4 Teile Terephthalsäuredichlorid gelöst in ca. 150 Teilen Dioxan bei 0 bis 5° zugetropft und dabei der pH-Wert durch Zugabe von Natriumhydroxidlösung bei 11 gehalten. Man lässt 1 Stunde ausreagieren und gibt dann weitere 10,4 Teile Terephthalsäuredichlorid gelöst in ca. 150 Teilen Dioxan bei 0 bis 5° zu, wobei der pH-Wert durch Zugabe von Natriumhydroxidlösung bei ca. 12 gehalten wird. Man lässt wiederum ausreagieren, stellt dann den pH-Wert mit Salzsäure in etwa neutral ein und lässt weitere 30 Minuten rühren.

Danach wird das Produkt abfiltriert und getrocknet; es handelt sich um ein Gemisch oligomerer Verbindungen der Formel (1), worin R und $R_1$ je Wasserstoff, A 1,2-Ethylen, X -NH-, n 0, Z Hydroxy, p 1, $R_2$ und $R_3$ je Chlor und B ein Rest der Formel

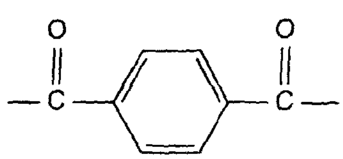

bedeuten, und welches Baumwolle in einer blauen Nuance mit guten Allgemeinechtheiten färbt.

Beispiele 2 bis 11: Verfährt man wie in Beispiel 1 angegeben, verwendet jedoch in Schritt a) des Beispiels 1 anstelle von 96 Teilen 4-(2-Aminoethylamino)-anilin-3-sulfonsäure eine äquimolare Menge einer der in der folgenden Tabelle 1 in Spalte 2 angegebenen Anilinoverbindungen, so erhält man analoge Gemische von Farbstoffen, die Baumwolle in einer brillanten blauen Nuance mit guten Allgemeinechtheiten färben.

Tabelle 1

Bsp. Anilinoverbindung

2 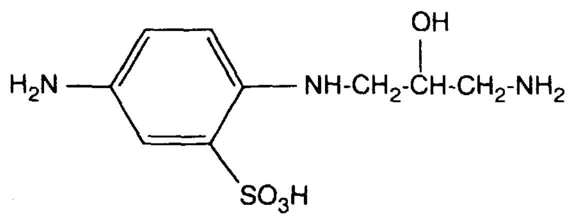

3 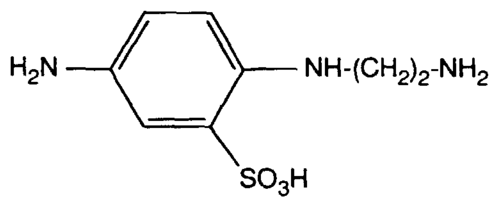

4 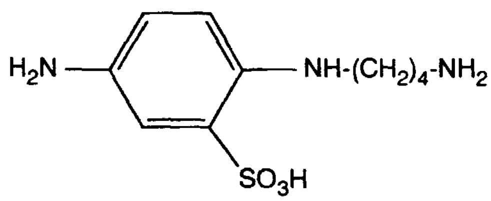

5 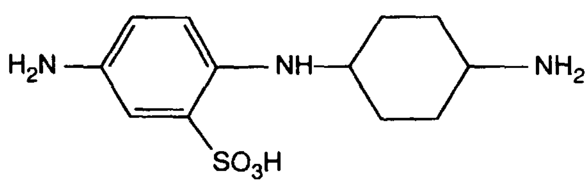

6 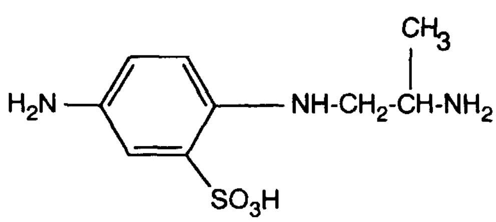

7 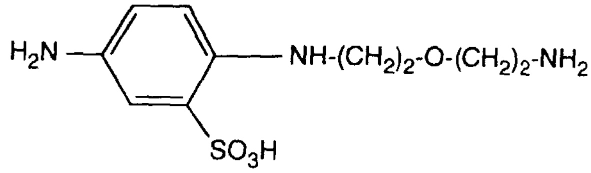

8 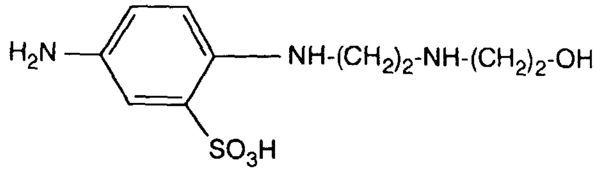

9 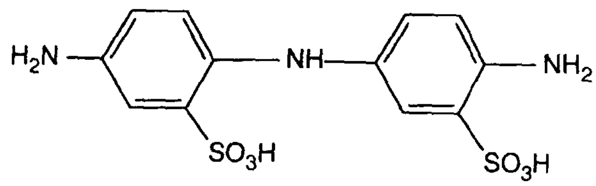

10 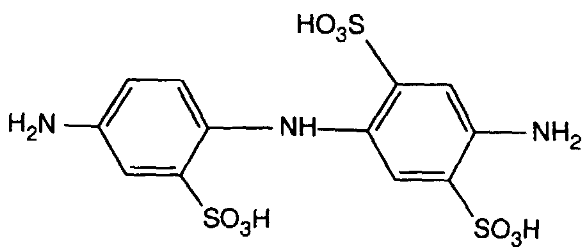

11 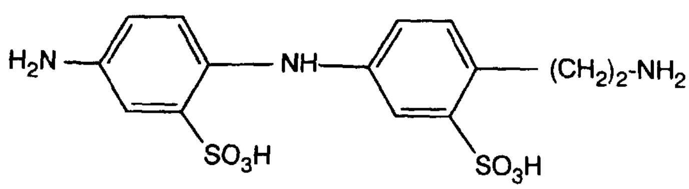

Beispiele 12 bis 23: Verfährt man wie in Beispiel 1 angegeben, verwendet jedoch in Schritt c) des Beispiels 1 anstelle von 10,4 Teile Terephthalsäuredichlorid eine äquimolare Menge einer der in der folgenden Tabelle 2 in Spalte 2 angegebenen Dicarbonylchlorid- bzw. Disulfonylchloridverbindungen, so erhält man analoge Gemische von Farbstoffen, die Baumwolle in einer brillanten blauen Nuance mit guten Allgemeinechtheiten färben.

Tabelle 2

| Bsp. | Dicarbonylchlorid- bzw. Disulfonylchloridverbindungen |
|---|---|
| 12 | 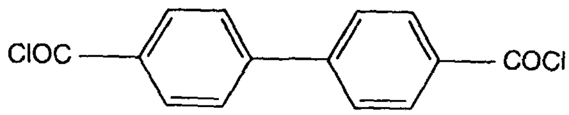 |
| 13 | 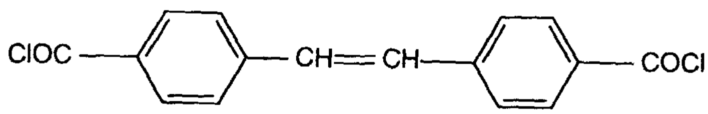 |
| 14 | 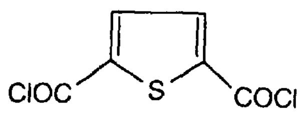 |
| 15 | 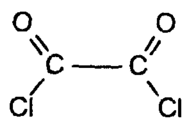 |
| 16 | 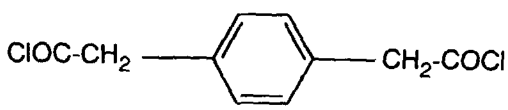 |
| 17 | 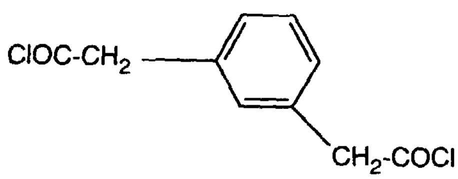 |
| 18 | 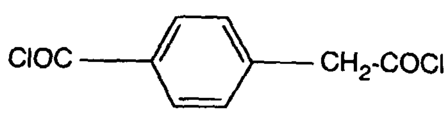 |
| 19 | $ClOC-CH=CH-COCl$ |
| 20 | $ClOC-(CH_2)_4-COCl$ |

21 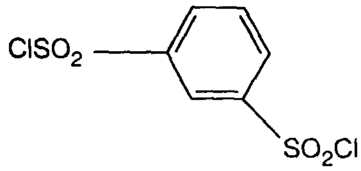

22 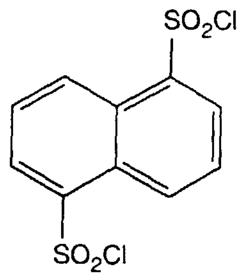

23 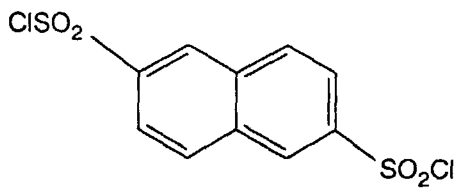

Beispiel 24: 9,74 Teile der wie in Beispiel 1 angegeben erhältlichen Verbindung der Formel (102) werden in 100 Teilen Wasser suspendiert und mit 4-normaler Lithiumhydroxidlösung auf einen pH-Wert von 11,5 bis 12 gestellt. Die Mischung wird auf eine Temperatur von 15° abgekühlt, und es werden 0,93 Teile 2,4-Toluylendiisocyanat, gelöst in 10 Teilen Dioxan, unter gutem Rühren zugetropft. Nach beendeter Reaktion wird durch Zugabe von Salzsäure sauergestellt, das ausgefallene Produkt abgenutscht und mit wässriger Natriumchloridlösung gewaschen. Man erhält 4 Teile eines Gemisches oligomerer Verbindungen der Formel (1), worin R und $R_1$ je Wasserstoff, A 1,2-Ethylen, X -NH-, n 0, Z Hydroxy, p 1, $R_2$ und $R_3$ je Chlor und B ein Rest der Formel

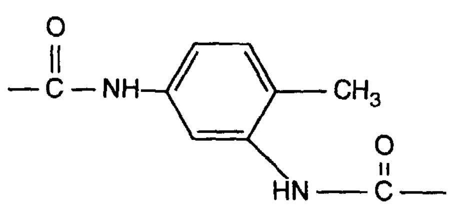

bedeuten, und welches Baumwolle in einer blauen Nuance mit guten Allgemeinechtheiten färbt.

Beispiele 25 bis 30: Verfährt man wie in Beispiel 24 angegeben, verwendet jedoch anstelle von 0,93 Teile 2,4-Toluylendiisocyanat eine äquimolare Menge einer der in der folgenden Tabelle 3 in Spalte 2 angegebenen Isocyano- bzw. Isothiocyanoverbindungen, so erhält man analoge Gemische von Farbstoffen, die Baumwolle in einer brillanten blauen Nuance mit guten Allgemeinechtheiten färben.

Tabelle 3

| Bsp. | Isocyano- bzw. Isothiocyanoverbindungen |
|---|---|
| 25 | 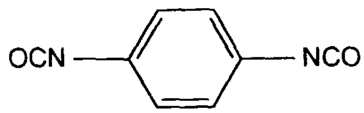 |
| 26 | 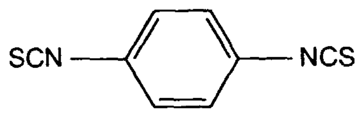 |
| 27 | 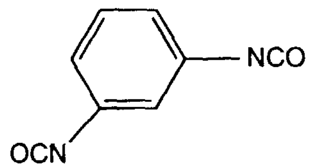 |
| 28 | 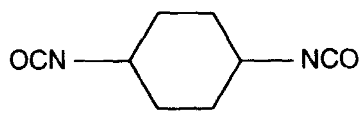 |
| 29 | 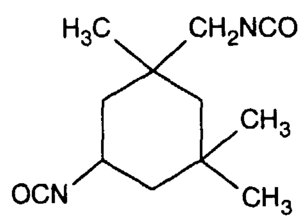 |
| 30 | $OCN-(CH_2)_6-NCO$ |

Färbevorschrift I

12,5 Teile eines nicht mercerisierten, ungebleichten Baumwollgewebes werden mit einem Teil eines nichtionogenen Netzmittels bei einer Temperatur von 80° eingenetzt. Das Baumwollgewebe wird in eine Farbstofflösung, welche 2% des gemäss Schritt c) des Beispiels 1 erhaltenen Farbstoffgemisches und 2 g/l Natriumsulfat enthält, bei einem Flottenverhältnis von 1:20 eingetragen. Anschliessend wird das Färbebad innerhalb von 30 Minuten auf eine Temperatur von 95° erhitzt, es werden 8 g/l Natriumsulfat zugegeben, das Färbebad 45 Minuten bei der Temperatur von 95° belassen, innerhalb von 15 Minuten auf eine Temperatur von 80° abgekühlt und weitere 15 Minuten bei dieser Temperatur belassen. Nach Spülung mit Wasser und Trocknung erhält man ein in einer reinblauen Nuance gefärbtes Baumwollgewebe, welches gute Allgemeinechtheiten aufweist.

Färbevorschrift II

12,5 Teile Polyamid-6.6-Gewebe werden bei einer Temperatur von 40° in ein Färbebad, welches durch Zugabe von 2 g/l eines Phosphatpuffers auf pH 6 gestellt ist, gegeben. Das Flottenverhältnis beträgt 1:20. Nach 10 Minuten

werden 2% des gemäss Schritt c) des Beispiels 1 erhaltenen Farbstoffgemisches zugegeben und das Färbebad innerhalb von 45 Minuten zum Sieden erhitzt und 45 Minuten bei dieser Temperatur belassen. Nach Spülung mit Wasser und Trocknung erhält man ein in einer reinblauen Nuance gefärbtes Polyamid-6.6-Gewebe, welches gute Allgemeinechtheiten aufweist.

**Patentansprüche**

**Patentansprüche für folgende Vertragsstaaten : BE, CH, DE, FR, GB, IT, LI**

1. Oligomerengemisch enthaltend mindestens zwei Verbindungen der Formel

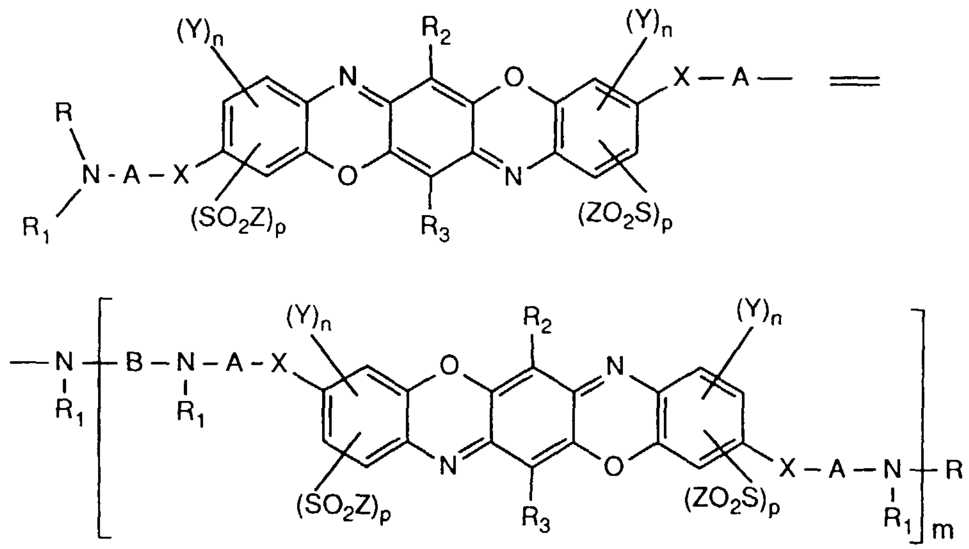

(1),

worin
$R_1$ für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes $C_1$-$C_4$-Alkyl, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl steht,
R unabhängig die Bedeutung von $R_1$ hat, oder
ein Pyridin-, Pyrimidin-, Chinoxalin oder Triazinrest ist, wobei diese Reste unsubstituiert oder durch Hydroxy; $C_1$-$C_4$-Alkyl; Phenyl; $C_1$-$C_4$-Alkoxy; $C_1$-$C_4$-Alkylthio; Amino; unsubstituiertes oder im Alkylteil durch Hydroxy, Carboxy, Cyano, Sulfo, Sulfato oder $C_1$-$C_4$-Alkoxy substituiertes N-Mono- oder N,N-Di-$C_1$-$C_4$-Alkylamino; Cyclohexylamino; unsubstituiertes oder im Phenylteil durch $C_1$-$C_4$-Alky-, $C_1$-$C_4$-Alkoxy, Phenoxy, Carboxy, Sulfo und/oder Halogen substituiertes Phenylamino oder N-$C_1$-$C_4$-Alkyl-N-phenylamino; Morpholino; oder 3-Carboxy- oder 3-Carbamoylpyridin-1-yl; sind, oder
R ein Rest der Formel

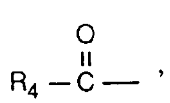

ist, worin $R_4$ ein Chinoxalin- oder Pyrimidinrest ist oder die oben für $R_1$ angegebenen Bedeutungen hat, jedoch nicht Wasserstoff bedeutet,

A ein unsubstituierter oder durch Hydroxy, Sulfo, Sulfato, $C_1$-$C_4$-Alkoxy, Carboxy, Cyano, Halogen, Phenyl, Sulfophenyl oder $C_2$-$C_5$-Alkoxycarbonyl substituierter und/oder gegebenenfalls durch 1-2 Gruppen -O-, -N($R_8$)-, worin $R_8$ $C_1$-$C_4$-Alkyl, Acetyl oder insbesondere Wasserstoff bedeutet, -S- oder -$SO_2$- unterbrochener $C_2$-$C_6$-Alkylenrest; unsubstituiertes oder einfach oder mehrfach durch $C_1$-$C_3$-Alkyl substituiertes Cyclopentylen oder Cyclohexylen; ein unsubstituierter oder durch $C_1$-$C_4$-Alkyl, $C_1$-$C_4$-Alkoxy, Sulfo, Halogen oder Carboxy substituierter Phenylen-, Biphenylen- oder Naphthylenrest; oder

ein $C_1$-$C_6$-Alkylen-phenylen-, Phenylen-$C_1$-$C_6$-alkylen-phenylen-, $C_1$-$C_3$-Alkylenphenylen-$C_1$-$C_3$-alkylen- oder ein Methylen-naphthylen-methylenrest ist, wobei in diesen Aralkylenresten das Phenylen und Naphthylen gegebenenfalls noch 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino tragen kann,

X -O-, -S- oder -N($R_5$)- ist, worin $R_5$ die oben für $R_1$ angegebenen Bedeutungen hat, oder worin die Gruppe

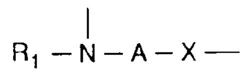

ein Piperazin-1,4-diylrest ist,

Y $C_1$-$C_4$-Alkyl, $C_1$-$C_4$-Alkoxy, Halogen, Sulfo, Carboxy, Carbamoyl, N-Mono- oder N,N-Di-$C_1$-$C_4$-Alkylcarbamoyl, N-Phenyl- oder N,N-Diphenylcarbamoyl, Sulfamoyl, N-Mono- oder N,N-Di-$C_1$-$C_4$-Alkylsulfamoyl oder N-Phenyl- oder N,N-Diphenylsulfamoyl ist,

Z Hydroxy oder $C_1$-$C_4$-Alkyl ist,

$R_2$ und $R_3$ unabhängig voneinander je Wasserstoff; Halogen; Cyano; $C_1$-$C_4$-Alkyl; $C_1$-$C_4$-Alkoxy; Sulfo; Carboxy; Carbamoyl; Phenylcarbamoyl; $C_2$-$C_5$-Alkanoylamino;

oder gegebenenfalls durch $C_1$-$C_4$-Alkyl, $C_1$-$C_4$-Alkoxy, Acetylamino, Halogen, Nitro oder Sulfo substituiertes Phenyl, Benzyl, Benzoylamino oder Phenoxy sind,

B ein Brückenglied der Formel

$$Q'\text{-}E'\text{-}Q' \qquad (3'),$$

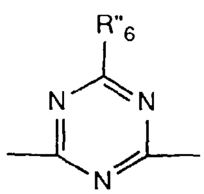

(4'),

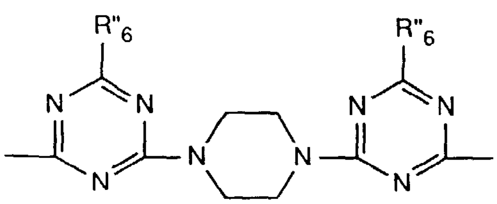

(6'),

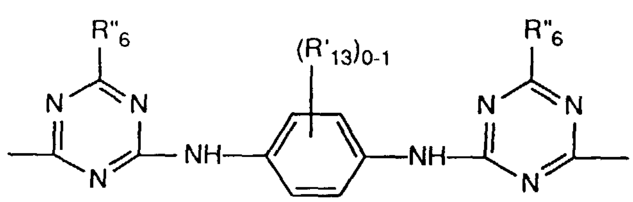

(7')

oder

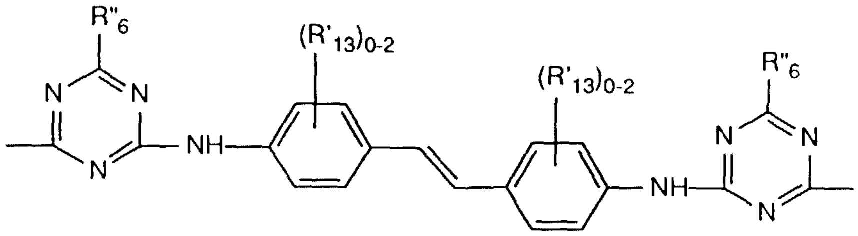

(8)

ist,
worin Q' eine Gruppe

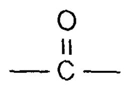 , 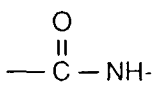 , 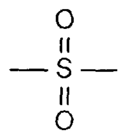 oder 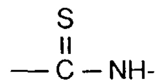

bedeutet,
E' für die direkte Bindung;
unsubstituiertes oder durch Hydroxy, Sulfo, Sulfato, Methoxy, Carboxy, Phenyl oder Sulfophenyl substituiertes $C_1$-$C_6$-Alkylen oder $C_2$-$C_6$-Alkenylen;
unsubstituiertes oder durch 1 bis 3 Methylgruppen substituiertes Cyclohexylen oder $C_1$-$C_2$-Alkylen-cyclohexylen; Piperazin-1,4-diyl; Thiophen-2,5-diyl; Biphenyl-4,4'-diyl; Stilben-4,4'-diyl;
unsubstituiertes oder durch $C_1$-$C_4$-Alkyl, $C_1$-$C_4$-Alkoxy, Sulfo, Halogen oder Carboxy substituiertes Phenylen oder Naphthylen; oder für
unsubstituiertes oder im Phenylteil durch Methyl, Methoxy, Chlor oder Sulfo substituiertes $C_1$-$C_3$-Alkylen-phenylen oder $C_1$-$C_2$-Alkylen-phenylen-$C_1$-$C_2$-alkylen steht, $R''_6$ Chlor; Hydroxy; $C_1$-$C_4$-Alkoxy; $C_1$-$C_2$-Alkylthio; Amino; unsubstituiertes oder im Alkylteil durch Hydroxy, Sulfo oder Sulfato substituiertes N-Mono- oder N,N-Di-$C_1$-$C_4$-Alkylamino; Cyclohexylamino; unsubstituiertes oder im Phenylteil durch Methyl, Methoxy, Carboxy, Sulfo oder Chlor substituiertes Phenylamino oder
N-$C_1$-$C_4$-Alkyl-N-phenylamino; oder Morpholino bedeutet und $R'_{13}$ Sulfo, Methyl oder Methoxy bedeutet,
m eine Zahl von 1 bis 6 bedeutet und
n und p unabhängig voneinander je für die Zahl 0 oder 1 stehen,
wobei sich die verschiedenen Verbindungen der Formel (1) ausschliesslich im Wert von m unterscheiden.

2. Oligomerengemisch gemäss Anspruch 1, dadurch gekennzeichnet, dass $R_1$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet.

3. Oligomerengemisch gemäss einem der Ansprüche 1 und 2, dadurch gekennzeichnet, dass $R_1$ Wasserstoff ist.

4. Oligomerengemisch gemäss einem der Ansprüche 1 bis 3, dadurch gekennzeichnet, dass R Wasserstoff, $C_1$-$C_4$-Alkyl, unsubstituiertes oder durch Methyl, Methoxy, Chlor und/oder Sulfo substituiertes Phenyl oder Benzyl oder einen Rest der Formel

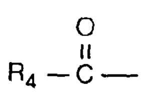 ,

worin $R_4$ für Methyl, Ethyl oder unsubstituiertes oder durch Sulfo, Chlor, Methyl und/oder Methoxy substituiertes Phenyl steht, bedeutet.

5. Oligomerengemisch gemäss einem der Ansprüche 1 bis 4, dadurch gekennzeichnet, dass R Wasserstoff, Methyl, Ethyl, Benzyl, Acetylamino oder Benzoylamino, besonders Wasserstoff, bedeutet.

6. Oligomerengemisch gemäss einem der Ansprüche 1 bis 5, dadurch gekennzeichnet, dass A einen unsubstituierten oder durch Hydroxy, Sulfo, Sulfato, Methoxy, Carboxy oder Sulfophenyl substituierten $C_2$-$C_4$-Alkylenrest, $-CH_2-CH_2-Z'-CH_2-CH_2-$ worin Z' -O-, -S-, $-SO_2-$, -NH- oder $-N(CH_3)-$ bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder einen $C_1$-$C_3$-Alkylen-phenylen- oder $C_1$-$C_2$-Alkylen-phenylen-$C_1$-$C_2$-alkylenrest, worin das Phenylen jeweils unsubstituiert oder durch Methyl, Methoxy, Chlor oder Sulfo substituiert ist, bedeutet.

7. Oligomerengemisch gemäss einem der Ansprüche 1 bis 6, dadurch gekennzeichnet, dass A unsubstituiertes oder durch Hydroxy, Sulfo, Sulfato, Methoxy, Carboxy oder Sulfophenyl substituiertes $C_2$-$C_4$-Alkylen bedeutet.

8. Oligomerengemisch gemäss einem der Ansprüche 1 bis 7, dadurch gekennzeichnet, dass A unsubstituiertes oder durch Hydroxy oder Sulfato substituiertes 1,2-Ethylen oder 1,2- oder 1,3-Propylen bedeutet.

9. Oligomerengemisch gemäss einem der Ansprüche 1 bis 8, dadurch gekennzeichnet, dass X für die Gruppe $-N(R_5)-$, worin $R_5$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet, steht.

10. Oligomerengemisch gemäss Anspruch 9, dadurch gekennzeichnet, dass $R_5$ Wasserstoff oder $C_1$-$C_4$-Alkyl, besonders Wasserstoff, ist.

11. Oligomerengemisch gemäss einem der Ansprüche 1 bis 10, dadurch gekennzeichnet, dass n für die Zahl 0 steht.

12. Oligomerengemisch gemäss einem der Ansprüche 1 bis 11, dadurch gekennzeichnet, dass p für die Zahl 1 steht und Z Hydroxy oder $C_1$-$C_4$-Alkyl, besonders Hydroxy, bedeutet.

13. Oligomerengemisch gemäss einem der Ansprüche 1 bis 12, dadurch gekennzeichnet, dass $R_2$ und $R_3$ jeweils Wasserstoff, Fluor, Chlor, Brom, Methyl, Methoxy, Acetylamino, Phenoxy oder Cyano bedeuten.

14. Oligomerengemisch gemäss einem der Ansprüche 1 bis 13, dadurch gekennzeichnet, dass $R_2$ und $R_3$ je Chlor bedeuten.

15. Oligomerengemisch gemäss einem der Ansprüche 1 bis 14, dadurch gekennzeichnet, dass B ein Brückenglied der Formel

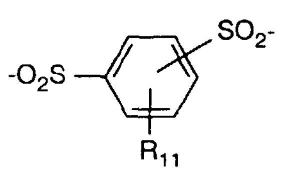
,
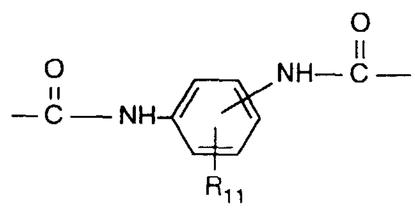
,
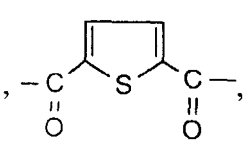
,

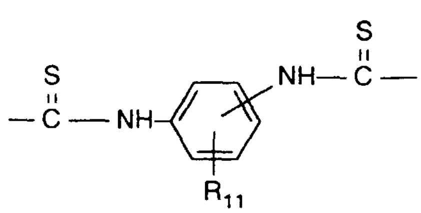
,
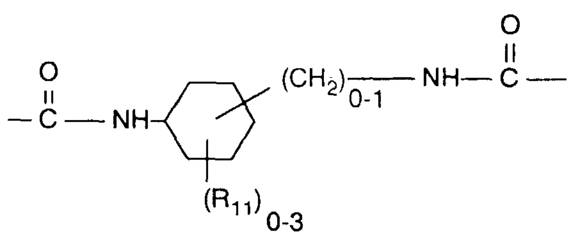
,

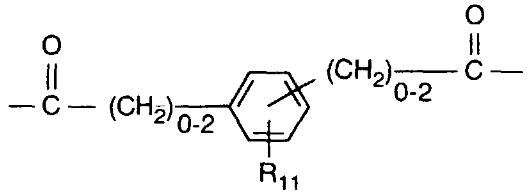 , 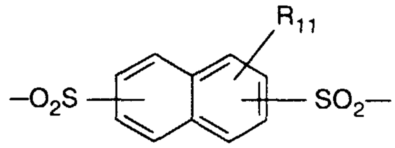 ,

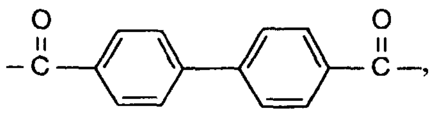 , 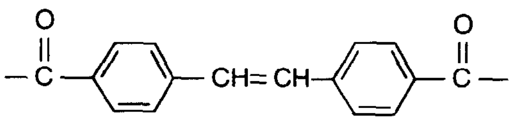 ,

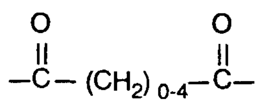 , 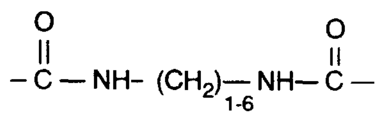 , 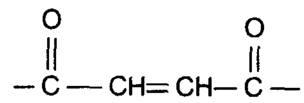

oder

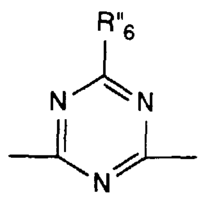

ist,
worin
$R_{11}$ Sulfo, Methyl, Methoxy, Chlor, Carboxy oder vorzugsweise Wasserstoff bedeutet und $R''_6$ Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m-, oder p-Methoxyphenylamino, o-, m- oder p-Chlorphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder Morpholino ist.

**16.** Oligomerengemisch gemäss einem der Ansprüche 1 bis 15, dadurch gekennzeichnet, dass B einen Rest der Formel

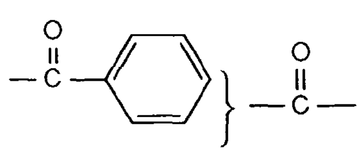

oder

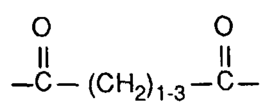

bedeutet.

**17.** Oligomerengemisch gemäss Anspruch 1 der Formel (1), worin

$R_1$ für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes $C_1$-$C_4$-Alkyl, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl steht,
R Wasserstoff, $C_1$-$C_4$-Alkyl, unsubstituiertes oder durch Methyl, Methoxy, Chlor und/oder Sulfo substituiertes Phenyl oder Benzyl oder einen Rest der Formel

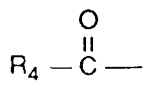,

worin $R_4$ für Methyl, Ethyl oder unsubstituiertes oder durch Sulfo, Chlor, Methyl und/oder Methoxy substituiertes Phenyl steht, bedeutet,
A für einen unsubstituierten oder durch Hydroxy, Sulfo, Sulfato, Methoxy, Carboxy oder Sulfophenyl substituierten $C_2$-$C_4$-Alkylenrest, $-CH_2-CH_2-Z'-CH_2-CH_2-$ worin Z' -O-, -S-, $-SO_2-$, -NH- oder $-N(CH_3)-$ bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder für einen $C_1$-$C_3$-Alkylen-phenylen- oder $C_1$-$C_2$-Alkylen-phenylen-$C_1$-$C_2$-alkylenrest, worin das Phenylen jeweils unsubstituiert oder durch Methyl, Methoxy, Chlor oder Sulfo substituiert ist, steht,
X eine Gruppe $-N(R_5)-$, worin $R_5$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet, ist, oder worin die Gruppe

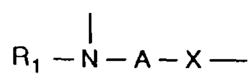

den Piperazin-1,4-diylrest bedeutet,
Y Methoxy, Methyl, Chlor oder Sulfo ist,
n für die Zahl 0 oder 1 steht,
Z Hydroxy, Methyl oder Ethyl bedeutet,
p die Zahl 1 darstellt,
$R_2$ und $R_3$ jeweils Wasserstoff, Fluor, Chlor, Brom, Methyl, Methoxy, Acetylamino, Phenoxy oder Cyano bedeuten, und
m eine beliebige Zahl von 1 bis 3 bedeutet.

**18.** Oligomerengemisch gemäss Anspruch 1 der Formel

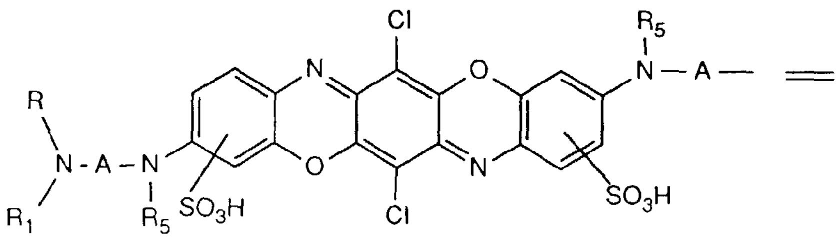

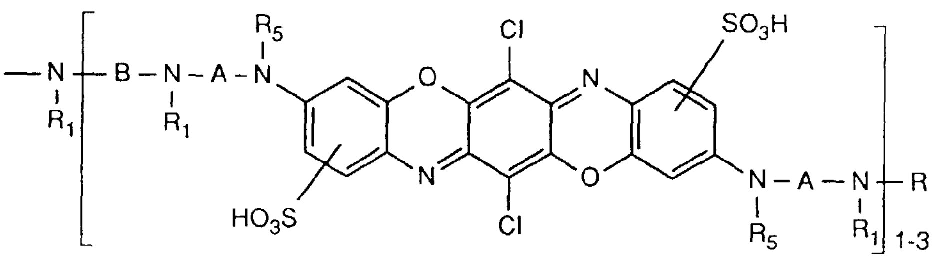

(1a),

worin n

$R_1$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet,
R für Wasserstoff, Methyl, Ethyl, Benzyl, Acetylamino oder Benzoylamino steht,
A einen unsubstituierten oder durch Hydroxy, Sulfo, Sulfato, Methoxy, Carboxy oder Sulfophenyl substituierten $C_2$-$C_4$-Alkylenrest darstellt,
$R_5$ Wasserstoff, Methyl oder Ethyl bedeutet, und
B für einen Rest der Formel

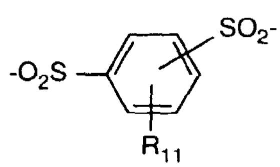

,

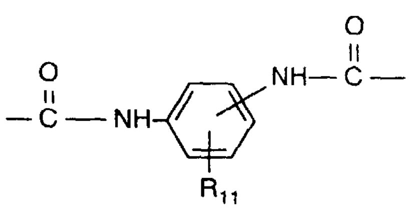

,

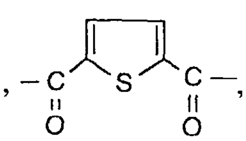

,

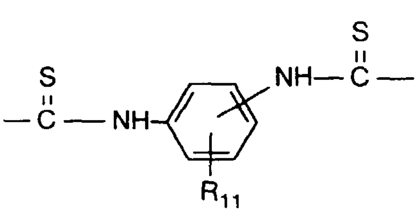

,

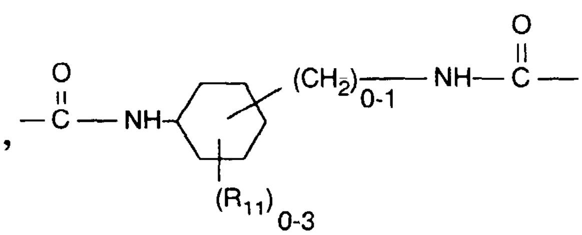

,

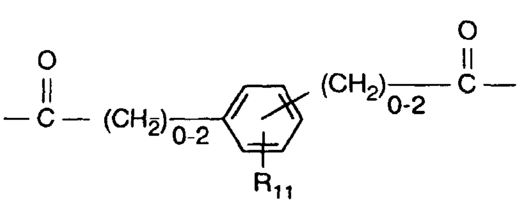

,

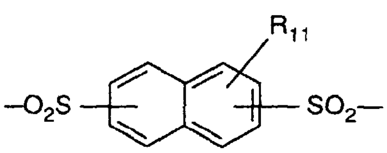

,

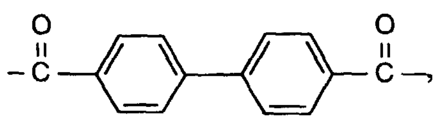, 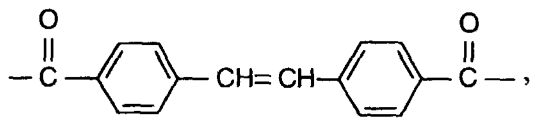,

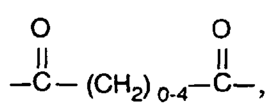, 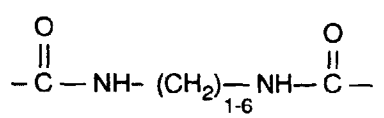, 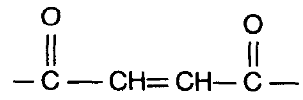

oder

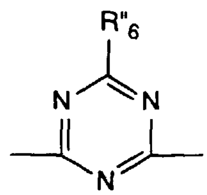,

worin
$R_{11}$ Sulfo, Methyl, Methoxy, Chlor, Carboxy oder vorzugsweise Wasserstoff bedeutet und $R''_6$ Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m-, oder p-Methoxyphenylamino, o-, m- oder p-Chlorphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder Morpholino ist, steht.

**19.** Oligomerengemisch gemäss Anspruch 1 der Formel

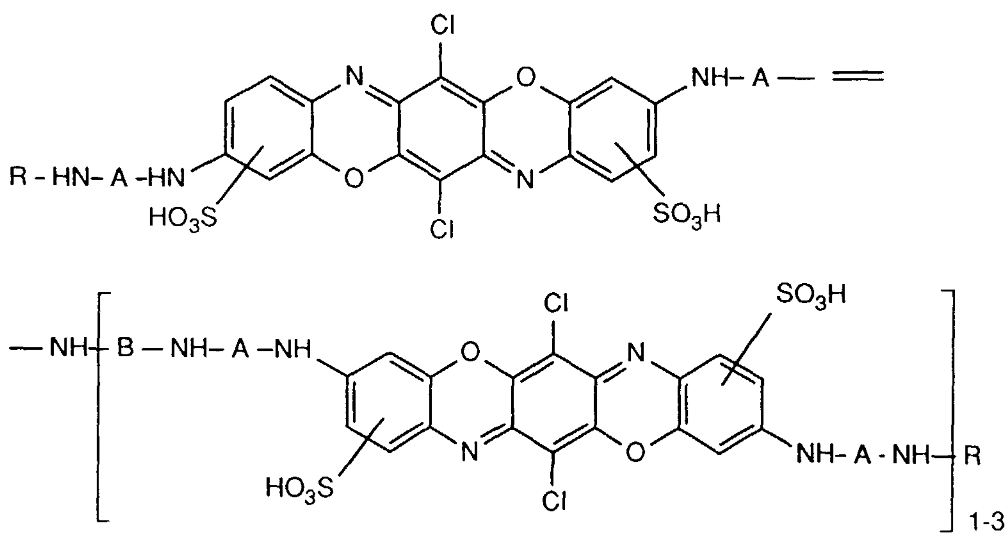

(1b),

worin

R Methyl, Ethyl, Benzyl, Acetylamino, Benzoylamino oder insbesondere Wasserstoff bedeutet,
A für einen unsubstituierten oder durch Hydroxy oder Sulfato substituierten 1,2-Ethylen- oder 1,2- oder 1,3-Propylenrest steht und

B ein Rest der Formel

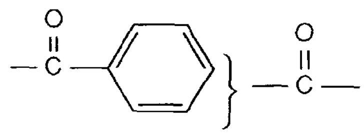

oder

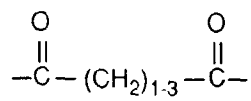

ist.

**20.** Oligomerengemisch gemäss einem der Ansprüche 1 bis 19, enthaltend eine Verbindung der Formel (1) mit m = 1, eine Verbindung der Formel (1) mit m = 2 und eine Verbindung der Formel (1) mit m = 3.

**21.** Verfahren zur Herstellung von Oligomerengemischen gemäss Anspruch 1, dadurch gekennzeichnet, dass man a) eine Verbindung der Formel

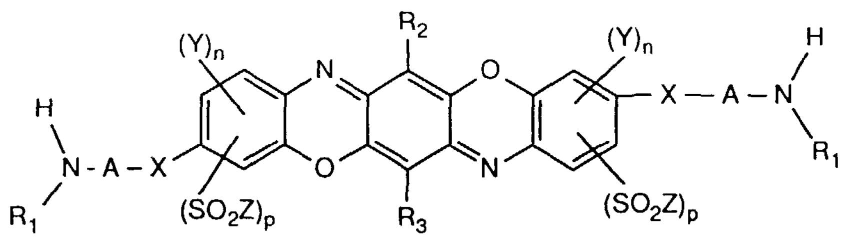

(9),

mit einer Verbindung der Formel

T-B-T (10),

kondensiert und gegebenenfalls b) das gemäss a) erhaltene Produkt mit einer Verbindung der Formel

R*-T (11),

umsetzt, worin A, B, $R_1$, $R_2$, $R_3$, X, Y, Z, p und n jeweils die zuvor angegebene Bedeutung haben, R* die zuvor für R angegebene Bedeutung mit Ausnahme von Wasserstoff hat und T Halogen, vorzugsweise Chlor, ist.

**22.** Verwendung der Gemische verschiedener oligomerer Verbindungen der Formel (1) gemäss Anspruch 1 als Farbstoffe zum Färben und Bedrucken von stickstoffhaltigen und insbesondere cellulosischen Fasermaterialien.

**23.** Verwendung nach Anspruch 22, dadurch gekennzeichnet, dass man Fasergemische aus Synthesefasern und cellulosischen Fasermaterialien, insbesondere Polyester/BaumwollMischgewebe, in Gegenwart eines Dispersionsfarbstoffes für die Polyesterfasern unter den Färbebedingungen für Polyesterfasern färbt.

**24.** Verfahren zum Färben von Polyester/Baumwoll-Mischgeweben mit Dispersions- und Direktfarbstoffen, dadurch gekennzeichnet, dass man in einem einstufigen, einbadigen Verfahren neben den Dispersionsfarbstoffen Gemische verschiedener oligomerer Verbindungen der Formel (1) gemäss Anspruch 1 als Farbstoffe verwendet und aus wässriger Flotte bei Temperaturen im Bereich von 100 bis 150°C, vorzugsweise 120 bis 130°C, und einem pH-Wert zwischen 4 und 7,5 färbt.

**Patentansprüche für folgenden Vertragsstaat : ES**

1. Verfahren zur Herstellung von Oligomerengemischen enthaltend mindestens zwei Verbindungen der Formel

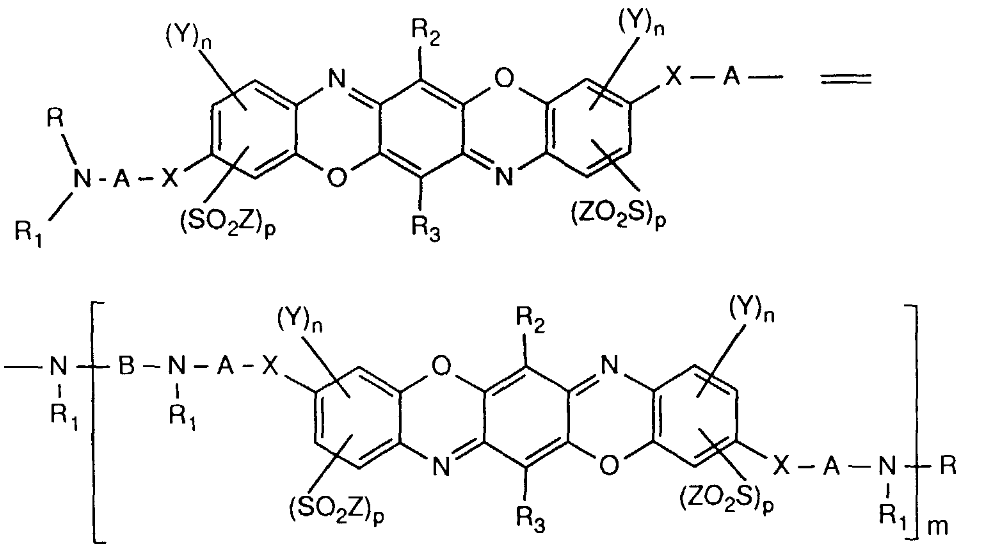

(1),

worin
$R_1$ für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes $C_1$-$C_4$-Alky-, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl steht,
R unabhängig die Bedeutung von $R_1$ hat, oder
ein Pyridin-, Pyrimidin-, Chinoxalin oder Triazinrest ist, wobei diese Reste unsubstituiert oder durch Hydroxy; $C_1$-$C_4$-Alkyl; Phenyl; $C_1$-$C_4$-Alkoxy; $C_1$-$C_4$-Alkylthio; Amino; unsubstituiertes oder im Alkylteil durch Hydroxy, Carboxy, Cyano, Sulfo, Sulfato oder $C_1$-$C_4$-Alkoxy substituiertes N-Mono- oder N,N-Di-$C_1$-$C_4$-Alkylamino; Cyclohexylamino; unsubstituiertes oder im Phenylteil durch $C_1$-$C_4$-Alkyl, $C_1$-$C_4$-Alkoxy, Phenoxy, Carboxy, Sulfo und/oder Halogen substituiertes Phenylamino oder N-$C_1$-$C_4$-Alkyl-N-phenylamino; Morpholino; oder 3-Carboxy- oder 3-Carbamoylpyridin-1-yl; sind, oder
R ein Rest der Formel

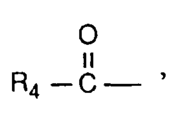

ist, worin $R_4$ ein Chinoxalin- oder Pyrimidinrest ist oder die oben für $R_1$ angegebenen Bedeutungen hat, jedoch nicht Wasserstoff bedeutet,
A ein unsubstituierter oder durch Hydroxy, Sulfo, Sulfato, $C_1$-$C_4$-Alkoxy, Carboxy, Cyano, Halogen, Phenyl, Sulfophenyl oder $C_2$-$C_5$-Alkoxycarbonyl substituierter und/oder gegebenenfalls durch 1-2 Gruppen -O-, -N($R_8$)-, worin $R_8$ $C_1$-$C_4$-Alkyl, Acetyl oder insbesondere Wasserstoff bedeutet, -S- oder -$SO_2$- unterbrochener $C_2$-$C_6$-Alkylenrest; unsubstituiertes oder einfach oder mehrfach durch $C_1$-$C_3$-Alkyl substituiertes Cyclopentylen oder Cyclohexylen;

ein unsubstituierter oder durch $C_1$-$C_4$-Alkyl, $C_1$-$C_4$-Alkoxy, Sulfo, Halogen oder Carboxy substituierter Phenylen-, Biphenylen- oder Naphthylenrest; oder

ein $C_1$-$C_6$-Alkylen-phenylen-, Phenylen-$C_1$-$C_6$-alkylen-phenylen-, $C_1$-$C_3$-Alkylenphenylen-$C_1$-$C_3$-alkylen- oder ein Methylen-naphthylen-methylenrest ist, wobei in diesen Aralkylenresten das Phenylen und Naphthylen gegebenenfalls noch 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino tragen kann,

X -O-, -S- oder -N($R_5$)- ist, worin $R_5$ die oben für $R_1$ angegebenen Bedeutungen hat, oder worin die Gruppe

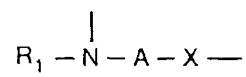

ein Piperazin-1,4-diylrest ist,

Y $C_1$-$C_4$-Alkyl, $C_1$-$C_4$-Alkoxy, Halogen, Sulfo, Carboxy, Carbamoyl, N-Mono- oder N,N-Di-$C_1$-$C_4$-Alkylcarbamoyl, N-Phenyl- oder N,N-Diphenylcarbamoyl, Sulfamoyl, N-Mono- oder N,N-Di-$C_1$-$C_4$-Alkylsulfamoyl oder N-Phenyl- oder N,N-Diphenylsulfamoyl ist,

Z Hydroxy oder $C_1$-$C_4$-Alkyl ist,

$R_2$ und $R_3$ unabhängig voneinander je Wasserstoff; Halogen; Cyano; $C_1$-$C_4$-Alkyl; $C_1$-$C_4$-Alkoxy; Sulfo; Carboxy; Carbamoyl; Phenylcarbamoyl; $C_2$-$C_5$-Alkanoylamino; oder gegebenenfalls durch $C_1$-$C_4$-Alkyl, $C_1$-$C_4$-Alkoxy, Acetylamino, Halogen, Nitro oder Sulfo substituiertes Phenyl, Benzyl, Benzoylamino oder Phenoxy sind,

B ein Brückenglied der Formel

$$Q'\text{-}E'\text{-}Q' \qquad (3'),$$

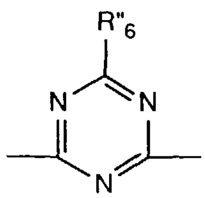

(4'),

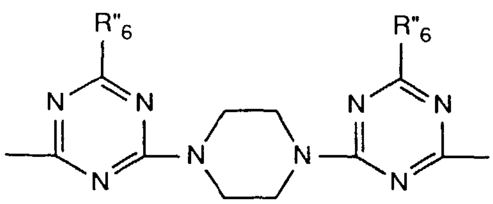

(6'),

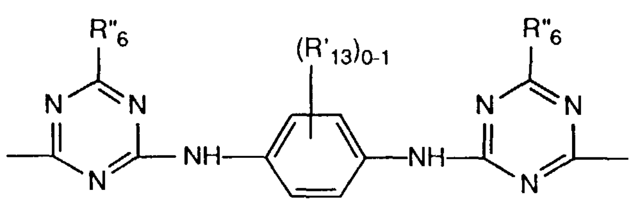

(7')

oder

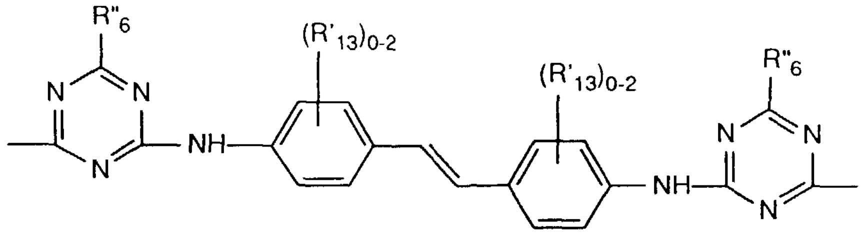

(8)

ist,
worin Q' eine Gruppe

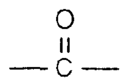 , 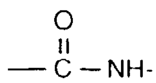 , 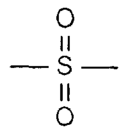 oder 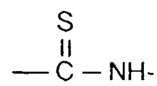

bedeutet,
E' für die direkte Bindung;
unsubstituiertes oder durch Hydroxy, Sulfo, Sulfato, Methoxy, Carboxy, Phenyl oder Sulfophenyl substituiertes $C_1$-$C_6$-Alkylen oder $C_2$-$C_6$-Alkenylen;
unsubstituiertes oder durch 1 bis 3 Methylgruppen substituiertes Cyclohexylen oder $C_1$-$C_2$-Alkylen-cyclohexylen; Piperazin-1,4-diyl; Thiophen-2,5-diyl; Biphenyl-4,4'-diyl; Stilben-4,4'-diyl; unsubstituiertes oder durch $C_1$-$C_4$-Alkyl, $C_1$-$C_4$-Alkoxy, Sulfo, Halogen oder Carboxy substituiertes Phenylen oder Naphthylen; oder für
unsubstituiertes oder im Phenylteil durch Methyl, Methoxy, Chlor oder Sulfo substituiertes $C_1$-$C_3$-Alkylen-phenylen oder $C_1$-$C_2$-Alkylen-phenylen-$C_1$-$C_2$-alkylen steht, $R''_6$ Chlor; Hydroxy; $C_1$-$C_4$-Alkoxy; $C_1$-$C_2$-Alkylthio; Amino; unsubstituiertes oder im Alkylteil durch Hydroxy, Sulfo oder Sulfato substituiertes N-Mono- oder N,N-Di-$C_1$-$C_4$-Alkylamino; Cyclohexylamino; unsubstituiertes oder im Phenylteil durch Methyl, Methoxy, Carboxy, Sulfo oder Chlor substituiertes Phenylamino oder N-$C_1$-$C_4$-Alkyl-N-phenylamino; oder Morpholino bedeutet und $R'_{13}$ Sulfo, Methyl oder Methoxy bedeutet,
m eine Zahl von 1 bis 6 bedeutet und
n und p unabhängig voneinander je für die Zahl 0 oder 1 stehen,
wobei sich die verschiedenen Verbindungen der Formel (1) ausschliesslich im Wert von m unterscheiden,
dadurch gekennzeichnet, dass man a) eine Verbindung der Formel

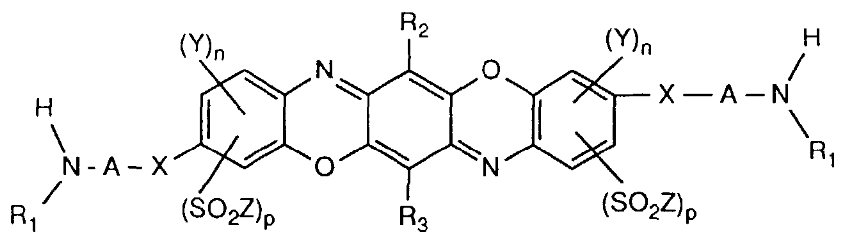

(9),

mit einer Verbindung der Formel

$$T\text{-}B\text{-}T \qquad (10),$$

kondensiert und gegebenenfalls b) das gemäss a) erhaltene Produkt mit einer Verbindung der Formel

$$R^*\text{-}T \qquad (11),$$

umsetzt, worin A, B, $R_1$, $R_2$, $R_3$, X, Y, Z, p und n jeweils die zuvor angegebene Bedeutung haben, R* die zuvor für R angegebene Bedeutung mit Ausnahme von Wasserstoff hat und T Halogen, vorzugsweise Chlor, ist.

**2.** Verfahren gemäss Anspruch 1, dadurch gekennzeichnet, dass man eine Verbindung der Formel (9) verwendet, worin $R_1$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet.

**3.** Verfahren gemäss einem der Ansprüche 1 und 2, dadurch gekennzeichnet, dass man eine Verbindung der Formel (9) verwendet, worin $R_1$ Wasserstoff ist.

**4.** Verfahren gemäss einem der Ansprüche 1 bis 3, dadurch gekennzeichnet, dass man eine Verbindung der Formel (9) mit einer Verbindung der Formel (10) umsetzt und gegebenenfalls das erhaltene Produkt mit einer Verbindung der Formel (11) umsetzt, worin R* $C_1$-$C_4$-Alkyl, unsubstituiertes oder durch Methyl, Methoxy, Chlor und/oder Sulfo substituiertes Phenvl oder Benzvl oder einen Rest der Formel

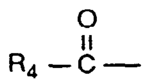 ,

worin $R_4$ für Methyl, Ethyl oder unsubstituiertes oder durch Sulfo, Chlor, Methyl und/oder Methoxy substituiertes Phenyl steht, bedeutet.

**5.** Verfahren gemäss einem der Ansprüche 1 bis 4, dadurch gekennzeichnet, dass man eine Verbindung der Formel (9) mit einer Verbindung der Formel (10) umsetzt und gegebenenfalls das erhaltene Produkt mit einer Verbindung der Formel (11) umsetzt, worin R* Methyl, Ethyl, Benzyl, Acetylamino oder Benzoylamino bedeutet, wobei die alleinige Umsetzung der Verbindung der Formel (9) mit einer Verbindung der Formel (10) bevorzugt ist.

**6.** Verfahren gemäss einem der Ansprüche 1 bis 5, dadurch gekennzeichnet, dass man eine Verbindung der Formel (9) verwendet, worin A einen unsubstituierten oder durch Hydroxy, Sulfo, Sulfato, Methoxy, Carboxy oder Sulfophenyl substituierten $C_2$-$C_4$-Alkylenrest, $-CH_2-CH_2-Z'-CH_2-CH_2-$ worin Z' -O-, -S-, $-SO_2-$, -NH- oder $-N(CH_3)-$ bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder einen $C_1$-$C_3$-Alkylen-phenylen- oder $C_1$-$C_2$-Alkylen-phenylen-$C_1$-$C_2$-alkylenrest, worin das Phenylen jeweils unsubstituiert oder durch Methyl, Methoxy, Chlor oder Sulfo substituiert ist, bedeutet.

**7.** Verfahren gemäss einem der Ansprüche 1 bis 6, dadurch gekennzeichnet, dass man eine Verbindung der Formel (9) verwendet, worin A unsubstituiertes oder durch Hydroxy, Sulfo, Sulfato, Methoxy, Carboxy oder Sulfophenyl substituiertes $C_2$-$C_4$-Alkylen bedeutet.

**8.** Verfahren gemäss einem der Ansprüche 1 bis 7, dadurch gekennzeichnet, dass man eine Verbindung der Formel (9) verwendet, worin A unsubstituiertes oder durch Hydroxy oder Sulfato substituiertes 1,2-Ethylen oder 1,2- oder 1,3-Propylen bedeutet.

**9.** Verfahren gemäss einem der Ansprüche 1 bis 8, dadurch gekennzeichnet, dass man eine Verbindung der Formel (9) verwendet, worin X für die Gruppe $-N(R_5)-$, worin $R_5$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet, steht.

**10.** Verfahren gemäss Anspruch 9, dadurch gekennzeichnet, dass man eine Verbindung der Formel (9) verwendet, worin $R_5$ Wasserstoff oder $C_1$-$C_4$-Alkyl, besonders Wasserstoff, ist.

**11.** Verfahren gemäss einem der Ansprüche 1 bis 10, dadurch gekennzeichnet, dass man eine Verbindung der Formel (9) verwendet, worin n für die Zahl 0 steht.

**12.** Verfahren gemäss einem der Ansprüche 1 bis 11, dadurch gekennzeichnet, dass man eine Verbindung der Formel (9) verwendet, worin p für die Zahl 1 steht und Z Hydroxy oder $C_1$-$C_4$-Alkyl, besonders Hydroxy, bedeutet.

**13.** Verfahren gemäss einem der Ansprüche 1 bis 12, dadurch gekennzeichnet, dass man eine Verbindung der Formel (9) verwendet, worin $R_2$ und $R_3$ jeweils Wasserstoff, Fluor, Chlor, Brom, Methyl, Methoxy, Acetylamino, Phenoxy oder Cyano bedeuten.

**14.** Verfahren gemäss einem der Ansprüche 1 bis 13, dadurch gekennzeichnet, dass man eine Verbindung der Formel

(9) verwendet, worin $R_2$ und $R_3$ je Chlor bedeuten.

**15.** Verfahren gemäss einem der Ansprüche 1 bis 14, dadurch gekennzeichnet, dass man eine Verbindung der Formel (10) verwendet, worin B ein Brückenglied der Formel

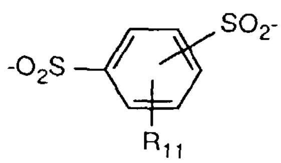 , 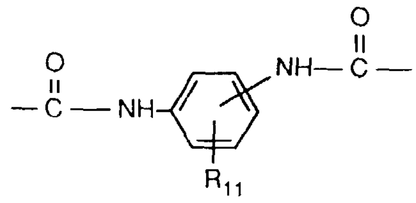 , 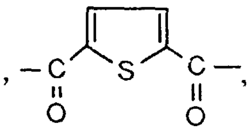

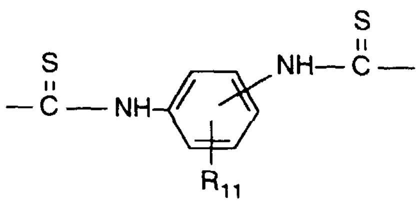 , 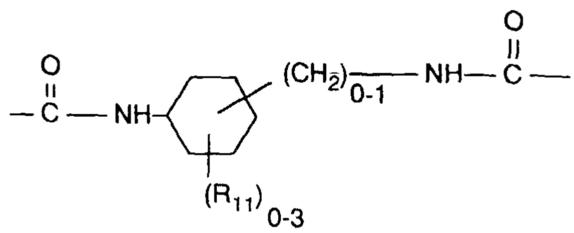 ,

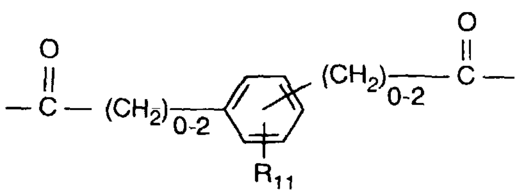 , 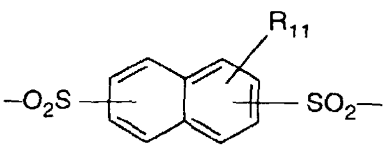 ,

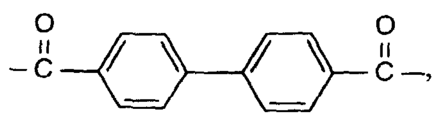 , 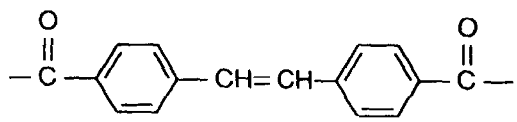 ,

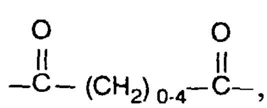 , 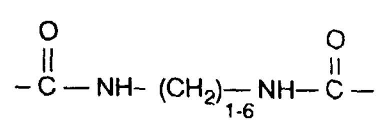 , 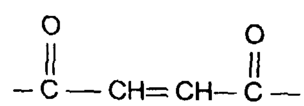

oder

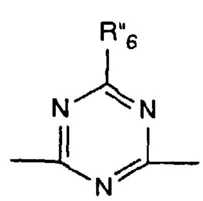 ,

worin $R_{11}$ Sulfo, Methyl, Methoxy, Chlor, Carboxy oder vorzugsweise Wasserstoff bedeutet und $R''_6$ Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m-, oder p-Methoxyphenylamino, o-, m- oder p-Chlorphenylamino, o-, m- oder p-Sulfophenylamino, 2,4- oder

2,5-Disulfophenylamino, o-Carboxyphenylamino, N-Ethyl-N-phenylamino, N-Methyl-N-phenylamino oder Morpholino ist, bedeutet.

**16.** Verfahren gemäss einem der Ansprüche 1 bis 15, dadurch gekennzeichnet, dass man eine Verbindung der Formel (10) verwendet, worin B einen Rest der Formel

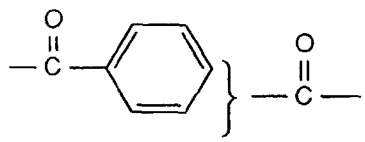

oder

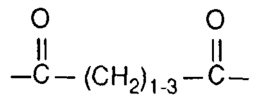

bedeutet.

**17.** Verfahren gemäss Anspruch 1, dadurch gekennzeichnet, dass man eine Verbindung der Formel (9), worin $R_1$ für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes $C_1$-$C_4$-Alkyl, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl steht,
A für einen unsubstituierten oder durch Hydroxy, Sulfo, Sulfato, Methoxy, Carboxy oder Sulfophenyl substituierten $C_2$-$C_4$-Alkylenrest, $-CH_2-CH_2-Z'-CH_2-CH_2-$ worin Z' -O-, -S-, $-SO_2-$, -NH- oder $-N(CH_3)-$ bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder für einen $C_1$-$C_3$-Alkylen-phenylen- oder $C_1$-$C_2$-Alkylen-phenylen-$C_1$-$C_2$-alkylenrest, worin das Phenylen jeweils unsubstituiert oder durch Methyl, Methoxy, Chlor oder Sulfo substituiert ist, steht,
X eine Gruppe $-N(R_5)-$, worin $R_5$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet, ist,
oder worin die Gruppe

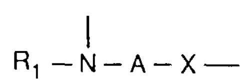

den Piperazin-1,4-diylrest bedeutet,
Y Methoxy, Methyl, Chlor oder Sulfo ist, n für die Zahl 0 oder 1 steht, Z Hydroxy, Methyl oder Ethyl bedeutet, p die Zahl 1 darstellt, $R_2$ und $R_3$ jeweils Wasserstoff, Fluor, Chlor, Brom, Methyl, Methoxy, Acetylamino, Phenoxy oder Cyano bedeuten, mit einer Verbindung der Formel (10) umsetzt,
und das erhaltene Produkt gegebenenfalls mit einer Verbindung der Formel (11) umsetzt, worin
R* $C_1$-$C_4$-Alkyl, unsubstituiertes oder durch Methyl, Methoxy, Chlor und/oder Sulfo substituiertes Phenyl oder Benzyl oder einen Rest der Formel

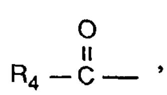

worin $R_4$ für Methyl, Ethyl oder unsubstituiertes oder durch Sulfo, Chlor, Methyl und/oder Methoxy substituiertes Phenyl steht, bedeutet, wobei im erhaltenen Oligomerengemisch m eine beliebige Zahl von 1 bis 3 ist.

**18.** Verfahren gemäss Anspruch 1, dadurch gekennzeichnet, dass man eine Verbindung der Formel

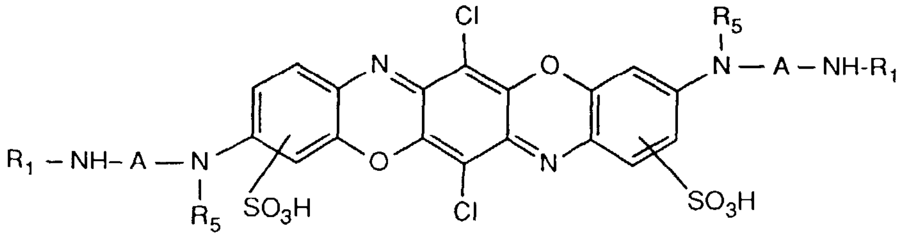

worin $R_1$ Wasserstoff, $C_1$-$C_4$-Alkyl, Cyclohexyl, unsubstituiertes oder durch Sulfo, Chlor, Methyl und/oder Methoxy substituiertes Phenyl oder Benzyl bedeutet,
A einen unsubstituierten oder durch Hydroxy, Sulfo, Sulfato, Methoxy, Carboxy oder Sulfophenyl substituierten $C_2$-$C_4$-Alkylenrest darstellt, $R_5$ Wasserstoff, Methyl oder Ethyl bedeutet,
mit einer Verbindung der Formel (10) umsetzt, worin
B für einen Rest der Formel

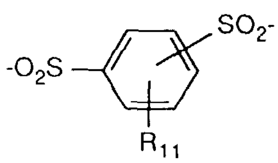 , 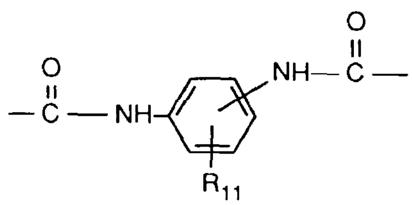 , 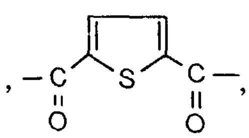

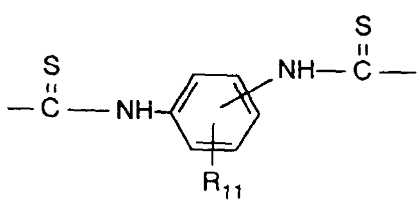 , 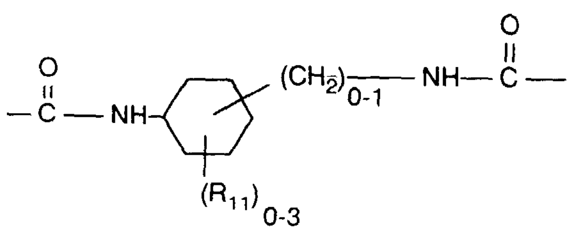 ,

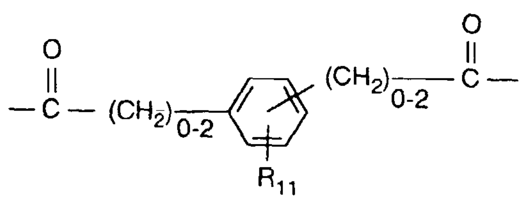 , 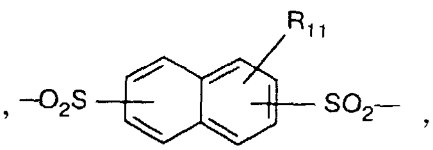 ,

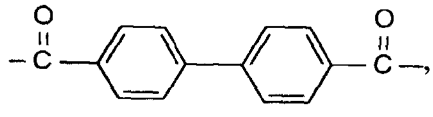 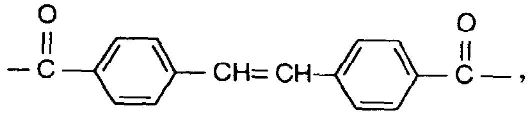

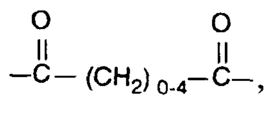, 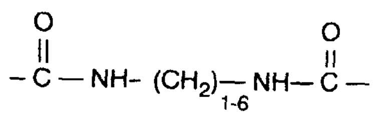 , 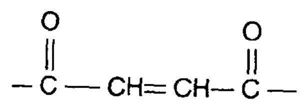

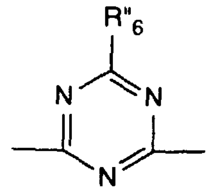 ,

oder worin $R_{11}$ Sulfo, Methyl, Methoxy, Chlor, Carboxy oder vorzugsweise Wasserstoff bedeutet und $R''_6$ Hydroxy, Chlor, Methyl- oder Ethylthio, Methoxy, Ethoxy, n- oder iso-Propoxy, Amino, Methylamino, Ethylamino, β-Hydroxyethylamino, N,N-Di-β-hydroxyethylamino, β-Sulfoethylamino, Cyclohexylamino, o-, m- oder p-Methylphenylamino, o-, m-, oder p-Methoxyphenylamino, o-, m- oder p-Chlorphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder Morpholino ist, steht,
und das erhaltene Produkt gegebenenfalls mit einer Verbindung der Formel (11) umsetzt, worin
R* für Methyl, Ethyl, Benzyl, Acetylamino oder Benzoylamino steht.

**19.** Verfahren gemäss Anspruch 1, dadurch gekennzeichnet, dass man eine Verbindung der Formel

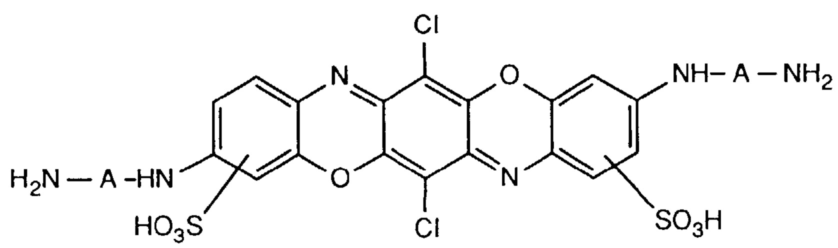

worin A für einen unsubstituierten oder durch Hydroxy oder Sulfato substituierten 1,2-Ethylen- oder 1,2- oder 1,3-Propylenrest steht, mit einer Verbindung der Formel (10), worin B ein Rest der Formel

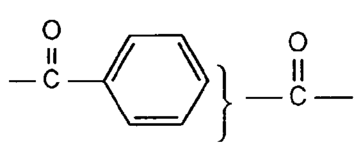

oder

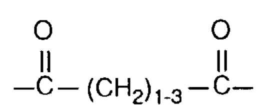

ist, umsetzt, und gegebenenfalls das erhaltene Produkt mit einer Verbindung der Formel (11), worin R* Methyl, Ethyl, Benzyl, Acetylamino oder Benzoylamino bedeutet, umsetzt.

**20.** Verwendung der gemäss Anspruch 1 erhaltenen Gemische verschiedener oligomerer Verbindungen der Formel (1) als Farbstoffe zum Färben und Bedrucken von stickstoffhaltigen und insbesondere cellulosischen Fasermaterialien.

**21.** Verwendung nach Anspruch 20, dadurch gekennzeichnet, dass man Fasergemische aus Synthesefasern und cellulosischen Fasermaterialien, insbesondere Polyester/Baumwoll-Mischgewebe, in Gegenwart eines Dispersionsfarbstoffes für die Polyesterfasem unter den Färbebedingungen für Polyesterfasern färbt.

**22.** Verfahren zum Färben von Polyester/Baumwoll-Mischgeweben mit Dispersions- und Direktfarbstoffen, dadurch gekennzeichnet, dass man in einem einstufigen, einbadigen Verfahren neben den Dispersionsfarbstoffen gemäss Anspruch 1 erhaltene Gemische verschiedener oligomerer Verbindungen der Formel (1) als Farbstoffe verwendet und aus wässriger Flotte bei Temperaturen im Bereich von 100 bis 150°C, vorzugsweise 120 bis 130°C, und einem pH-Wert zwischen 4 und 7,5 färbt.

## Claims

### Claims for the following Contracting States : BE, CH, DE, FR, GB, IT, LI

**1.** An oligomer mixture comprising at least two compounds of formula

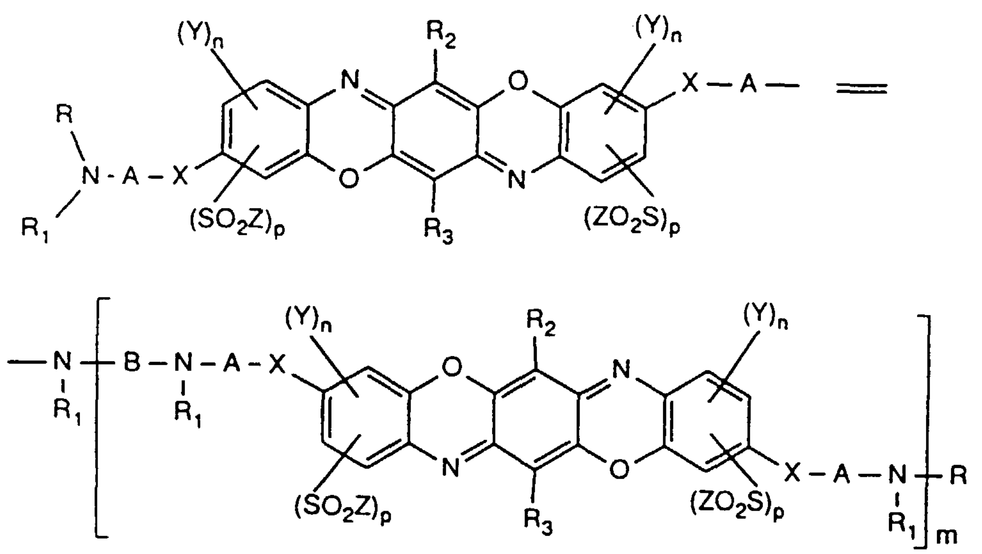

(1),

wherein
$R_1$ is hydrogen, unsubstituted $C_1$-$C_4$alkyl or $C_1$-$C_4$alkyl which is substituted by hydroxyl, sulfo, sulfato, chlorine, cyano or acetoxy and/or, with the exception of methyl, may be interrupted by a group -O-, cyclopentyl or cyclohexyl which are unsubstituted or substituted by 1 to 3 methyl groups, unsubstituted phenyl or phenyl which is substituted by sulfo, nitro, chlorine, methyl, methoxy, N-methylamino or N-ethylamino, N,N-dimethylamino or N,N-diethylamino, acetylamino, propionylamino, benzoylamino, methoxycarbonyl, ethoxycarbonyl, carboxyl or methylsulfonyl, or is unsubstituted 1- or 2-naphthyl or 1- or 2-naphthyl which is substituted by sulfo, nitro and/or chlorine, or is unsubstituted benzyl or benzyl which is substituted by methyl, methoxy, sulfo and/or chlorine,
R independently has the meaning of $R_1$ or is a pyridine, pyrimidine, quinoxaline or triazine radical, these radicals being unsubstituted or substituted by hydroxyl; $C_1$-$C_4$alkyl; phenyl; $C_1$-$C_4$alkoxy; $C_1$-$C_4$alkylthio; amino; N-mono- or N,N-di-$C_1$-$C_4$alkylamino which are unsubstituted or substituted in the alkyl moiety by hydroxyl, carboxyl, cyano, sulfo, sulfato or $C_1$-$C_4$alkoxy; cyclohexylamino; phenylamino or N-$C_1$-$C_4$alkyl-N-phenylamino which are unsubstituted or substituted in the phenyl moiety by $C_1$-$C_4$alkyl, $C_1$-$C_4$alkoxy, phenoxy, carboxyl, sulfo and/or halogen; morpholino; or 3-carboxyl- or 3-carbamoylpyridin-l-yl; or R is a radical of formula

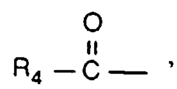

wherein $R_4$ is a quinoxaline or pyrimidine radical or has the meanings given above for $R_1$, but is not hydrogen,
A is a $C_2$-$C_6$alkylene radical which is unsubstituted or substituted by hydroxyl, sulfo, sulfato, $C_1$-$C_4$alkoxy, carboxyl, cyano, halogen, phenyl, sulfophenyl or $C_2$-$C_5$alkoxycarbonyl and/or which may be interrupted by 1 or 2 -O- or -N($R_8$)- groups, wherein $R_8$ is $C_1$-$C_4$alkyl, acetyl or, in particular, hydrogen, or by -S- or -$SO_2$-;
cyclopentylene or cyclohexylene which are unsubstituted or substituted by one or more $C_1$-$C_3$alkyl groups; a phenylene, biphenylene or naphthylene radical which is unsubstituted or substituted by $C_1$-$C_4$alkyl, $C_1$-$C_4$alkoxy, sulfo, halogen or carboxyl; or
is a $C_1$-$C_6$alkylene-phenylene, phenylene-$C_1$-$C_6$alkylene-phenylene, $C_1$-$C_3$alkylene-phenylene-$C_1$-$C_3$alkylene or a methylene-naphthylenemethylene radical, in which aralkylene radicals the phenylene and naphthylene moiety may additionally carry 1 or 2 substituents selected from the group consisting of sulfo, carboxyl, sulfamoyl, carbamoyl, methyl, ethyl, methoxy, ethoxy, nitro, chlorine, amino, N-methylamino and N-ethylamino, N,N-dimethylamino and N,N-diethylamino and phenylamino,
X is -O-, -S- or -N($R_5$)-, wherein $R_5$ has the meanings given above for $R_1$,
or wherein the group

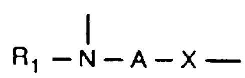

is a piperazine-1,4-diyl radical,
Y is $C_1$-$C_4$alkyl, $C_1$-$C_4$alkoxy, halogen, sulfo, carboxyl, carbamoyl, N-mono- or N,N-di-$C_1$-$C_4$alkylcarbamoyl, N-phenyl- or N,N-diphenylcarbamoyl, sulfamoyl, N-mono- or N,N-di-$C_1$-$C_4$alkylsulfamoyl or N-phenyl- or N,N-diphenylsulfamoyl,
Z is hydroxyl or $C_1$-$C_4$alkyl, $R_2$ and $R_3$ are each independently of the other hydrogen; halogen; cyano; $C_1$-$C_4$alkyl; $C_1$-$C_4$alkoxy; sulfo; carboxyl; carbamoyl; phenylcarbamoyl; $C_2$-$C_5$alkanoylamino; or phenyl, benzyl, benzoylamino or phenoxy which are unsubstituted or substituted by $C_1$-$C_4$alkyl, $C_1$-$C_4$alkoxy, acetylamino, halogen, nitro or sulfo,
B is a linking group of the formula

Q'-E'-Q' (3'),

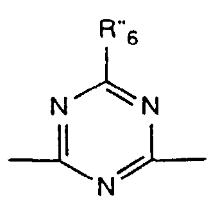

(4'),

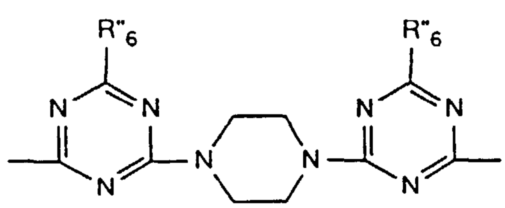

(6'),

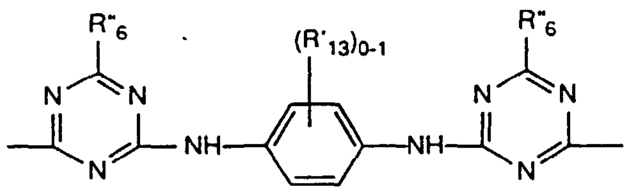

(7')

or

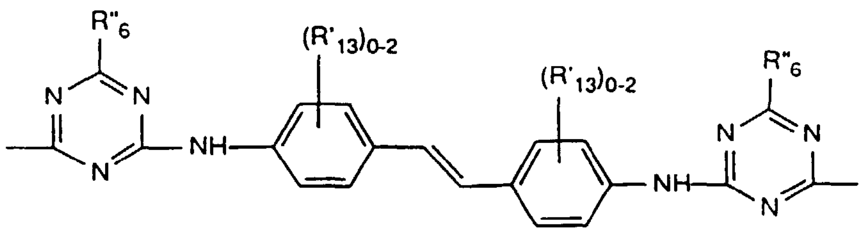

(8)

wherein Q' is a group

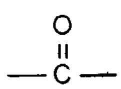 , 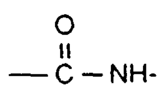 , 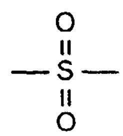 or 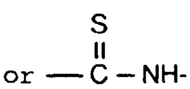 ,

E' is a direct bond; $C_1$-$C_6$alkylene or $C_2$-$C_6$alkenylene which are unsubstituted or substituted by hydroxyl, sulfo, sulfato, methoxy, carboxyl, phenyl or sulfophenyl; cyclohexylene or $C_1$-$C_2$alkylenecyclohexylene which are unsubstituted or substituted by 1 to 3 methyl groups; piperazine-1,4-diyl; thiophene-2,5-diyl; biphenyl-4,4'-diyl; stilbene-4,4'-diyl; unsubstituted phenylene or naphthylene, or phenylene or naphthylene which are substituted by $C_1$-$C_4$alkyl, $C_1$-$C_4$alkoxy, sulfo, halogen or carboxyl; or is $C_1$-$C_3$alkylene-phenylene or $C_1$-$C_2$alkylenephenylene-$C_1$-$C_2$alkylene which are unsubstituted or substituted in the phenyl moiety by methyl, methoxy, chlorine or sulfo,
$R''_6$ is chlorine; hydroxyl; $C_1$-$C_4$alkoxy; $C_1$-$C_2$alkylthio; amino; N-mono- or N,N-di-$C_1$-$C_4$alkylamino which are unsubstituted or substituted in the alkyl moiety by hydroxyl, sulfo or sulfato; cyclohexylamino; phenylamino or N-$C_1$-$C_4$alkyl-N-phenylamino which are unsubstituted or substituted in the phenyl moiety by methyl, methoxy, carboxyl, sulfo or chlorine; or is morpholino, and
$R'_{13}$ is sulfo, methyl or methoxy;
m is an integer from 1 to 6 and
n and p are each independently of the other 0 or 1,
the different compounds of formula (1) differing solely in the value of m.

2. An oligomer mixture according to claim 1, characterized in that $R_1$ is hydrogen, $C_1$-$C_4$alkyl, cyclohexyl, unsubstituted phenyl or benzyl, or phenyl or benzyl which are substituted by sulfo, chlorine, methyl and/or methoxy.

3. An oligomer mixture according to either claim 1 or claim 2, characterized in that $R_1$ is hydrogen.

4. An oligomer mixture according to any one of claims 1 to 3, characterized in that R is hydrogen, $C_1$-$C_4$alkyl, unsubstituted phenyl or benzyl, or phenyl or benzyl which are substituted by methyl, methoxy, chlorine and/or sulfo, or is a radical of formula

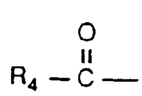 ,

wherein $R_4$ is methyl, ethyl or unsubstituted phenyl or phenyl which is substituted by sulfo, chlorine, methyl and/or methoxy.

**5.** An oligomer mixture according to any one of claims 1 to 4, characterized in that R is hydrogen, methyl, ethyl, benzyl, acetylamino or benzoylamino, particularly hydrogen.

**6.** An oligomer mixture according to any one of claims 1 to 5, characterized in that A is a $C_2$-$C_4$alkylene radical which is unsubstituted or substituted by hydroxyl, sulfo, sulfato, methoxy, carboxyl or sulfophenyl, $-CH_2-CH_2-Z'-CH_2-CH_2-$, wherein Z' is -O-, -S-, $-SO_2-$, -NH- or $-N(CH_3)-$, a cyclohexylene radical which is unsubstituted or substituted by 1 to 3 methyl groups, an unsubstituted or sulfo-substituted 1,3- or 1,4-phenylene radical, or a $C_1$-$C_3$alkylene-phenylene or $C_1$-$C_2$alkylenephenylene-$C_1$-$C_2$alkylene radical, wherein the phenylene radical is unsubstituted or substituted by methyl, methoxy, chlorine or sulfo.

**7.** An oligomer mixture according to any one of claims 1 to 6, characterized in that A is $C_2$-$C_4$alkylene which is unsubstituted or substituted by hydroxyl, sulfo, sulfato, methoxy, carboxyl or sulfophenyl.

**8.** An oligomer mixture according to any one of claims 1 to 7, characterized in that A is 1,2-ethylene or 1,2- or 1,3-propylene which is unsubstituted or substituted by hydroxyl or sulfato.

**9.** An oligomer mixture according to any one of claims 1 to 8, characterized in that X is the group $-N(R_5)-$, wherein $R_5$ is hydrogen, $C_1$-$C_4$alkyl, cyclohexyl, unsubstituted phenyl or benzyl, or phenyl or benzyl which are substituted by sulfo, chlorine, methyl and/or methoxy.

**10.** An oligomer mixture according to claim 9, characterized in that $R_5$ is hydrogen or $C_1$-$C_4$alkyl, particularly hydrogen.

**11.** An oligomer mixture according to any one of claims 1 to 10, characterized in that n is 0.

**12.** An oligomer mixture according to any one of claims 1 to 11, characterized in that p is 1 and Z is hydroxyl or $C_1$-$C_4$alkyl, particularly hydroxyl.

**13.** An oligomer mixture according to any one of claims 1 to 12, characterized in that $R_2$ and $R_3$ are each hydrogen, fluorine, chlorine, bromine, methyl, methoxy, acetylamino, phenoxy or cyano.

**14.** An oligomer mixture according to any one of claims 1 to 13, characterized in that $R_2$ and $R_3$ are each chlorine.

**15.** An oligomer mixture according to any one of claims 1 to 14, characterized in that B is a linking group of formula

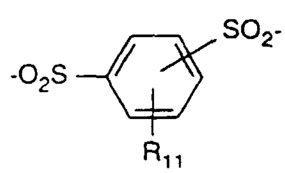 , 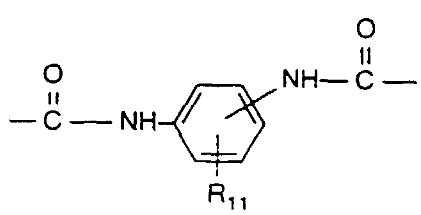 , 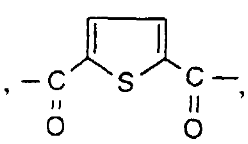 ,

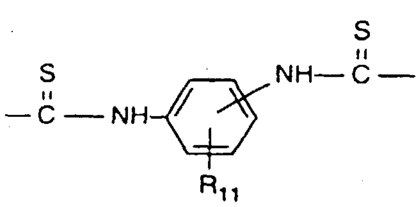 , 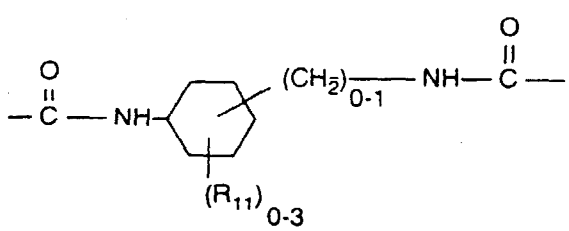 ,

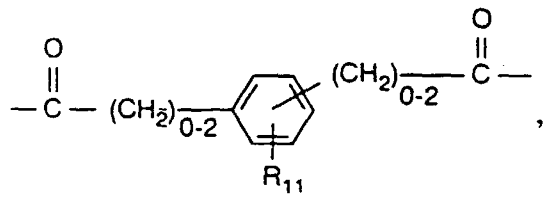, 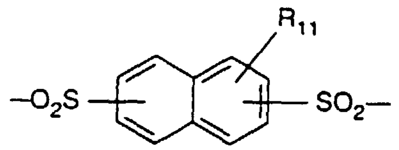,

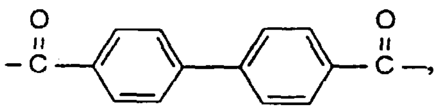, 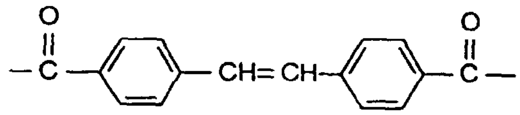,

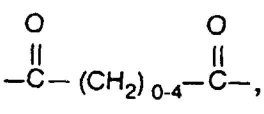, 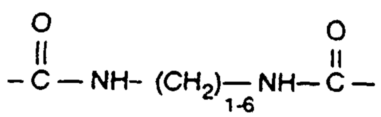, 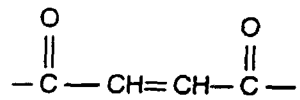

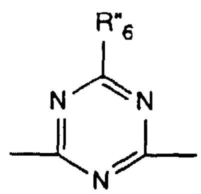,

wherein $R_{11}$ is sulfo, methyl, methoxy, chlorine, carboxyl or preferably hydrogen, and $R''_6$ is hydroxyl, chlorine, methylthio or ethylthio, methoxy, ethoxy, n- or isopropoxy, amino, methylamino, ethylamino, β-hydroxyethylamino, N,N-di-β-hydroxyethylamino, β-sulfoethylamino, cyclohexylamino, o-, m- or p-methylphenylamino, o-, m-, or p-methoxyphenylamino, o-, m- or p-chlorophenylamino, o-, m- or p-sulfophenylamino, 2,4- or 2,5-disulfophenylamino, o-carboxyphenylamino, N-ethyl-N-phenylamino, N-methyl-N-phenylamino or morpholino.

**16.** An oligomer mixture according to any one of claims 1 to 15, characterized in that B is a radical of formula

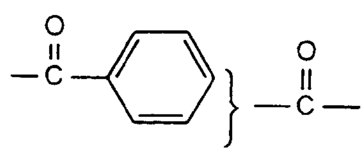

or

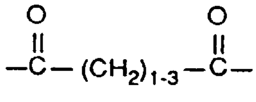

**17.** An oligomer mixture according to claim 1 of formula (1), wherein $R_1$ is hydrogen, unsubstituted $C_1$-$C_4$alkyl or $C_1$-$C_4$alkyl which is substituted by hydroxyl, sulfo, sulfato, chlorine, cyano, or acetoxy and/or, with the exception of methyl, may be interrupted by a group -O-, cyclopentyl or cyclohexyl which are unsubstituted or substituted by 1 to 3 methyl groups, unsubstituted phenyl or phenyl which is substituted by sulfo, nitro, chlorine, methyl, methoxy, N-methylamino or N-ethylamino, N,N-dimethylamino or N,N-diethylamino, acetylamino, propionylamino, benzoylamino, methoxycarbonyl, ethoxycarbonyl, carboxyl or methylsulfonyl, or is unsubstituted 1- or 2-naphthyl or 1- or 2-naphthyl which is substituted by sulfo, nitro and/or chlorine, or is unsubstituted benzyl or benzyl which is substituted by methyl, methoxy, sulfo and/or chlorine, R is hydrogen, $C_1$-$C_4$alkyl, unsubstituted phenyl or benzyl

or phenyl or benzyl which are substituted by methyl, methoxy, chlorine and/or sulfo, or is a radical of formula

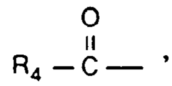

wherein $R_4$ is methyl, ethyl or unsubstituted phenyl or phenyl which is substituted by sulfo, chlorine, methyl and/or methoxy,

A is a $C_2$-$C_4$alkylene radical which is unsubstituted or substituted by hydroxyl, sulfo, sulfato, methoxy, carboxyl or sulfophenyl, $-CH_2-CH_2-Z'-CH_2-CH_2-$, wherein Z' is -O-, -S-, $-SO_2-$, -NH- or $-N(CH_3)-$, a cyclohexylene radical which is unsubstituted or substituted by 1 to 3 methyl groups, an unsubstituted or sulfo-substituted 1,3- or 1,4-phenylene radical or is a $C_1$-$C_3$alkylene-phenylene or $C_1$-$C_2$alkylenephenylene-$C_1$-$C_2$alkylene radical, wherein the phenylene moiety is in each case unsubstituted or substituted by methyl, methoxy, chlorine or sulfo, X is a group $-N(R_5)-$, wherein $R_5$ is hydrogen, $C_1$-$C_4$alkyl, cyclohexyl, unsubstituted phenyl or benzyl or phenyl or benzyl which are substituted by sulfo, chlorine, methyl and/or methoxy, or where the group

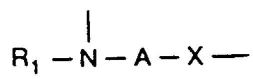

is the piperazine-1,4-diyl radical,
Y is methoxy, methyl, chlorine or sulfo,
n is 0 or 1,
Z is hydroxyl, methyl or ethyl,
p is 1,
$R_2$ and $R_3$ are each hydrogen, fluorine, chlorine, bromine, methyl, methoxy, acetylamino, phenoxy or cyano and
m is any integer from 1 to 3.

**18.** An oligomer mixture according to claim 1 of formula

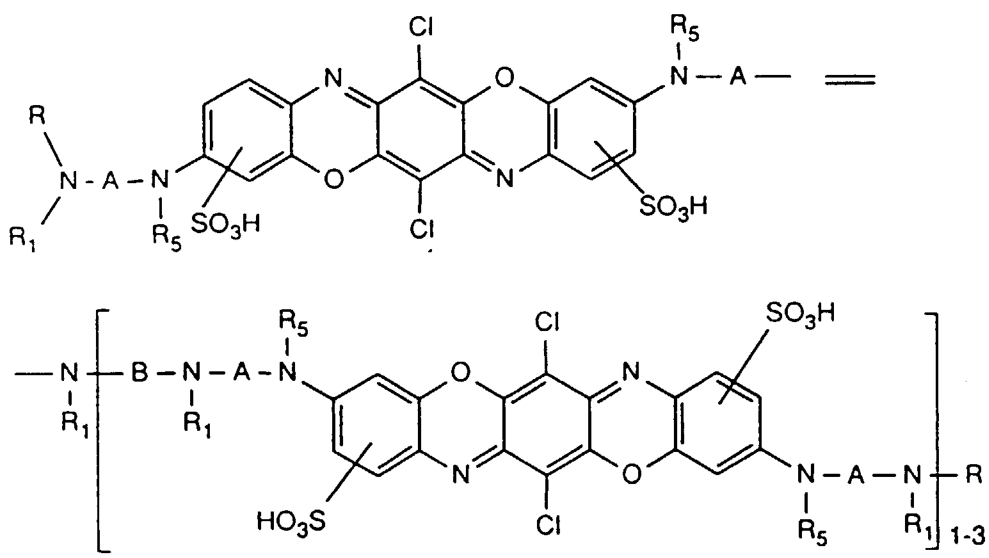

(1a),

wherein $R_1$ is hydrogen, $C_1$-$C_4$alkyl, cyclohexyl, unsubstituted phenyl or benzyl, or phenyl or benzyl which are substituted by sulfo, chlorine, methyl and/or methoxy,

R is hydrogen, methyl, ethyl, benzyl, acetylamino or benzoylamino,
A is a $C_2$-$C_4$alkylene radical which is unsubstituted or substituted by hydroxyl, sulfo, sulfato, methoxy, carboxyl

or sulfophenyl,
$R_5$ is hydrogen, methyl or ethyl, and
B is a radical of formula

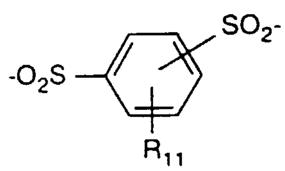
,
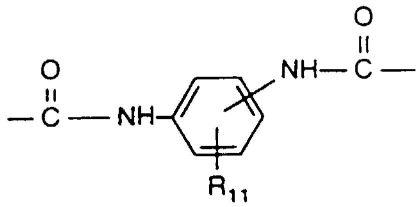
,
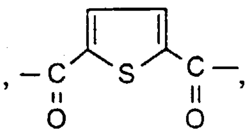
,

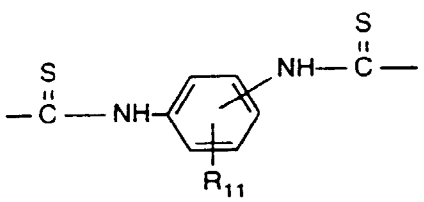
,
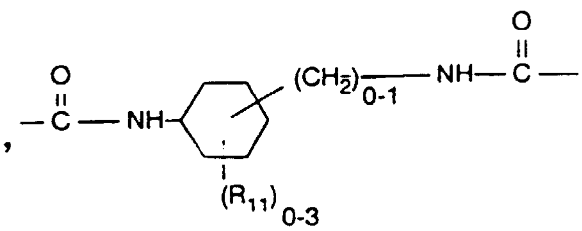
,

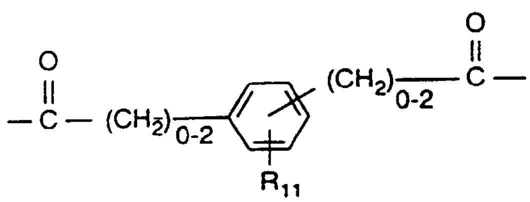
,
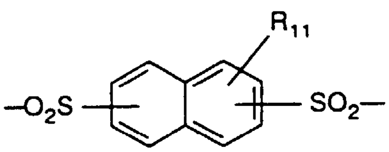
,

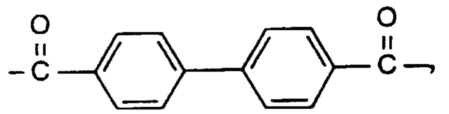
,
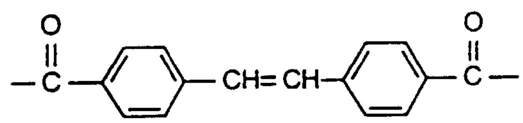
,

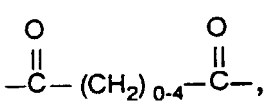
,
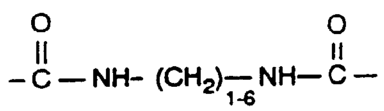
,
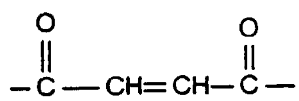

or

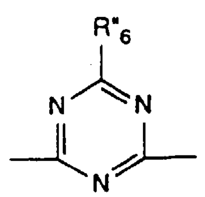
,

wherein $R_{11}$ is sulfo, methyl, methoxy, chlorine, carboxyl or preferably hydrogen, and $R''_6$ is hydroxyl, chlorine, methylthio or ethylthio, methoxy, ethoxy, n- or isopropoxy, amino, methylamino, ethylamino, β-hydroxyethylamino, N,N-di-β-hydroxyethylamino, β-sulfoethylamino, cyclohexylamino, o-, m- or p-methylphenylamino, o-, m- or p-methoxyphenylamino, o-, m- or p-chlorophenylamino, o-, m- or p-sulfophenylamino, 2,4- or 2,5-disulfophenylamino, o-carboxyphenylamino, N-ethyl-N-phenylamino, N-methyl-N-phenylamino or morpholino.

**19.** An oligomer mixture according to claim 1 of formula

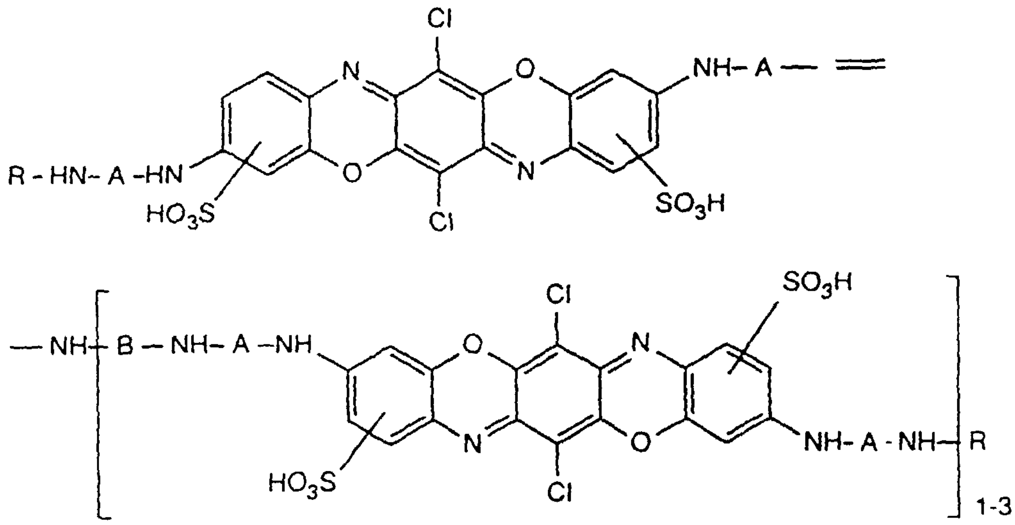

(1b),

wherein R is methyl, ethyl, benzyl, acetylamino, benzoylamino or, in particular, hydrogen, A is a 1,2-ethylene or 1,2- or 1,3-propylene radical which is unsubstituted or substituted by hydroxyl or sulfato, and B is a radical of formula

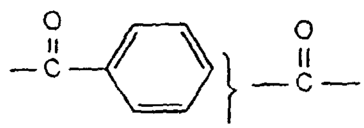

or

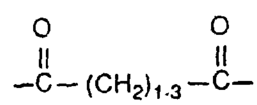

**20.** An oligomer mixture according to any one of claims 1 to 19, comprising a compound of formula (1) wherein m = 1, a compound of formula (1) wherein m = 2 and a compound of formula (1) wherein m = 3.

**21.** A process for the preparation of a mixture of oligomers according to claim 1, characterized in that a) a compound of formula

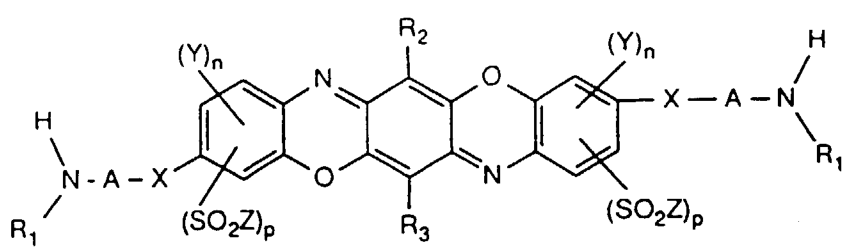

(9),

is condensed with a compound of formula

T-B-T (10),

and, optionally, b) the product obtained in a) is reacted with a compound of formula

R*-T (11),

wherein A, B, $R_1$, $R_2$, $R_3$, X, Y, Z, p and n are each as defined hereinbefore, R* has the meaning previously given for R, with the exception of hydrogen, and T is halogen, preferably chlorine.

**22.** Use of the mixtures of different oligomeric compounds of formula (1) as claimed in claim 1 as dyes for dyeing or printing nitrogen-containing and, in particular, cellulosic fibre materials.

**23.** Use according to claim 22, characterized in that blends of synthetic fibres and cellulosic fibre materials, in particular polyester/cotton blends, are dyed in the presence of a disperse dye for the polyester fibres under the dyeing conditions for polyester fibres.

**24.** A process for dyeing polyester/cotton blends with disperse and direct dyes, characterized in that mixtures of different oligomeric compounds of formula (1) as claimed in claim 1 are used as dyes, in a one-step, single bath process and in addition to said disperse dyes, and dyeing takes place from an aqueous liquor in the temperature range from 100 to 150°C, preferably from 120 to 130°C, and in the pH range from 4 to 7.5.

**Claims for the following Contracting State : ES**

**1.** A process for preparing an oligomer mixture comprising at least two compounds of formula

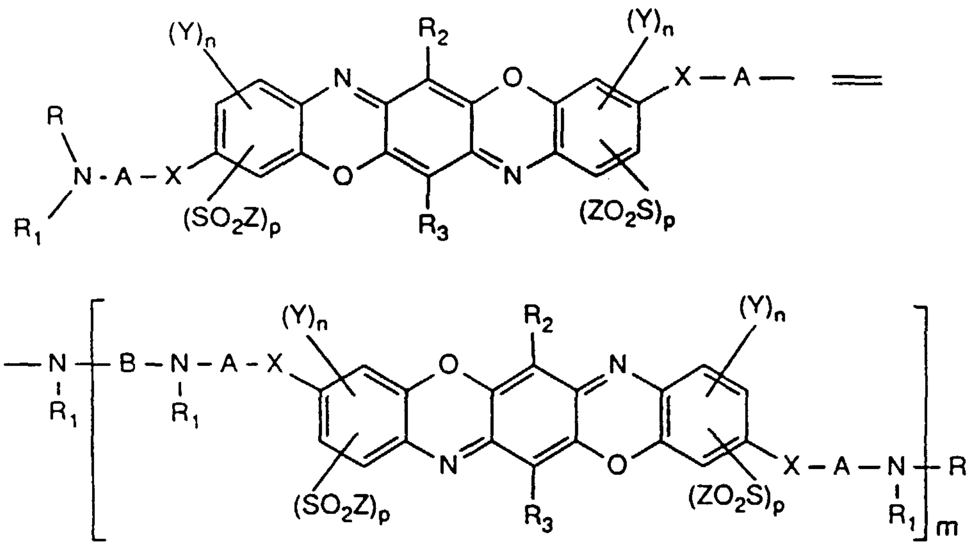

(1),

wherein

$R_1$ is hydrogen, unsubstituted $C_1$-$C_4$alkyl or $C_1$-$C_4$alkyl which is substituted by hydroxyl, sulfo, sulfato, chlorine, cyano or acetoxy and/or, with the exception of methyl, may be interrupted by a group -O-, cyclopentyl or cyclohexyl which are unsubstituted or substituted by 1 to 3 methyl groups, unsubstituted phenyl or phenyl which is substituted by sulfo, nitro, chlorine, methyl, methoxy, N-methylamino or N-ethylamino, N,N-dimethylamino or N,N-diethylamino, acetylamino, propionylamino, benzoylamino, methoxycarbonyl, ethoxycarbonyl, carboxyl or methylsulfonyl, or is unsubstituted 1- or 2-naphthyl or 1- or 2-naphthyl which is substituted by sulfo, nitro and/or chlorine, or is unsubstituted benzyl or benzyl which is substituted by methyl, methoxy, sulfo and/or chlorine,

R independently has the meaning of $R_1$ or is a pyridine, pyrimidine, quinoxaline or triazine radical, these radicals being unsubstituted or substituted by hydroxyl; $C_1$-$C_4$alkyl; phenyl; $C_1$-$C_4$alkoxy; $C_1$-$C_4$alkylthio; amino; N-mono- or N,N-di-$C_1$-$C_4$alkylamino which are unsubstituted or substituted in the alkyl moiety by hydroxyl, carboxyl, cyano,

sulfo, sulfato or $C_1$-$C_4$alkoxy; cyclohexylamino; phenylamino or N-$C_1$-$C_4$alkyl-N-phenylamino which are unsubstituted or substituted in the phenyl moiety by $C_1$-$C_4$alkyl, $C_1$-$C_4$alkoxy, phenoxy, carboxyl, sulfo and/or halogen; morpholino; or 3-carboxyl- or 3-carbamoylpyridin-1-yl; or R is a radical of formula

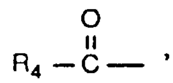

wherein $R_4$ is a quinoxaline or pyrimidine radical or has the meanings given above for $R_1$, but is not hydrogen,
A is a $C_2$-$C_6$alkylene radical which is unsubstituted or substituted by hydroxyl, sulfo, sulfato, $C_1$-$C_4$alkoxy, carboxyl, cyano, halogen, phenyl, sulfophenyl or $C_2$-$C_5$alkoxycarbonyl and/or which may be interrupted by 1 or 2 -O- or -N($R_8$)- groups, wherein $R_8$ is $C_1$-$C_4$alkyl, acetyl or, in particular, hydrogen, or by -S- or -$SO_2$-;
cyclopentylene or cyclohexylene which are unsubstituted or substituted by one or more $C_1$-$C_3$alkyl groups; a phenylene, biphenylene or naphthylene radical which is unsubstituted or substituted by $C_1$-$C_4$alkyl, $C_1$-$C_4$alkoxy, sulfo, halogen or carboxyl; or
is a $C_1$-$C_6$alkylene-phenylene, phenylene-$C_1$-$C_6$alkylene-phenylene, $C_1$-$C_3$alkylene-phenylene-$C_1$-$C_3$alkylene or a methylene-naphthylenemethylene radical, in which aralkylene radicals the phenylene and naphthylene moiety may additionally carry 1 or 2 substituents selected from the group consisting of sulfo, carboxyl, sulfamoyl, carbamoyl, methyl, ethyl, methoxy, ethoxy, nitro, chlorine, amino, N-methylamino and N-ethylamino, N,N-dimethylamino and N,N-diethylamino and phenylamino,
X is -O-, -S- or -N($R_5$)-, wherein $R_5$ has the meanings given above for $R_1$, or wherein the group

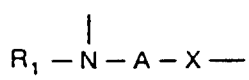

is a piperazine-1,4-diyl radical,
Y is $C_1$-$C_4$alkyl, $C_1$-$C_4$alkoxy, halogen, sulfo, carboxyl, carbamoyl, N-mono- or N,N-di-$C_1$-$C_4$alkylcarbamoyl, N-phenyl- or N,N-diphenylcarbamoyl, sulfamoyl, N-mono- or N,N-di-$C_1$-$C_4$alkylsulfamoyl or N-phenyl- or N,N-diphenylsulfamoyl,
Z is hydroxyl or $C_1$-$C_4$alkyl, $R_2$ and $R_3$ are each independently of the other hydrogen; halogen; cyano; $C_1$-$C_4$alkyl; $C_1$-$C_4$alkoxy; sulfo; carboxyl; carbamoyl; phenylcarbamoyl; $C_2$-$C_5$alkanoykamino; or phenyl, benzyl, benzoylamino or phenoxy which are unsubstituted or substituted by $C_1$-$C_4$alkyl, $C_1$-$C_4$alkoxy, acetylamino, halogen, nitro or sulfo,
B is a linking group of the formula

Q'-E'-Q' (3'),

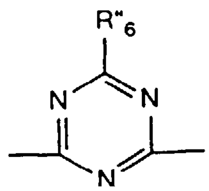

(4'),

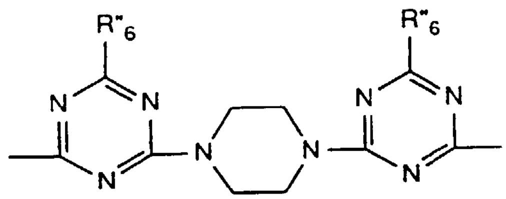

(6'),

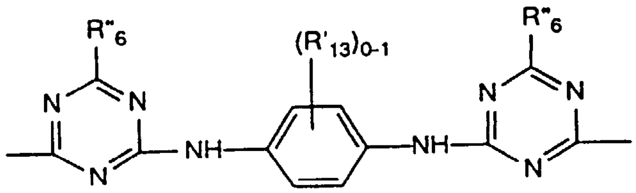

(7')

or

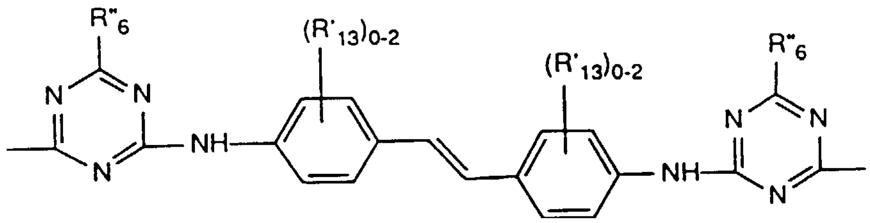

(8)

wherein Q' is a group

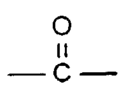 , 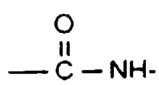 , 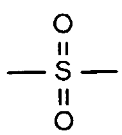 or 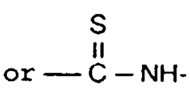 ,

E' is a direct bond; $C_1$-$C_6$alkylene or $C_2$-$C_6$alkenylene which are unsubstituted or substituted by hydroxyl, sulfo, sulfato, methoxy, carboxyl, phenyl or sulfophenyl; cyclohexylene or $C_1$-$C_2$alkylenecyclohexylene which are unsubstituted or substituted by 1 to 3 methyl groups; piperazine-1,4-diyl; thiophene-2,5-diyl; biphenyl-4,4'-diyl; stilbene-4,4'-diyl; unsubstituted phenylene or naphthylene, or phenylene or naphthylene which are substituted by $C_1$-$C_4$alkyl, $C_1$-$C_4$alkoxy, sulfo, halogen or carboxyl; or is $C_1$-$C_3$alkylene-phenylene or $C_1$-$C_2$alkylenephenylene-$C_1$-$C_2$alkylene which are unsubstituted or substituted in the phenyl moiety by methyl, methoxy, chlorine or sulfo,
$R''_6$ is chlorine; hydroxyl; $C_1$-$C_4$alkoxy; $C_1$-$C_2$alkylthio; amino; N-mono- or N,N-di-$C_1$-$C_4$alkylamino which are unsubstituted or substituted in the alkyl moiety by hydroxyl, sulfo or sulfato; cyclohexylamino; phenylamino or N-$C_1$-$C_4$alkyl-N-phenylamino which are unsubstituted or substituted in the phenyl moiety by methyl, methoxy, carboxyl, sulfo or chlorine; or is morpholino, and
$R'_{13}$ is sulfo, methyl or methoxy;
m is an integer from 1 to 6 and
n and p are each independently of the other 0 or 1,
the different compounds of formula (1) differing solely in the value of m, characterized in that a) a compound of formula

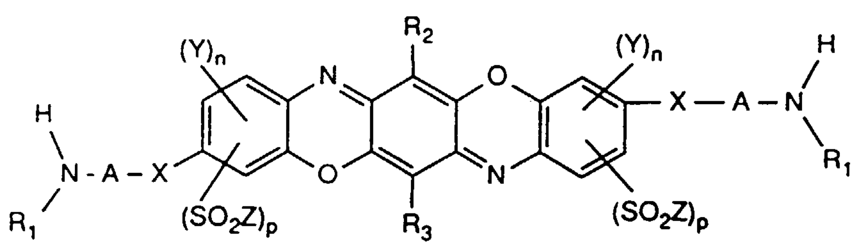

(9),

is condensed with a compound of formula

$$T\text{-}B\text{-}T \qquad (10),$$

and, optionally, b) the product obtained in a) is reacted with a compound of formula

$$R^*\text{-}T \qquad (11),$$

wherein A, B, $R_1$, $R_2$, $R_3$, X, Y, Z, p and n are each as defined hereinbefore, R* has the meaning previously given for R, with the exception of hydrogen, and T is halogen, preferably chlorine.

**2.** A process according to claim 1, characterized in that a compound of formula (9) is used, wherein $R_1$ is hydrogen, $C_1$-$C_4$alkyl, cyclohexyl, unsubstituted phenyl or benzyl, or phenyl or benzyl which are substituted by sulfo, chlorine, methyl and/or methoxy.

**3.** A process according to either claim 1 or claim 2, characterized in that a compound of formula (9) is used wherein $R_1$ is hydrogen.

**4.** A process according to any one of claims 1 to 3, characterized in that a compound of formula (9) is reacted with a compound of formula (10) and, optionally, the product obtained is reacted with a compound of formula (11) wherein R* is $C_1$-$C_4$alkyl, unsubstituted phenyl or benzyl, or phenyl or benzyl which are substituted by methyl, methoxy, chlorine and/or sulfo, or is a radical of formula

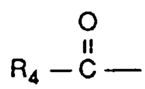 ,

wherein $R_4$ is methyl, ethyl or unsubstituted phenyl or phenyl which is substituted by sulfo, chlorine, methyl and/or methoxy.

**5.** A process according to any one of claims 1 to 4, characterized in that a compound of formula (9) is reacted with a compound of formula (10) and, optionally, the product obtained is reacted with a compound of formula (11) wherein R* is methyl, ethyl, benzyl, acetylamino or benzoylamino the reaction only of the compound of formula (9) with a compound of formula (10) being preferred.

**6.** A process according to any one of claims 1 to 5, characterized in that a compound of formula (9) is used wherein A is a $C_2$-$C_4$alkylene radical which is unsubstituted or substituted by hydroxyl, sulfo, sulfato, methoxy, carboxyl or sulfophenyl, $-CH_2-CH_2-Z'-CH_2-CH_2-$ wherein Z' is -O-, -S-, $-SO_2-$, -NH- or $-N(CH_3)-$, a cyclohexylene radical which is unsubstituted or substituted by 1 to 3 methyl groups, an unsubstituted or sulfo-substituted 1,3- or 1,4-phenylene radical, or a $C_1$-$C_3$alkylenephenylene or $C_1$-$C_2$alkylene-phenylene-$C_1$-$C_2$alkylene radical wherein the phenylene radical is unsubstituted or substituted by methyl, methoxy, chlorine or sulfo.

**7.** A process according to any one of claims 1 to 6, characterized in that a compound of formula (9) is used wherein A is $C_2$-$C_4$alkylene which is unsubstituted or substituted by hydroxyl, sulfo, sulfato, methoxy, carboxyl or sulfophenyl.

**8.** A process according to any one of claims 1 to 7, characterized in that a compound of formula (9) is used wherein A is 1,2-ethylene or 1,2- or 1,3-propylene which is unsubstituted or substituted by hydroxyl or sulfato.

**9.** A process according to any one of claims 1 to 8, characterized in that a compound of formula (9) is used wherein X is the group $-N(R_5)-$ wherein $R_5$ is hydrogen, $C_1$-$C_4$alkyl, cyclohexyl, unsubstituted phenyl or benzyl, or phenyl or benzyl which are substituted by sulfo, chlorine, methyl and/or methoxy.

**10.** A process according to claim 9, characterized in that a compound of formula (9) is used wherein $R_5$ is hydrogen or $C_1$-$C_4$alkyl, particularly hydrogen.

**11.** A process according to any one of claims 1 to 10, characterized in that a compound of formula (9) is used wherein n is 0.

**12.** A process according to any one of claims 1 to 11, characterized in that a compound of formula (9) is used wherein

p is 1 and Z is hydroxyl or $C_1$-$C_4$alkyl, particularly hydroxyl.

**13.** A process according to any one of claims 1 to 12, characterized in that a compound of formula (9) is used wherein $R_2$ and $R_3$ are each hydrogen, fluorine, chlorine, bromine, methyl, methoxy, acetylamino, phenoxy or cyano.

**14.** A process according to any one of claims 1 to 13, characterized in that a compound of formula (9) is used wherein $R_2$ and $R_3$ are each chlorine.

**15.** A process according to any one of claims 1 to 14, characterized in that a compound of formula (10) is used wherein B is a linking group of formula

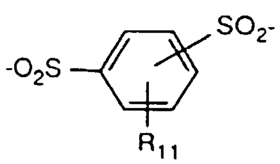 , 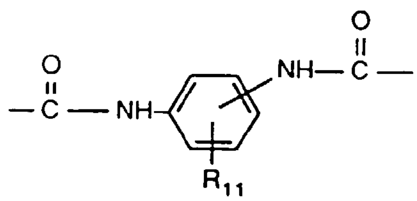 , 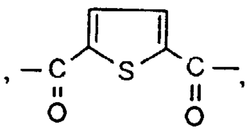 ,

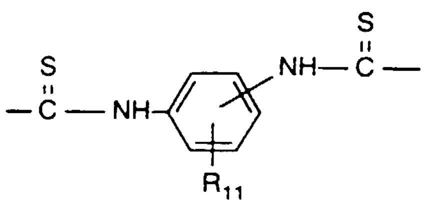 , 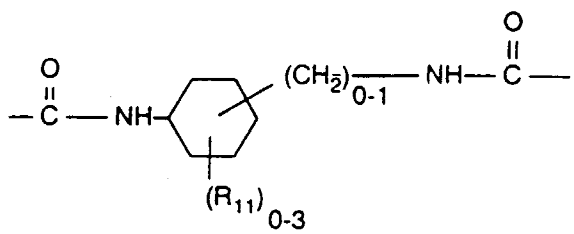 ,

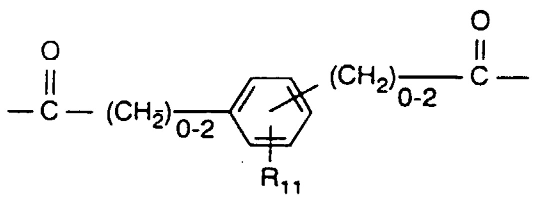 , 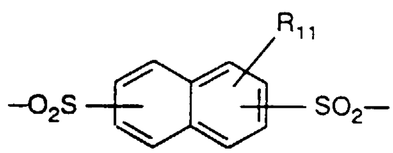 ,

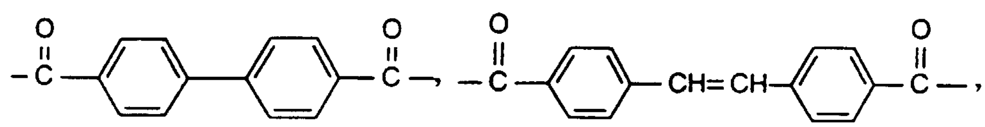 ,

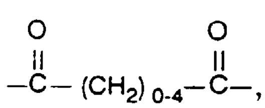 , 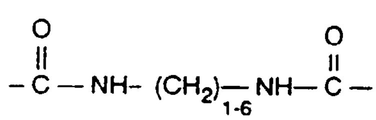 , 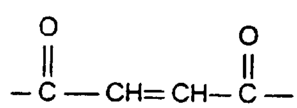

or

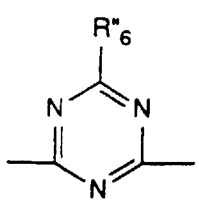 ,

wherein $R_{11}$ is sulfo, methyl, methoxy, chlorine, carboxyl or preferably hydrogen, and $R''_6$ is hydroxyl, chlorine, methylthio or ethylthio, methoxy, ethoxy, n- or isopropoxy, amino, methylamino, ethylamino, β-hydroxyethylamino, N,N-di-β-hydroxyethylamino, β-sulfoethylamino, cyclohexylamino, o-, m- or p-methylphenylamino, o-, m-, or p-methoxyphenylamino, o-, m- or p-chlorophenylamino, o-, m- or p-sulfophenylamino, 2,4- or 2,5-disulfophenylamino, o-carboxyphenylamino, N-ethyl-N-phenylamino, N-methyl-N-phenylamino or morpholino.

**16.** A process according to any one of claims 1 to 15, characterized in that a compound of formula (10) is used wherein B is a radical of formula

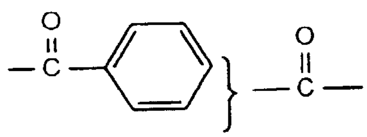

or

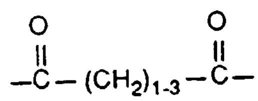

.

**17.** A process according to claim 1, characterized in that a compound of formula (9) wherein $R_1$ is hydrogen, unsubstituted $C_1$-$C_4$alkyl or $C_1$-$C_4$alkyl which is substituted by hydroxyl, sulfo, sulfato, chlorine, cyano, or acetoxy and/or, with the exception of methyl, may be interrupted by a group -O-, cyclopentyl or cyclohexyl which are unsubstituted or substituted by 1 to 3 methyl groups, unsubstituted phenyl or phenyl which is substituted by sulfo, nitro, chlorine, methyl, methoxy, N-methylamino or N-ethylamino, N,N-dimethylamino or N,N-diethylamino, acetylamino, propionylamino, benzoylamino, methoxycarbonyl, ethoxycarbonyl, carboxyl or methylsulfonyl, or is unsubstituted 1- or 2-naphthyl or 1- or 2-naphthyl which is substituted by sulfo, nitro and/or chlorine, or is unsubstituted benzyl or benzyl which is substituted by methyl, methoxy, sulfo and/or chlorine, A is a $C_2$-$C_4$alkylene radical which is unsubstituted or substituted by hydroxyl, sulfo, sulfato, methoxy, carboxyl or sulfophenyl, $-CH_2-CH_2-Z'-CH_2-CH_2-$ wherein Z' is -O-, -S-, $-SO_2-$, -NH- or $-N(CH_3)-$, a cyclohexylene radical which is unsubstituted or substituted by 1 to 3 methyl groups, an unsubstituted or sulfo-substituted 1,3- or 1,4-phenylene radical or is a $C_1$-$C_3$alkylene-phenylene or $C_1$-$C_2$alkylene-phenylene-$C_1$-$C_2$alkylene radical wherein the phenylene moiety is unsubstituted or substituted by methyl, methoxy, chlorine or sulfo,
X is a group $-N(R_5)-$ wherein $R_5$ is hydrogen, $C_1$-$C_4$alkyl, cyclohexyl, unsubstituted phenyl or benzyl or phenyl or benzyl which are substituted by sulfo, chlorine, methyl and/or methoxy, or where the group

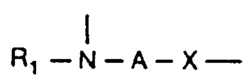

is the piperazine-1,4-diyl radical,
Y is methoxy, methyl, chlorine or sulfo,
n is 0 or 1, Z is hydroxyl, methyl or ethyl,
p is 1,
$R_2$ and $R_3$ are each hydrogen, fluorine, chlorine, bromine, methyl, methoxy, acetylamino, phenoxy or cyano
is reacted with a compound of formula (10), and, optionally, the product obtained is reacted with a compound of formula (11) wherein
R* is $C_1$-$C_4$alkyl, unsubstituted phenyl or benzyl or phenyl or benzyl which are substituted by methyl, methoxy, chlorine and/or sulfo, or is a radical of formula

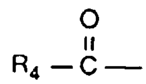 ,

wherein $R_4$ is methyl, ethyl or unsubstituted phenyl or phenyl which is substituted by sulfo, chlorine, methyl and/or methoxy, m being any integer from 1 to 3 in the oligomer mixture obtained.

**18.** A process according to claim 1, characterized in that a compound of formula

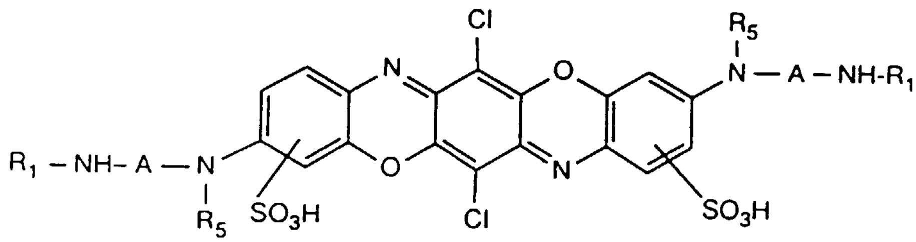

wherein $R_1$ is hydrogen, $C_1$-$C_4$alkyl, cyclohexyl, unsubstituted phenyl or benzyl, or phenyl or benzyl which are substituted by sulfo, chlorine, methyl and/or methoxy,

A is a $C_2$-$C_4$alkylene radical which is unsubstituted or substituted by hydroxyl, sulfo, sulfato, methoxy, carboxyl or sulfophenyl,
$R_5$ is hydrogen, methyl or ethyl, is reacted with a compound of formula (10) wherein
B is a radical of formula

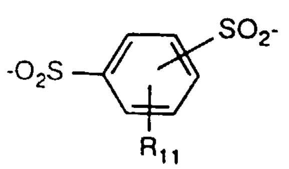 , 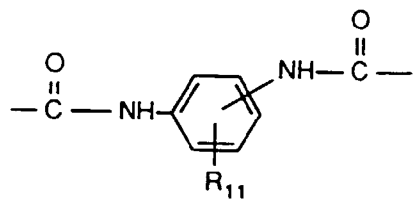 , 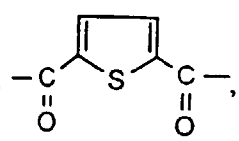 ,

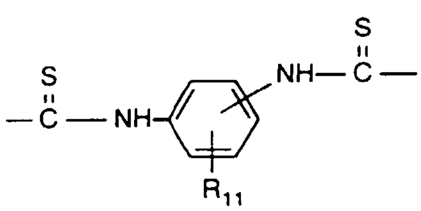 , 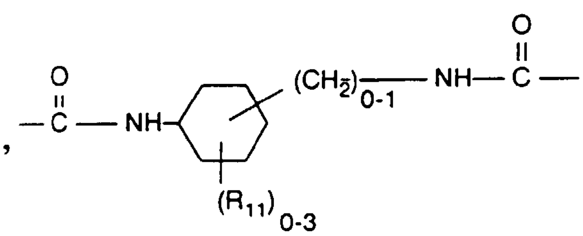 ,

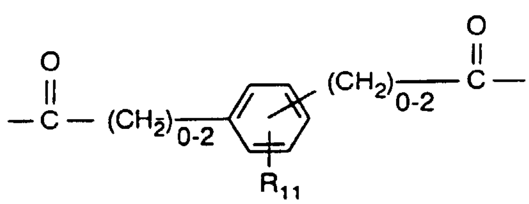 , 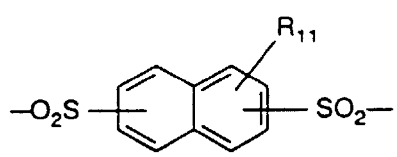 ,

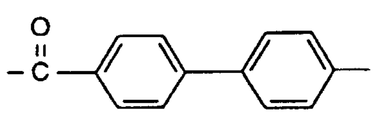, 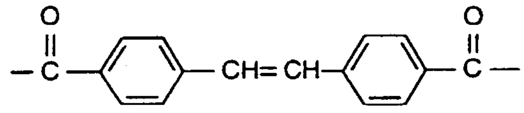

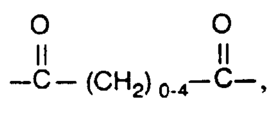, 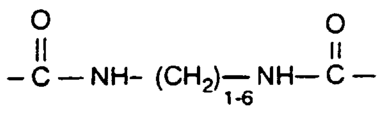, 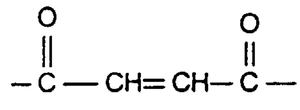

or

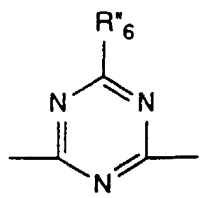,

wherein $R_{11}$ is sulfo, methyl, methoxy, chlorine, carboxyl or preferably hydrogen, and $R''_6$ is hydroxyl, chlorine, methylthio or ethylthio, methoxy, ethoxy, n- or isopropoxy, amino, methylamino, ethylamino, β-hydroxyethylamino, N,N-di-β-hydroxyethylamino, β-sulfoethylamino, cyclohexylamino, o-, m- or p-methylphenylamino, o-, m- or p-methoxyphenylamino, o-, m- or p-chlorophenylamino, o-, m- or p-sulfophenylamino, 2,4- or 2,5-disulfophenylamino, o-carboxyphenylamino, N-ethyl-N-phenylamino, N-methyl-N-phenylamino or morpholino, and, optionally, the product obtained is reacted with a compound of formula (11) wherein R* is methyl, ethyl, benzyl, acetylamino or benzoylamino.

**19.** A process according to claim 1, characterized in that a compound of formula

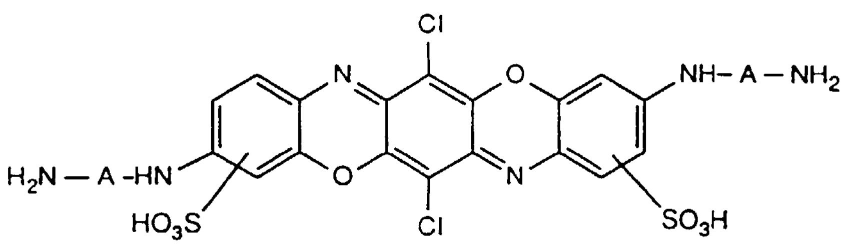

wherein A is a 1,2-ethylene or 1,2- or 1,3-propylene radical which is unsubstituted or substituted by hydroxyl or sulfato, is reacted with a compound of formula (10) wherein B is a radical of formula

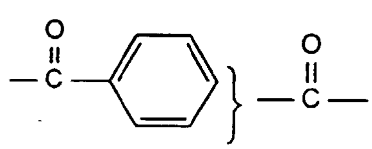

or

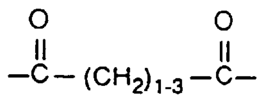

and, optionally, the product obtained is reacted with a compound of formula (11) wherein R* is methyl, ethyl, benzyl, acetylamino or benzoylamino.

**20.** Use of the mixture of different oligomeric compounds of formula (1) obtained as claimed in claim 1 as dyes for dyeing or printing nitrogen-containing and, in particular, cellulosic fibre materials.

**21.** Use according to claim 20, characterised in that blends of synthetic fibres and cellulosic fibre materials, in particular polyester/cotton blends, are dyed in the presence of a disperse dye for the polyester fibres under the dyeing conditions for polyester fibres.

**22.** A process for dyeing polyester/cotton blends with disperse and direct dyes, characterized in that mixtures of different oligomeric compounds of formula (1) obtained as claimed in claim 1 are used as dyes in a one-step, single bath process and in addition to said disperse dyes, and dyeing takes place from an aqueous liquor in the temperature range from 100 to 150°C, preferably from 120 to 130°C, and in the pH range from 4 to 7.5.

**Revendications**

**Revendications pour les Etats contractants suivants : BE, CH, DE, FR, GB, IT, LI**

**1.** Mélange d'oligomères contenant au moins deux composés de formule

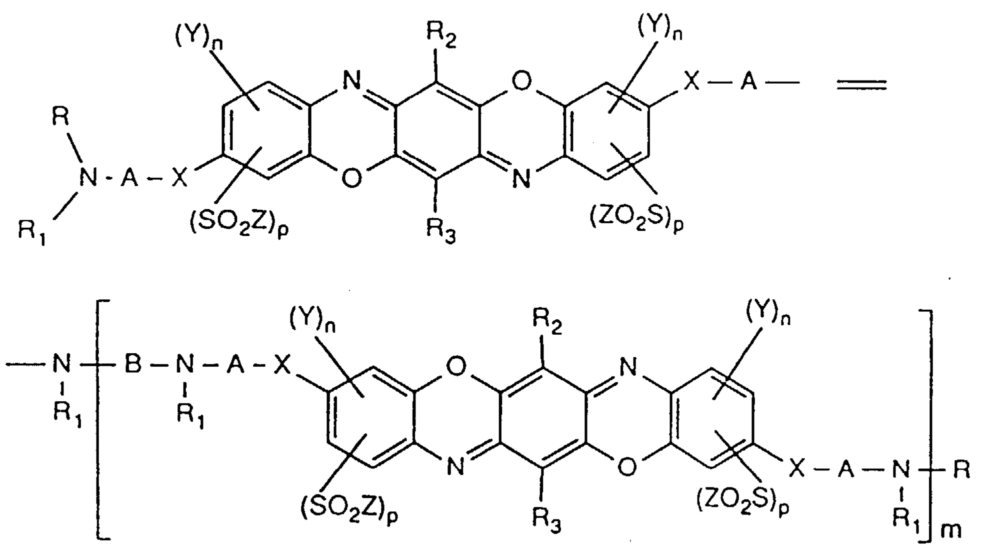

(1)

dans laquelle

$R_1$ représente un atome d'hydrogène, un groupe alkyle en $C_{1-4}$ non substitué ou portant un substituant hydroxy, sulfo, sulfato, chloro, cyano ou acétoxy et/ou pouvant être interrompu, sauf en ce qui concerne le groupe méthyle, par un groupe -O-, un résidu cyclopentyle ou cyclohexyle non substitué ou portant 1 à 3 substituants méthyle, un résidu phényle non substitué ou portant un substituant sulfo, nitro, chloro, méthyle, méthoxy, N-méthylamino ou N-éthylamino, N,N-diméthylamino ou N,N-diéthylamino, acétylamino, propionylamino, benzoylamino, méthoxycarbonyle, éthoxycarbonyle, carboxy ou méthylsulfonyle, un résidu 1-naphtyle ou 2-naphtyle non substitué ou portant un substituant sulfo, nitro et/ou chloro, ou encore un groupe benzyle non

substitué ou portant un substituant méthyle, méthoxy, sulfo et/ou chloro, R a indépendamment la signification de $R_1$ ou représente un résidu pyridine, pyrimidine, quinoxaline ou triazine, ces résidus pouvant être non substitués ou porter un substituant hydroxyle; alkyle en $C_{1-4}$; phényle; alcoxy en $C_{1-4}$; alkylthio en $C_{1-4}$; amino; N-mono- ou N,N-di(alkyle en $C_{1-4}$)-amino dont le ou les groupes alkyle portent un substituant hydroxy, carboxy, cyano, sulfo, sulfato ou alcoxy en $C_{1-4}$; cyclohexylamino; phénylamino ou N-(alkyle en $C_{1-4}$)-N-phénylamino portant sur le noyau phényle un substituant alkyle en $C_{1-4}$, alcoxy en $C_{1-4}$, phénoxy, carboxy, sulfo et/ou halogéno; morpholino; ou 3-carboxy- ou 3-carbamoylpyridine-1-yle,
ou R représente un résidu de formule

$$R_4\text{-C(=O)-}$$

dans laquelle $R_4$ représente un résidu quinoxaline ou pyrimidine ou a la signification indiquée ci-dessus pour $R_1$, mais n'est pas un atome d'hydrogène,

A représente un résidu alkylène en $C_{2-6}$ non substitué ou portant un substituant hydroxy, sulfo, sulfato, alcoxy en $C_{1-4}$, carboxy, cyano, halogéno, phényle, sulfophényle ou (alcoxy en $C_{2-5}$)-carbonyle et/ou pouvant être interrompu par 1 à 2 groupes -O-, -N($R_8$)-, où $R_8$ représente un résidu alkyle en $C_{1-4}$, acétyle ou en particulier un atome d'hydrogène, -S- ou -$SO_2$-; un résidu cyclopentylène ou cyclohexylène non substitué ou portant un ou plusieurs substituants alkyle en $C_{1-3}$, un résidu phénylène, biphénylène ou naphtylène non substitué ou portant un substituant alkyle en $C_{1-4}$, alcoxy en $C_{1-4}$, sulfo, halogéno ou carboxy, ou un résidu (alkylène en $C_{1-6}$)-phényléne, phénylène-(alkylène en $C_{1-6}$)-phénylène, (alkylène en $C_{1-3}$)-phénylène-(alkylène en $C_{1-3}$) ou méthylène-naphtylène-méthylène, la partie phénylène et naphtylène de ces résidus aralkylène pouvant porter 1 ou 2 substituants choisis dans le groupe formé par les résidus sulfo, carboxy, sulfamoyle, carbamoyle, méthyle, éthyle, méthoxy, éthoxy, nitro, chloro, amino, N-méthyl- et N-éthylamino, N,N-diméthyl- et N,N-di-éthylamino et phénylamino,
X représente un groupe -O-, -S- ou -N($R_5$)- où $R_5$ a la signification indiquée ci-dessus pour R1, ou dans lequel le groupe

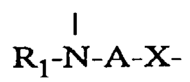

représente un résidu pipérazine-1,4-diyle,
Y est un résidu alkyle en $C_{1-4}$, alcoxy en $C_{1-4}$, halogéno, sulfo, carboxy, carbamoyle, N-mono- ou N,N-di(alkyle en $C_{1-4}$)-carbamoyle, N-phényl- ou N,N-diphénylcarbamoyle, sulfamoyle, N-mono- ou N,N-di-(alkyle en $C_{1-4}$)-sulfamoyle ou N-phényl- ou N,N-diphénylsulfamoyle,
Z représente un groupe hydroxy ou alkyle en $C_{1-4}$,
$R_2$ et $R_3$ représentent, indépendamment l'un de l'autre, un atome d'hydrogène, un atome d'halogène, un groupe cyano, alkyle en $C_{1-4}$, alcoxy en $C_{1-4}$, sulfo, carboxy, carbamoyle, phénylcarbamoyle, (alcanoyle en $C_{2-5}$)-amino, ou un résidu phényle, benzyle, benzoylamino ou phénoxy éventuellement substitués par un groupe alkyle en $C_{1-4}$, alcoxy en $C_{1-4}$, acétylamino, halogéno, nitro ou sulfo,
B représente un chaînon pontant de formule

$$Q'\text{-}E'\text{-}Q' \qquad (3'),$$

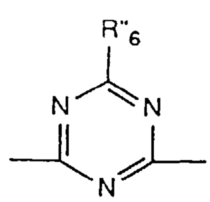

(4'),

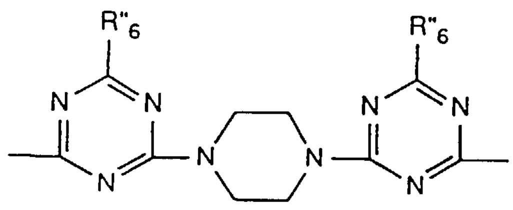

(6'),

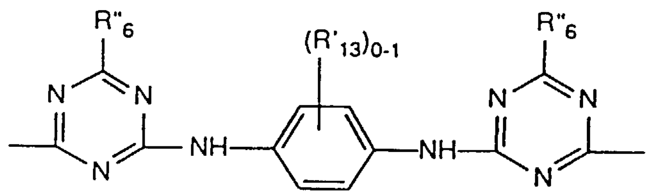

(7')

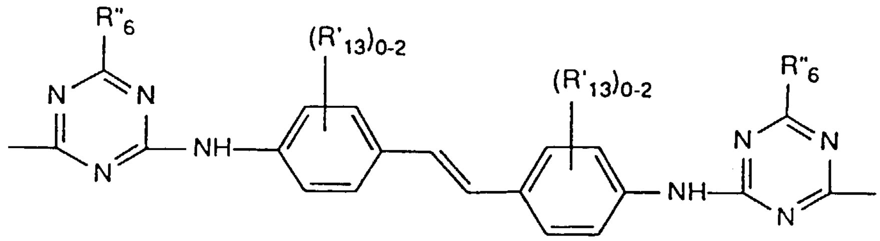

(8)

où Q' représente un groupe

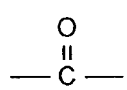 , 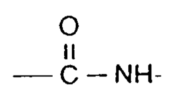 , 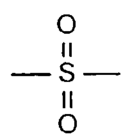 ou 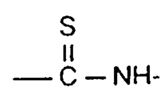

E représente une liaison directe, un groupe alkylène en $C_{1-6}$ ou alcénylène en $C_{2-6}$ non substitué ou portant un substituant hydroxy, sulfo, sulfato, méthoxy, carboxy, phényle ou sulfophényle, un résidu cyclohexylène ou (alkylène en $C_{1-2}$)-cyclohexylène non substitué ou portant de 1 à 3 substituants méthyle, un résidu pipérazine-1,4-diyle, thiophène-2,5-diyle, biphényl-4,4'-diyle, stilbène-4,4'-diyle, phénylène ou naphtylène non substitué ou portant un substituant alkyle en $C_{1-4}$, alcoxy en $C_{1-4}$, sulfo, halogéno ou carboxy, ou un résidu (alkylène en $C_{1-3}$)-phényléne ou (alkylène en $C_{1-2}$)-phénylène-(alkylène en $C_{1-2}$) non substitué ou portant sur le noyau phénylène un substituant méthyle, méthoxy, chloro ou sulfo, $R''_6$ représente un atome de chlore, un groupe hydroxy, alcoxy en $C_{1-4}$, alkylthio en $C_{1-2}$, amino, N-mono- ou N,N-di(alkyle en $C_{1-4}$)-amino non substitué ou portant sur le ou les résidus alkyle un substituant hydroxy, sulfo ou sulfato, un groupe cylohexylamino, un groupe phénylamino ou N-(alkyle en $C_{1-4}$)-N-phénylamino non substitué ou portant sur le noyau phényle un groupe méthyle, méthoxy, carboxy, sulfo ou chloro, ou un groupe morpholino, et
$R'_{13}$ représente un groupe sulfo, méthyle ou méthoxy,
m est un nombre compris entre 1 et 6, et
n et p représentent, indépendamment l'un de l'autre, un nombre égal à 0 ou 1, les différents composés de formule (1) se distinguant uniquement par la valeur que prend la variable m.

**2.** Mélange d'oligomères conforme à la revendication 1, caractérisé en ce que $R_1$ représente un atome d'hydrogène, un groupe alkyle en $C_{1-4}$, cyclohexyle, ou phényle ou benzyle non substitué ou portant un substituant sulfo, chloro, méthyle et/ou méthoxy.

**3.** Mélange d'oligomères conforme à une des revendications 1 et 2 caractérisé en ce que $R_1$ représente un atome d'hydrogène.

**4.** Mélange d'oligomères conforme à une des revendications 1 à 3, caractérisé en ce que R représente un atome d'hydrogène, un groupe alkyle en $C_{1-4}$, ou phényle ou benzyle non substitué ou portant un substituant sulfo, chloro, méthyle et/ou méthoxy ou un groupe de formule

$$R_4\text{-}C(=O)\text{-}$$

où $R_4$ représente un résidu méthyle, éthyle ou phényle non substitué ou portant un substituant sulfo, chloro, méthyle et/ou méthoxy.

**5.** Mélange d'oligomères conforme à une des revendications 1 à 4, caractérisé en ce que R représente un atome d'hydrogène, un groupe méthyle, éthyle, benzyle, acétylamino ou benzoylamino, en particulier un atome d'hydrogène.

**6.** Mélange d'oligomères conforme à une des revendications 1 à 5, caractérisé en ce que A représente un résidu alkylène en $C_{2-4}$ non substitué ou portant un résidu hydroxy, sulfo, sulfato, méthoxy, carboxy ou sulfophényle, un groupe $-CH_2-CH_2-Z'-CH_2-CH_2-$ où Z' est un chaînon -O-, -S-, $-SO_2-$, -NH- ou $-N(CH_3)-$, un résidu cyclohexylène non substitué ou portant de 1 à 3 résidus méthyle, un résidu 1,3-phénylène ou 1,4-phénylène non substitué ou sulfoné, ou un résidu (alkylène en $C_{1-3}$)-phénylène ou (alkylène en $C_{1-2}$)-phénylène-(alkylène en $C_{1-2}$) dans lesquels le noyau phénylène peut être non substitué ou porter un résidu méthyle, méthoxy, chloro ou sulfo.

**7.** Mélange d'oligomères conforme à une des revendications 1 à 6, caractérisé en ce que A représente un résidu alkylène en $C_{2-4}$ non substitué ou portant un substituant hydroxy, sulfo, sulfato, méthoxy, carboxy ou sulfophényle.

**8.** Mélange d'oligomères conforme à une des revendications 1 à 7, caractérisé en ce que A représente un résidu 1,2-éthylène ou 1,2-propylène ou 1,3-propylène non substitué ou portant un substituant hydroxy ou sulfato.

**9.** Mélange d'oligomères conforme à une des revendications 1 à 8, caractérisé en ce que X représente un résidu -N($R_5$)- où $R_5$ représente un atome d'hydrogène, un groupe alkyle en $C_{1-4}$, cyclohexyle, ou un groupe phényle ou benzyle non substitué ou portant un substituant sulfo, chloro, méthyle et /ou méthoxy.

**10.** Mélange d'oligomères conforme à une des revendications 1 à 9, caractérisé en ce que $R_5$ représente un atome d'hydrogène ou un groupe alkyle en $C_{1-4}$, en particulier un atome d'hydrogène.

**11.** Mélange d'oligomères conforme à une des revendications 1 à 10, caractérisé en ce que n vaut 0.

**12.** Mélange d'oligomères conforme à une des revendications 1 à 11, caractérisé en ce que p vaut 1 et Z représente un groupe hydroxy ou alkyle en $C_{1-4}$, en particulier un groupe hydroxy.

**13.** Mélange d'oligomères conforme à une des revendications 1 à 12, caractérisé en ce que $R_2$ et $R_3$ représentent chacun un atome d'hydrogène, un atome de fluor, de chlore ou de brome, un résidu méthyle, méthoxy, acétylamino, phénoxy ou cyano.

**14.** Mélange d'oligomères conforme à une des revendications 1 à 13 caractérisé en ce que $R_2$ et $R_3$ représentent chacun un atome de chlore.

**15.** Mélange d'oligomères conforme à une des revendications 1 à 14, caractérisé en ce que B représente un chaînon pontant de formule

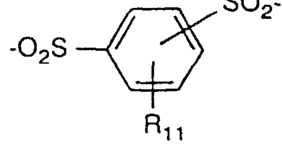 , 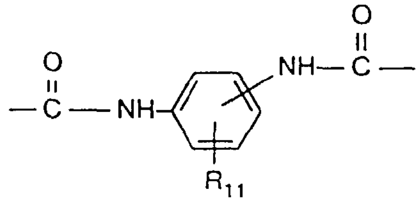 , 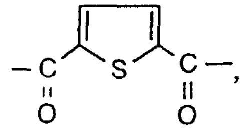 ,

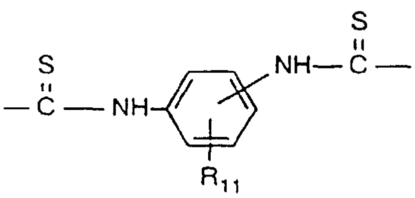, 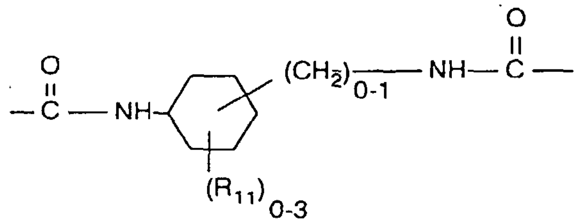,

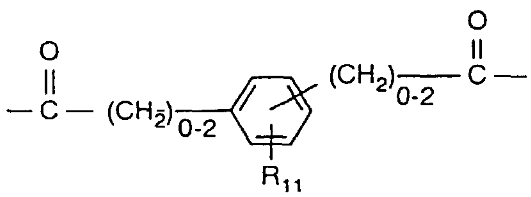, 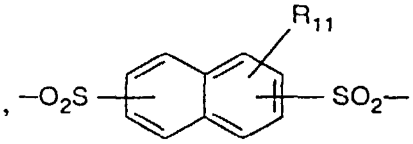,

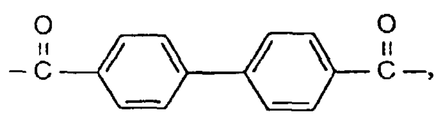, 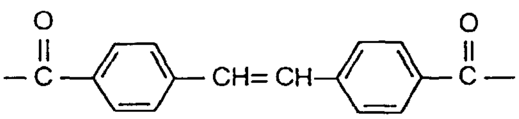,

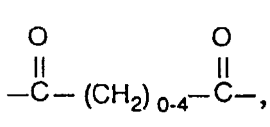, 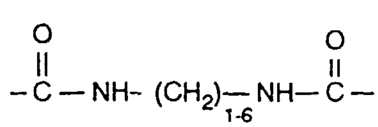, 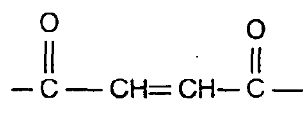

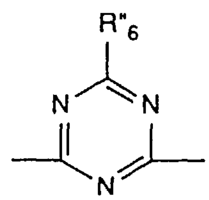

dans lesquelles $R_{11}$ représente un résidu sulfo, méthyle, méthoxy, chloro, carboxy ou, de préférence, un atome d'hydrogène, et $R''_6$ représente un groupe hydroxy, chloro, méthylthio ou éthylthio, méthoxy, éthoxy, *n*- ou *iso*-propoxy, amino, méthylamino, éthylamino, β-hydroxyéthylamino, N,N-di-β-hydroxyéthylamino, β-sulfoéthylamino, cyclohexylamino, o-, m- ou p-méthylphénylamino, o-, m- ou p-méthoxyphénylamino, o-, m- ou p-chlorophénylamino, o-, m- ou p-sulfophénylamino, 2,4- ou 2,5-disulfophénylamino, o-carboxyphénylamino, N-éthyl-N-phénylamino, N-méthyl-N-phénylamino ou morpholino.

**16.** Mélange d'oligomères conforme à une des revendications 1 à 15, caractérisé en ce que B représente un résidu de formule

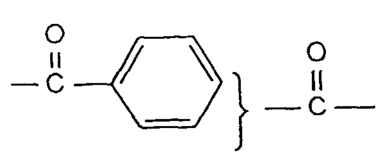

ou

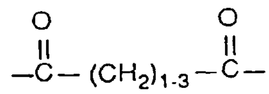

**17.** Mélange d'oligomères conforme à la revendication 1 de formule (1) dans lequel $R_1$ représente un atome d'hydrogène, un groupe alkyle en $C_{1-4}$ non substitué ou portant un substituant hydroxy, sulfo, sulfato, chloro, cyano ou acétoxy et/ou pouvant être interrompu, à l'exception du groupe méthyle, par un groupe -O-, un groupe cyclopentyle ou cyclohexyle non substitué ou portant de 1 à 3 substituants méthyle, un groupe phényle non substitué ou portant un substituant sulfo, nitro, chloro, méthyle, méthoxy, N-méthylamino ou N-éthylamino, N,N-diméthylamino ou N,N-diéthylamino, acétylamino, propionylamino, benzoylamino, méthoxycarbonyle, éthoxycarbonyle, carboxy ou méthylsulfonyle, un groupe 1-naphtyle ou 2-naphtyle non substitué ou portant un substituant sulfo, nitro et/ou chloro, ou encore un résidu benzyle non substitué ou portant un résidu méthyle, méthoxy, sulfo, et/ou chloro, R représente un atome d'hydrogène, un groupe alkyle en $C_{1-4}$, un groupe phényle ou benzyle non substitué ou portant un substituant méthyle, méthoxy, chloro et/ou sulfo, ou un résidu de formule

$$R_4\text{-C(=O)-}$$

dans laquelle $R_4$ représente un groupe méthyle, éthyle ou un résidu phényle non substitué ou portant un résidu sulfo, chloro, méthyle et/ou méthoxy,

A représente un groupe alkylène en $C_{2-4}$ non substitué ou portant un substituant hydroxy, sulfo, sulfato, méthoxy, carboxy ou sulfophényle, un groupe $-CH_2-CH_2-Z'-CH_2-CH_2-$ où Z' représente un groupe -O-, -S-, $-SO_2-$, -NH- ou $-N(CH_3)-$, un résidu cyclohexylène non substitué ou portant de 1 à 3 substituants méthyle, un résidu 1,3-phénylène ou 1,4-phénylène non substitué ou portant un groupe sulfo ou un résidu (alkylène en $C_{1-3}$)-phénylène ou (alkylène en $C_{1-2}$)-phénylène-(alkylène en $C_{1-2}$) non substitué ou portant, sur leur noyau phénylène, un substituant méthyle, méthoxy, chloro ou sulfo,
X représente un groupe $-N(R_5)-$ où $R_5$ représente un atome d'hydrogène, un groupe alkyle en $C_{1-4}$, cyclohexyle, phényle ou benzyle non substitué ou portant un substituant sulfo, chloro, méthyle et/ou méthoxy, ou dans lequel le groupe

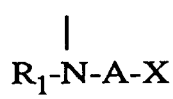

représente un résidu pipérazine-1,4-diyle,
Y est un groupe méthoxy, méthyle, chloro ou sulfo,
n vaut 0 ou 1,
Z représente un groupe hydroxy, méthyle ou éthyle,
p vaut 1,
$R_2$ et $R_3$ représentent chacun un atome d'hydrogène, un atome de fluor, de chlore ou de brome, un résidu méthyle, méthoxy, acétylamino, phénoxy ou cyano et
m est un nombre compris entre 1 et 3.

**18.** Mélange d'oligomères conforme à la revendication 1 de formule

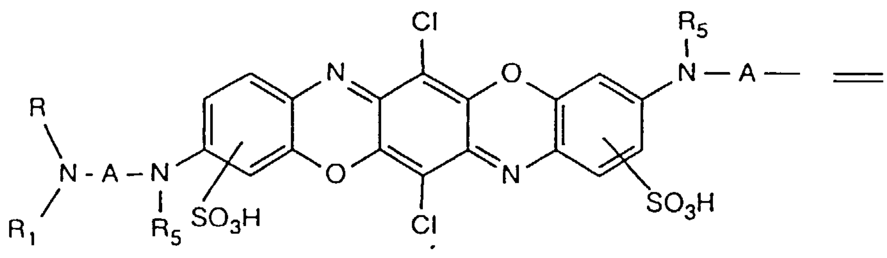

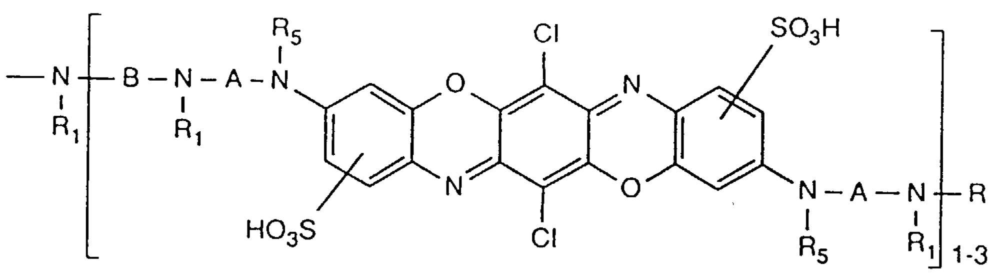

(1a),

dans lesquelles

$R_1$ représente un atome d'hydrogène, un groupe alkyle en $C_{1-4}$, cyclohexyle, un groupe phényle ou benzyle non substitué ou portant un substituant sulfo, chloro, méthyle et/ou méthoxy,
R représente un atome d'hydrogène, un groupe méthyle, éthyle, benzyle, acétylamino ou benzoylamino,
A représente un résidu alkylène en $C_{2-4}$ non substitué ou portant un résidu hydroxy, sulfo, sulfato, méthoxy, carboxy ou sulfophényle,
$R_5$ représente un atome d'hydrogène, méthyle ou éthyle, et
B un résidu de formule

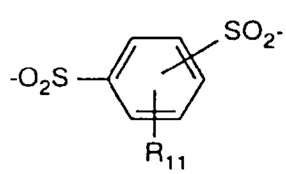 , 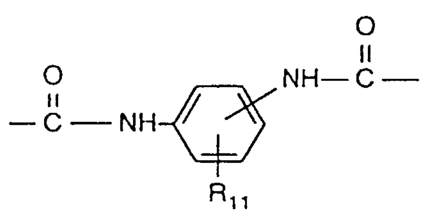 , 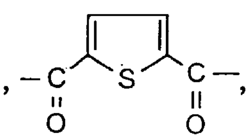 ,

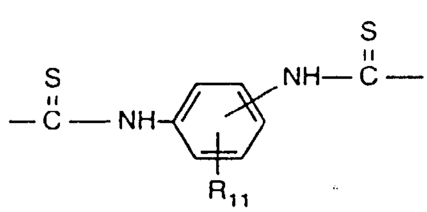 , 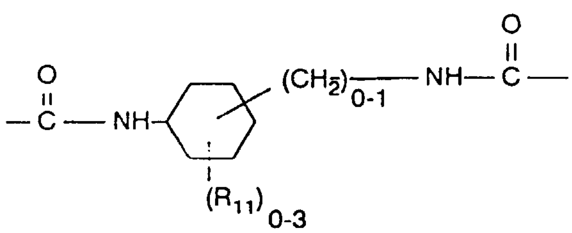

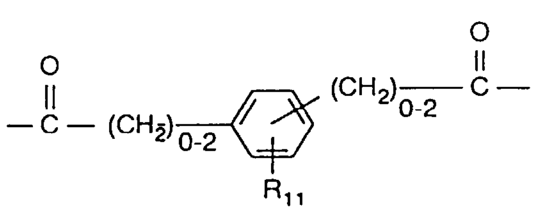 , 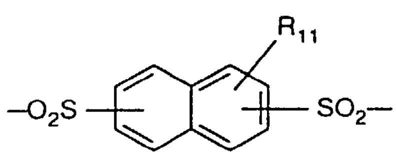 ,

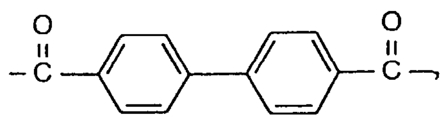,
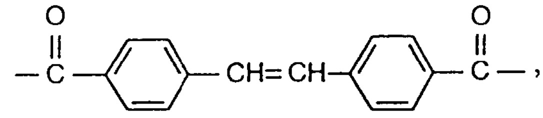,

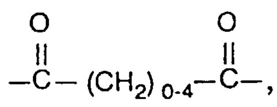,
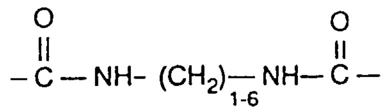,
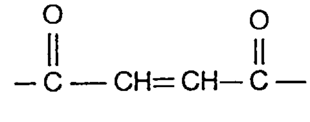

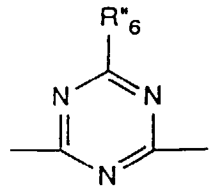,

où $R_{11}$ représente un résidu sulfo, méthyle, méthoxy, chloro, carboxy ou, de préférence, un atome d'hydrogène, et $R''_6$ est un groupe hydroxy, chloro, méthylthio ou éthylthio, méthoxy, éthoxy, *n*- ou *iso*-propoxy, amino, méthylamino, éthylamino, β-hydroxyéthylamino, N,N-di-β-hydroxyéthylamino, β-sulfoéthylamino, cyclohexylamino, o-, m- ou p-méthylphénylamino, o-, m- ou p-méthoxyphénylamino, o-, m- ou p-chlorophénylamino, o-, m- ou p-sulfophénylamino, 2,4- ou 2,5-disulfophénylamino, o-carboxyphénylamino, N-éthyl-N-phénylamino, N-méthyl-N-phénylamino ou morpholino.

**19.** Mélange d'oligomères conforme à la revendication 1 de formule

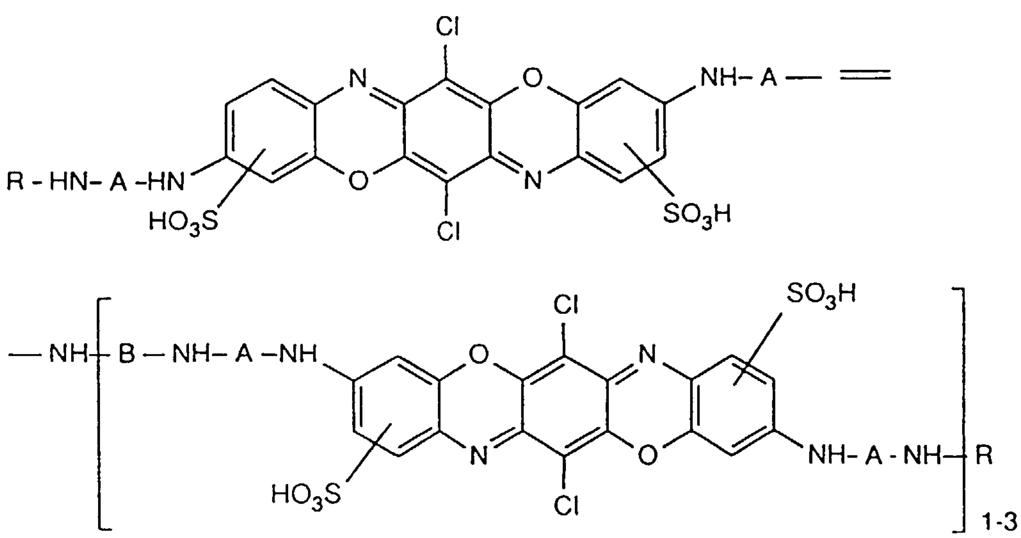

(1b),

où R représente un groupe méthyle, éthyle, benzyle, acétylamino, benzoylamino ou, en particulier, un atome d'hydrogène, A représente un groupe 1,2-éthylène ou 1,2- ou 1,3-propylène non substitué ou portant un substituant hydroxy ou sulfato et B représente un résidu de formule

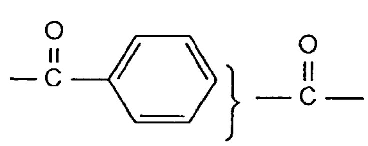

ou

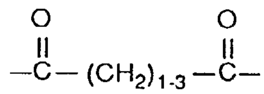

**20.** Mélange d'oligomères conforme à une des revendications 1 à 19 contenant un composé de formule (1) pour lequel m = 1, un composé de formule (1) pour lequel m = 2 et un composé de formule (1) pour lequel m = 3.

**21.** Procédé de préparation de mélanges d'oligomères conformes à la revendication 1, caractérisé

a) en ce que l'on effectue la condensation d'un composé de formule

(9) 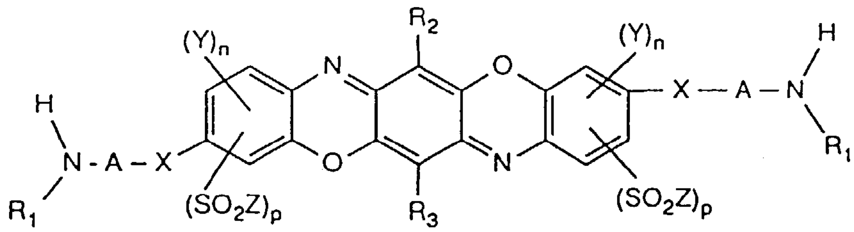

avec un composé de formule

(10) T-B-T

et b) en ce que l'on fait réagir éventuellement le produit obtenu dans a) avec un composé de formule

(11) R*-T

où A, B, $R_1$, $R_2$, $R_3$, X, Y, Z, p et n ont la signification indiquée ci-avant, R* a la signification indiquée précédemment pour R, à l'exception d'un atome d'hydrogène, et T représente un atome d'halogène, de préférence un atome de chlore.

**22.** Utilisation des mélanges de différents oligomères de formule (1) conformes à la revendication 1 en tant que colorants pour la teinture et l'impression de matériaux fibreux contenant des atomes d'azote et en particulier des matériaux fibreux cellulosiques.

**23.** Utilisation conforme à la revendication 22, caractérisée en ce que l'on teint des mélanges de fibres contenant des fibres synthétiques et des matériaux fibreux cellulosiques, en particulier des tissus mixtes polyester/coton, en présence d'un colorant de dispersion pour les fibres de polyester dans des conditions de teinture du polyester.

**24.** Procédé de teinture de tissus mixtes polyester/coton avec des colorant directs et des colorants de dispersion, caractérisé en ce que l'on utilise dans un procédé à bain unique et étape unique, en tant que colorants, en plus des colorants de dispersion, des mélanges de différents composés oligomères de formule (1) conformes à la revendication 1, et en ce que l'on effectue la teinture dans un bain aqueux à des températures comprises entre 100 et 150 °C, de préférence entre 120 et 130 °C et à un pH compris entre 4 et 7,5.

**Revendications pour l'Etat contractant suivant : ES**

**1.** Procédé de préparation de mélanges d'oligomères contenant au moins deux comnosés de formule

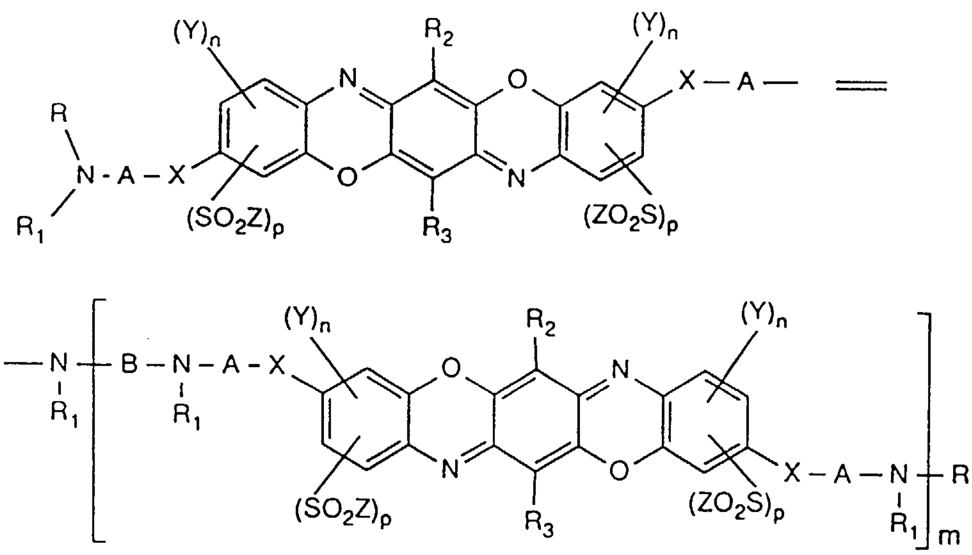

(1),

dans laquelle

$R_1$ représente un atome d'hydrogène, un groupe alkyle en $C_{1-4}$ non substitué ou portant un substituant hydroxy, sulfo, sulfato, chloro, cyano ou acétoxy et/ou pouvant être interrompu, sauf en ce qui concerne le groupe méthyle, par un groupe -O-, un résidu cyclopentyle ou cyclohexyle non substitué ou portant 1 à 3 substituants méthyle, un résidu phényle non substitué ou portant un substituant sulfo, nitro, chloro, méthyle, méthoxy, N-méthylamino ou N-éthylamino, N,N-diméthylamino ou N,N-diéthylamino, acétylamino, propionylamino, benzoylamino, méthoxycarbonyle, éthoxycarbonyle, carboxy ou méthylsulfonyle, un résidu 1-naphtyle ou 2-naphtyle non substitué ou portant un substituant sulfo, nitro et/ou chloro, ou encore un groupe benzyle non substitué ou portant un substituant méthyle, méthoxy, sulfo et/ou chloro, R a indépendamment la signification de $R_1$ ou représente un résidu pyridine, pyrimidine, quinoxaline ou triazine, ces résidus pouvant être non substitués ou porter un substituant hydroxyle; alkyle en $C_{1-4}$; phényle; alcoxy en $C_{1-4}$; alkylthio en $C_{1-4}$; amino; N-mono- ou N,N-di(alkyle en $C_{1-4}$)-amino dont le ou les groupes alkyle portent un substituant hydroxy, carboxy, cyano, sulfo, sulfato ou alcoxy en $C_{1-4}$; cyclohexylamino; phénylamino ou N-(alkyle en $C_{1-4}$)-N-phénylamino portant sur le noyau phényle un substituant alkyle en $C_{1-4}$, alcoxy en $C_{1-4}$, phénoxy, carboxy, sulfo et/ou halogéno; morpholino; ou 3-carboxy- ou 3-carbamoylpyridine-1-yle,
ou R représente un résidu de formule

$$R_4\text{-C(=O)-}$$

dans laquelle $R_4$ représente un résidu quinoxaline ou pyrimidine ou a la signification indiquée ci-dessus pour $R_1$, mais n'est pas un atome d'hydrogène,

A représente un résidu alkylène en $C_{2-6}$ non substitué ou portant un substituant hydroxy, sulfo, sulfato, alcoxy en $C_{1-4}$, carboxy, cyano, halogéno, phényle, sulfophényle ou (alcoxy en $C_{2-5}$)-carbonyre et/ou pouvant être interrompu par 1 à 2 groupes -O-, -N($R_8$)-, où $R_8$ représente un résidu alkyle en $C_{1-4}$, acétyle ou en particulier un atome d'hydrogène, -S- ou -$SO_2$-; un résidu cyclopentylène ou cyclohexylène non substitué ou portant un ou plusieurs substituants alkyle en $C_{1-3}$, un résidu phénylène, biphénylène ou naphtylène non substitué ou portant un substituant alkyle en $C_{1-4}$, alcoxy en $C_{1-4}$, sulfo, halogéno ou carboxy, ou un résidu (alkylène en $C_{1-6}$)-phénylène, phénylène-(alkylène en $C_{1-6}$)-phénylène, (alkylène en $C_{1-3}$)-phénylène-(alkylène en $C_{1-3}$) ou méthylène-naphtylène-méthylène, la partie phénylène et naphtylène de ces résidus aralkylène pouvant porter 1 ou 2 substituants choisis dans le groupe formé par les résidus sulfo, carboxy, sulfamoyle, carbamoyle, méthyle, éthyle, méthoxy, éthoxy, nitro, chloro, amino, N-méthyl- et N-éthylamino, N,N-diméthyl- et N,N-di-éthylamino et phénylamino,

X représente un groupe -O-, -S- ou -N($R_5$)- où $R_5$ a la signification indiquée ci-dessus pour $R_1$, ou dans lequel le groupe

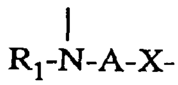

représente un résidu pipérazine-1,4-diyle,

Y est un résidu alkyle en $C_{1-4}$, alcoxy en $C_{1-4}$, halogéno, sulfo, carboxy, carbamoyle, N-mono- ou N,N-di(alkyle en $C_{1-4}$)-carbamoyle, N-phényl- ou N,N-diphénylcarbamoyle, sulfamoyle, N-mono- ou N,N-di-(alkyle en $C_{1-4}$)-sulfamoyle ou N-phényl- ou N,N-diphénylsulfamoyle,

Z représente un groupe hydroxy ou alkyle en $C_{1-4}$,

$R_2$ et $R_3$ représentent, indépendamment l'un de l'autre, un atome d'hydrogène, un atome d'halogène, un groupe cyano, alkyle en $C_{1-4}$ alcoxy en $C_{1-4}$, sulfo, carboxy, carbamoyle, phénylcarbamoyle, (alcanoyle en $C_{2-5}$) amino, ou un résidu phényle, benzyle, benzoylamino ou phénoxy éventuellement substitués par un groupe alkyle en $C_{1-4}$, alcoxy en $C_{1-4}$, acétylamino, halogéno, nitro ou sulfo,

B représente un chaînon pontant de formule

$$Q'\text{-}E'\text{-}Q' \qquad (3'),$$

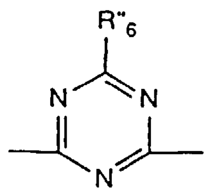

(4'),

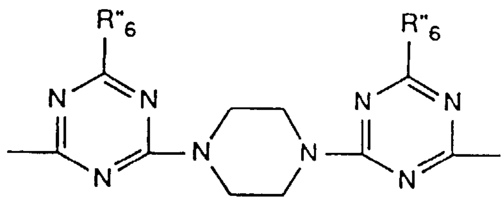

(6'),

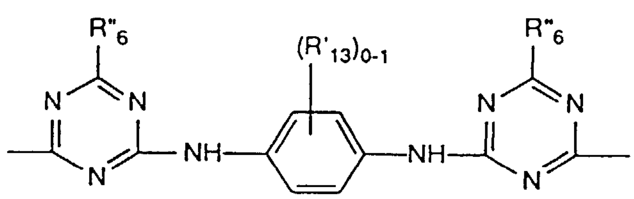

(7').

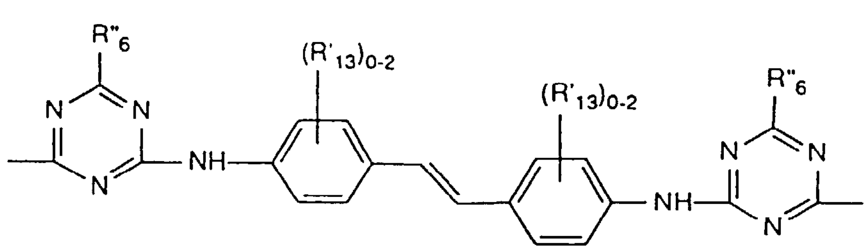

(8)

où Q' représente un groupe

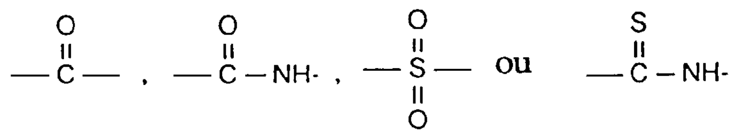

, , ou

E représente une liaison directe, un groupe alkylène en $C_{1-6}$ ou alcénylène en $C_{2-6}$ non substitué ou portant un substituant hydroxy, sulfo, sulfato, méthoxy, carboxy, phényle ou sulfophényle, un résidu cyclohexylène ou (alkylène en $C_{1-2}$)-cyclohexylène non substitué ou portant de 1 à 3 substituants méthyle, un résidu pipérazine-1 ,4-diyle, thiophène-2,5-diyle, biphényl-4,4'-diyle, stilbène-4,4'-diyle, phénylène ou naphtylène non substitué ou portant un substituant alkyle en $C_{1-4}$, alcoxy en $C_{1-4}$, sulfo, halogéno ou carboxy, ou un résidu (alkylène en $C_{1-3}$)-phényléne ou (alkylène en $C_{1-2}$)-phénylène-(alkylène en $C_{1-2}$) non substitué ou portant sur le noyau phénylène un substituant méthyle, méthoxy, chloro ou sulfo, $R''_6$ représente un atome de chlore, un groupe hydroxy, alcoxy en $C_{1-4}$, alkylthio en $C_{1-2}$, amino, N-mono- ou N,N-di(alkyle en $C_{1-4}$)-amino non substitué ou portant sur le ou les résidus alkyle un substituant hydroxy, sulfo ou sulfato, un groupe cylohexylamino, un groupe phénylamino ou N-(alkyle en $C_{1-4}$)-N-phénylamino non substitué ou portant sur le noyau phényle un groupe méthyle, méthoxy, carboxy, sulfo ou chloro, ou un groupe morpholino, et
$R'_{13}$ représente un groupe sulfo, méthyle ou méthoxy,
m est un nombre compris entre 1 et 6, et
n et p représentent, indépendamment l'un de l'autre, un nombre égal à 0 ou
1, les différents composés de formule (1) se distinguant uniquement par la valeur que prend la variable m,

caractérisé en ce que l'on

a) effectue la condensation d'un composé de formule

(9) 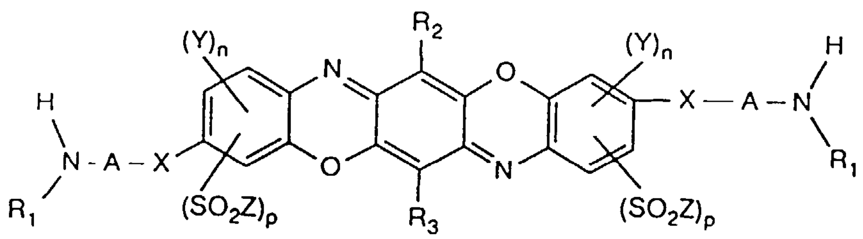

avec un composé de formule

(10) T-B-T

et b) en ce que l'on fait réagir éventuellement le produit obtenu dans a) avec un composé de formule

(11) R*-T

où A, B, $R_1$, $R_2$, $R_3$, X, Y, Z, p et n ont la signification indiquée ci-avant, R* a la signification indiquée précédemment pour R, à l'exception d'un atome d'hydrogène, et T représente un atome d'halogène, de préférence un atome de chlore.

**2.** Procédé conforme à la revendication 1, caractérisé en ce que l'on utilise un composé de formule (9) dans lequel $R_1$ représente un atome d'hydrogène, un groupe alkyle en $C_{1-4}$, cyclohexyle, ou phényle ou benzyle non substitué ou portant un substituant sulfo, chloro, méthyle et/ou méthoxy.

**3.** Procédé conforme à une des revendications 1 et 2, caractérisé en ce que l'on utilise un composé de formule (9) dans lequel $R_1$ représente un atome d'hydrogène.

**4.** Procédé conforme à une des revendications 1 à 3, caractérisé en ce que l'on fait réagir un composé de formule

(9) avec un composé de formule (10) et en ce que l'on fait réagir éventuellement le produit obtenu avec un composé de formule (11) où R* représente un groupe alkyle en $C_{1-4}$, phényle ou benzyle non substitué ou portant un substituant sulfo, chloro, méthyle et/ou méthoxy ou un groupe de formule

$$R_4\text{-C(=O)-}$$

où $R_4$ représente un résidu méthyle, éthyle ou phényle non substitué ou portant un substituant sulfo, chloro, méthyle et/ou méthoxy.

**5.** Procédé conforme à une des revendications 1 à 4, caractérisé en ce que l'on fait réagir un composé de formule (9) avec un composé de formule (10) et en ce que l'on fait réagir éventuellement le produit obtenu avec un composé de formule (11) où R* représente un groupe méthyle, éthyle, benzyle, acétylamino ou benzoylamino, il étant recommandé de faire réagir seulement le composé de formule (9) avec le composé de formule (10).

**6.** Procédé conforme à une des revendications 1 à 5, caractérisé en ce que l'on utilise un composé de formule (9) dans lequel A représente un résidu alkylène en $C_{2-4}$ non substitué ou portant un résidu hydroxy, sulfo, sulfato, méthoxy, carboxy ou sulfophényle, un groupe $-CH_2-CH_2-Z'-CH_2-CH_2-$ où Z' est un chaînon -O-, -S-, $-SO_2-$, -NH- ou $-N(CH_3)-$, un résidu cyclohexylène non substitué ou portant de 1 à 3 résidus méthyle, un résidu 1,3-phénylène ou 1,4-phénylène non substitué ou sulfoné, ou un résidu (alkylène en $C_{1-3}$)-phényléne ou (alkylène en $C_{1-2}$)-phénylène-(alkylène en $C_{1-2}$) dans lesquels le noyau phénylène peut être non substitué ou porter un résidu méthyle, méthoxy, chloro ou sulfo.

**7.** Procédé conforme à une des revendications 1 à 6, caractérisé en ce que l'on utilise un composé de formule (9) dans lequel A représente un résidu alkylène en $C_{2-4}$ non substitué ou portant un substituant hydroxy, sulfo, sulfato, méthoxy, carboxy ou sulfophényle.

**8.** Procédé conforme à une des revendications 1 à 7, caractérisé en ce que l'on utilise un composé de formule (9) dans lequel A représente un résidu 1,2-éthylène ou 1,2-propylène ou 1,3-propylène non substitué ou portant un substituant hydroxy ou sulfato.

**9.** Procédé conforme à une des revendications 1 à 8, caractérisé en ce que l'on utilise un composé de formule (9) dans lequel X représente un résidu $-N(R_5)-$ où $R_5$ représente un atome d'hydrogène, un groupe alkyle en $C_{1-4}$, cyclohexyle, ou un groupe phényle ou benzyle non substitué ou portant un substituant sulfo, chloro, méthyle et /ou méthoxy.

**10.** Procédé conforme à la revendication 9, caractérisé en ce que l'on utilise un composé de formule (9) dans lequel $R_5$ représente un atome d'hydrogène ou un groupe alkyle en $C_{1-4}$, en particulier un atome d'hydrogène.

**11.** Procédé conforme à une des revendications 1 à 10, caractérisé en ce que l'on utilise un composé de formule (9) dans lequel n vaut 0.

**12.** Procédé conforme à une des revendications 1 à 11, caractérisé en ce que l'on utilise un composé de formule (9) dans lequel p vaut 1 et Z représente un groupe hydroxy ou alkyle en $C_{1-4}$, en particulier un groupe hydroxy.

**13.** Procédé conforme à une des revendications 1 à 12, caractérisé en ce que l'on utilise un composé de formule (9) dans lequel $R_2$ et $R_3$ représentent chacun un atome d'hydrogène, un atome de fluor, de chlore ou de brome, un résidu méthyle, méthoxy, acétylamino, phénoxy ou cyano.

**14.** Procédé conforme à une des revendications 1 à 13, caractérisé en ce que l'on utilise un composé de formule (9) dans lequel $R_2$ et $R_3$ représentent chacun un atome de chlore.

**15.** Procédé conforme à une des revendications 1 à 14, caractérisé en ce que l'on utilise un composé de formule (10) dans lequel B représente un chaînon pontant de formule

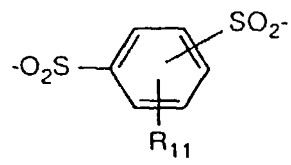 , 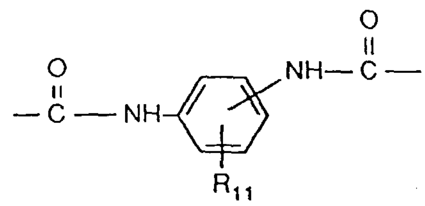 , 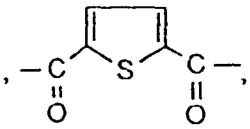

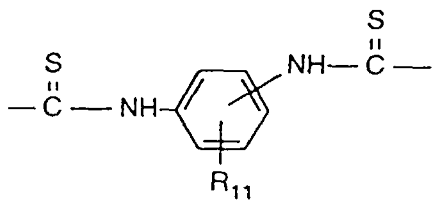 , 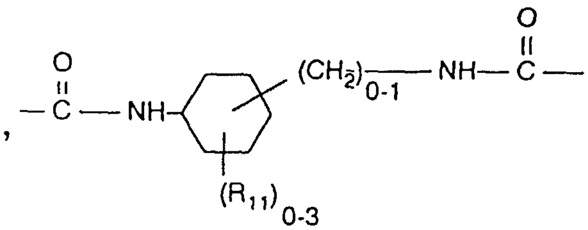

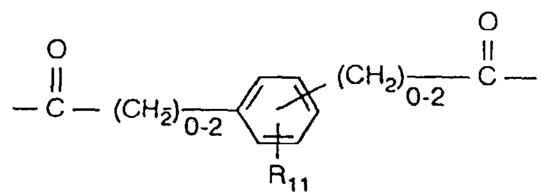 , 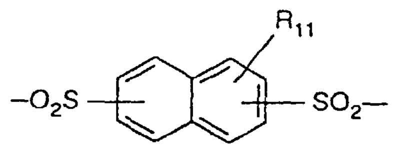 ,

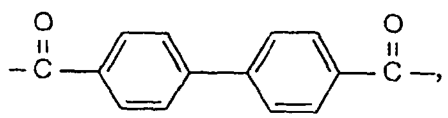 , 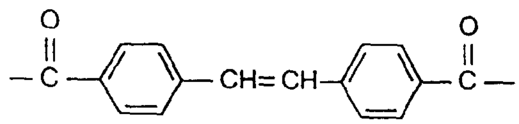 ,

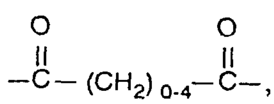 , 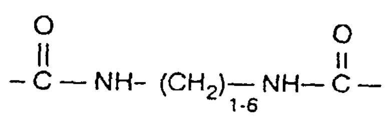 , 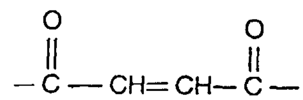

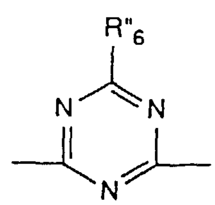 ,

dans lesquelles $R_{11}$ représente un résidu sulfo, méthyle, méthoxy, chloro, carboxy ou, de préférence, un atome d'hydrogène, et $R''_6$ représente un groupe hydroxy, chloro, méthylthio ou éthylthio, méthoxy, éthoxy, *n*- ou *iso*-propoxy, amino, méthylamino, éthylamino, β-hydroxyéthylamino, N,N-di-β-hydroxyéthylamino, β-sulfoéthylamino, cyclohexylamino, o-, m- ou p-méthylphénylamino, o-, m- ou p-méthoxyphénylamino, o-, m- ou p-chlorophénylamino, o-, m- ou p-sulfophénylamino, 2,4- ou 2,5-disulfophénylamino, o-carboxyphénylamino, N-éthyl-N-phénylamino, N-méthyl-N-phénylamino ou morpholino.

**16.** Procédé conforme à une des revendications 1 à 15, caractérisé en ce que l'on utilise un composé de formule (10) dans lequel B représente un résidu de formule

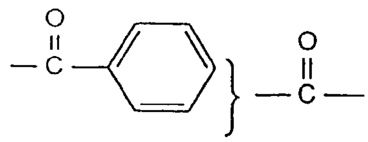

ou

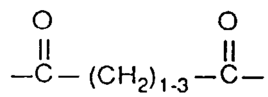

**17.** Procédé conforme à la revendication 1, caractérisé en ce que l'on fait réagir un composé de formule (9) dans lequel $R_1$ représente un atome d'hydrogène, un groupe alkyle en $C_{1-4}$ non substitué ou portant un substituant hydroxy, sulfo, sulfato, chloro, cyano ou acétoxy et/ou pouvant être interrompu, à l'exception du groupe méthyle, par un groupe -O-, un groupe cyclopentyle ou cyclohexyle non substitué ou portant de 1 à 3 substituants méthyle, un groupe phényle non substitué ou portant un substituant sulfo, nitro, chloro, méthyle, méthoxy, N-méthylamino ou N-éthylamino, N,N-diméthylamino ou N,N-diéthylamino, acétylamino, propionylamino, benzo-ylamino, méthoxycarbonyle, éthoxycarbonyle, carboxy ou méthylsulfonyle, un groupe 1-naphtyle ou 2-naphtyle non substitué ou portant un substituant sulfo, nitro et/ou chloro, ou encore un résidu benzyle non substitué ou portant un résidu méthyle, méthoxy, sulfo, et/ou chloro,

A représente un groupe alkylène en $C_{2-4}$ non substitué ou portant un substituant hydroxy, sulfo, sulfato, méthoxy, carboxy ou sulfophényle, un groupe $-CH_2-CH_2-Z'-CH_2-CH_2-$ où Z' représente un groupe -O-, -S-, $-SO_2-$, -NH- ou $-N(CH_3)-$, un résidu cyclohexylène non substitué ou portant de 1 à 3 substituants méthyle, un résidu 1,3-phénylène ou 1,4-phénylène non substitué ou portant un groupe sulfo ou un résidu (alkylène en $C_{1-3}$)-phénypène ou (alkylène en $C_{1-2}$)-phénylène-(alkylène en $C_{1-2}$) non substitué ou portant, sur leur noyau phénylène, un substituant méthyle, méthoxy, chloro ou sulfo,
X représente un groupe $-N(R_5)-$ où $R_5$ représente un atome d'hydrogène, un groupe alkyle en $C_{1-4}$, cyclohexyle, phényle ou benzyle non substitué ou portant un substituant sulfo, chloro, méthyle et/ou méthoxy, ou dans lequel le groupe

$$R_1\text{-}N\text{-}A\text{-}X$$

représente un résidu piperazine-1,4-alyle,
Y est un groupe méthoxy, méthyle, chloro ou sulfo,
n vaut 0 ou 1,
Z représente un groupe hydroxy, méthyle ou éthyle,
p vaut 1,
$R_2$ et $R_3$ représentent chacun un atome d'hydrogène, un atome de fluor, de chlore ou de brome, un résidu méthyle, méthoxy, acétylamino, phénoxy ou cyano,

avec un composé de formule (10), et en ce que l'on fait réagir éventuellement le produit obtenu avec un composé de formule de formule (11) dans lequel R* représente un un résidu alkyle en $C_{1-4}$, phényle ou benzyle non substitué ou portant un substituant méthyle, méthoxy, chloro et/ou sulfo, ou un résidu de formule

$$R_4\text{-}C(=O)\text{-}$$

dans laquelle $R_4$ représente un groupe méthyle, éthyle ou un résidu phényle non substitué ou portant un résidu sulfo, chloro, méthyle et/ou méthoxy, m étant, dans le mélange d'oligomères obtenu, un nombre compris entre 1 et 3.

**18.** Procédé conforme à la revendication 1 caractérisé en ce que l'on fait réagir un composé de formule

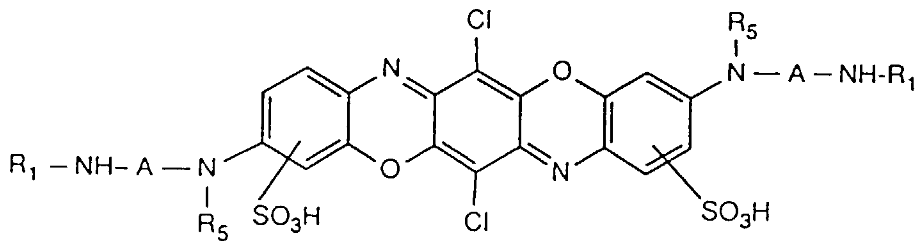

dans lequel

$R_1$ représente un atome d'hydrogène, un groupe alkyle en $C_{1-4}$, cyclohexyle, un groupe phényle ou benzyle non substitué ou portant un substituant sulfo, chloro, méthyle et/ou méthoxy,
A représente un résidu alkylène en $C_{2-4}$ non substitué ou portant un résidu hydroxy, sulfo, sulfato, méthoxy, carboxy ou sulfophényle,
$R_5$ représente un atome d'hydrogène, ou un groupe méthyle ou éthyle, avec un composé de formule (10) dans lequel
B est un résidu de formule

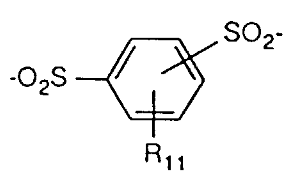 , 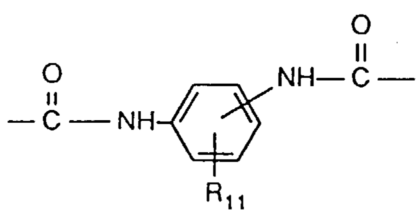 , 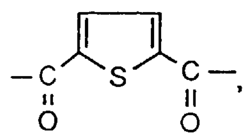 ,

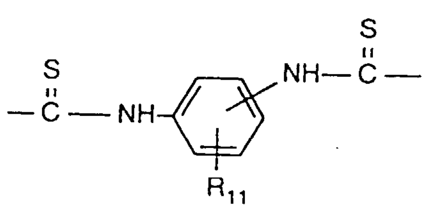 , 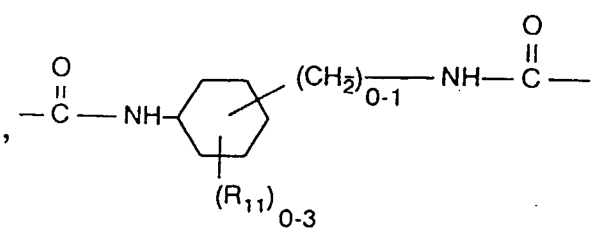

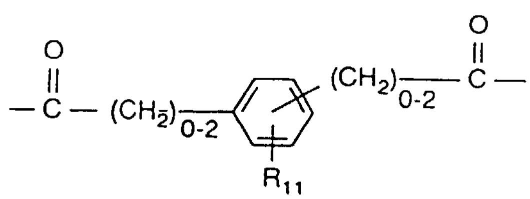 , 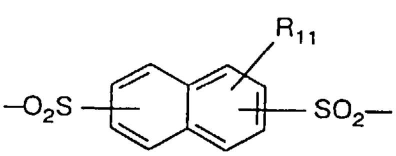 ,

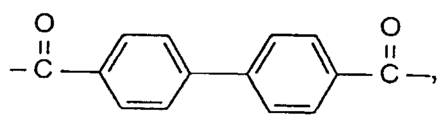 , 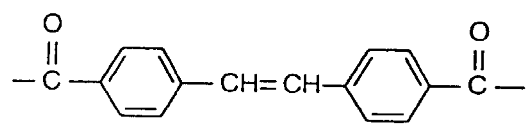 ,

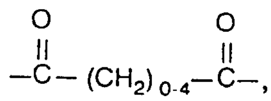, 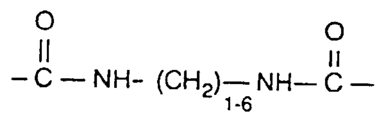 , 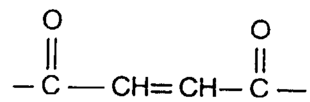

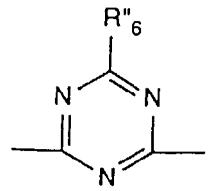 ,

où $R_{11}$ représente un résidu sulfo, méthyle, méthoxy, chloro, carboxy ou, de préférence, un atome d'hydrogène, et $R''_6$ est un groupe hydroxy, chloro, méthylthio ou éthylthio, méthoxy, éthoxy, *n*- ou *iso*-propoxy, amino, méthylamino, éthylamino, β-hydroxyéthylamino, N,N-di-β-hydroxyéthylamino, β-sulfoéthylamino, cyclohexylamino, o-, m- ou p-méthylphénylamino, o-, m- ou p-méthoxyphénylamino, o-, m- ou p-chlorophénylamino, o-, m- ou p-sulfophénylamino, 2,4- ou 2,5-disulfophénylamino, o-carboxyphénylamino, N-éthyl-N-phénylamino, N-méthyl-N-phénylamino ou morpholino, et en ce que l'on fait réagir éventuellement le produit obtenu avec un composé de formule (11) dans lequel R* est un groupe méthyle, éthyle, benzyle, acétylamino ou benzoylamino.

**19.** Procédé conforme à la revendication 1, caractérisé en ce que l'on fait réagir un composé de formule

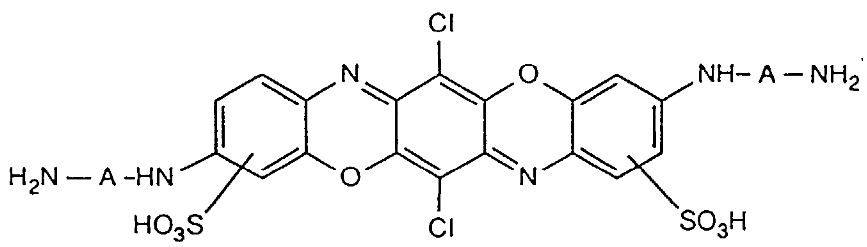

où A représente un groupe 1,2-éthylène ou 1,2- ou 1,3-propylène non substitué ou portant un substituant hydroxy ou sulfato, avec un composé de formule (10) dans lequel B représente un résidu de formule

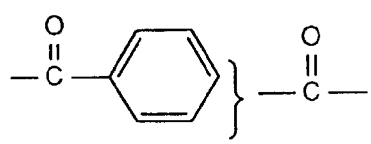

ou

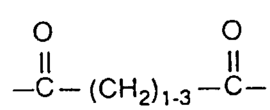

et en ce que l'on fait réagir éventuellement le produit obtenu avec un composé de formule (11) dans lequel R* représente un résidu méthyle, éthyle, benzyle, acétylamino ou benzoylamino.

**20.** Utilisation des mélanges de différents oligomères de formule (1) obtenus conformément à la revendication 1 en tant que colorants pour la teinture et l'impression de matériaux fibreux contenant des atomes d'azote et en particulier des matériaux fibreux cellulosiques.

**21.** Utilisation conforme à la revendication 20, caractérisée en ce que l'on teint des mélanges de fibres contenant des fibres synthétiques et des matériaux fibreux cellulosiques, en particulier des tissus mixtes polyester/coton, en présence d'un colorant de dispersion pour les fibres de polyester dans des conditions de teinture du polyester.

**22.** Procédé de teinture de tissus mixtes polyester/coton avec des colorant directs et des colorants de dispersion, caractérisé en ce que l'on utilise dans un procédé à bain unique et étape unique, en tant que colorants, en plus des colorants de dispersion, des mélanges de différents composés oligomères de formule (1) obtenus conformément à la revendication 1, et en ce que l'on effectue la teinture dans un bain aqueux à des températures comprises entre 100 et 150 °C, de préférence entre 120 et 130 °C et à un pH compris entre 4 et 7,5.